# EUROPEAN PATENT SPECIFICATION

(11) **EP 2 631 654 B1**
(45) Date of publication and mention of the grant of the patent: **15.04.2015**
(21) Application number: 13169122.2
(22) Date of filing: 07.05.2012
(51) Int. Cl.: G01N 33/567

(54) **Neuropeptide release assay for sodium channels**
Neuropeptid-Freisetzungsanalyse für Natriumkanäle
Analyse de libération de neuropeptides pour canaux de sodium

(30) Priority: 12.05.2011 US 201161485488 P; 08.06.2011 US 201113155491; 19.09.2011 US 201113236117
(43) Date of publication of application: 28.08.2013
(62) Divisional of application: 12167014.5
(73) Proprietor: Regeneron Pharmaceuticals, Inc., Tarrytown, NY 10591 (US)
(72) Inventor: Alessandri Haber, Nicole M., Rye Brook, NY 10573 (US); MacDonald, Lynn, White Plains, NY 10605 (US); Lacroix-Fralish, Michael L., Yorktown Heights, NY 10598 (US); Murphy, Andrew J., Croton-on-Hudson, NY 10520 (US)
(74) Representative: Grund, Martin

(56) References cited:
- WO-A1-2010/087864
- WO-A2-2007/109324
- GHELARDINI C ET AL: "Effects of a new potent analog of tocainide on hNav1.7 sodium channels and in vivo neuropathic pain models", NEUROSCIENCE, NEW YORK, NY, US, vol. 169, no. 2, 25 August 2010 (2010-08-25), pages 863-873, XP027221974, ISSN: 0306-4522 [retrieved on 2010-05-16]
- TRIVEDI SHEPHALI ET AL: "Cellular HTS assays for pharmacological characterization of Na(V)1.7 modulators", ASSAY AND DRUG DEVELOPMENT TECHNOLOGIES, vol. 6, no. 2, April 2008 (2008-04), pages 167-179, XP009162258, ISSN: 1540-658X
- WILLIAMS BRANDE S ET AL: "Characterization of a new class of potent inhibitors of the voltage-gated sodium channel Nav1.7", BIOCHEMISTRY, vol. 46, no. 50, December 2007 (2007-12), pages 14693-14703, XP009162257, ISSN: 0006-2960

### SEQUENCE LISTING

<110> Regeneron Pharmaceuticals, Inc.
<120> NEUROPEPTIDE RELEASE ASSAY FOR SODIUM CHANNELS
<130> 210-002EPT2
<150> EP 12167014.5
   <151> 2012-05-07
<150> US 13/236,117
   <151> 2011-09-19
<150> US 13/155,491
   <151> 2011-06-08
<150> US 61/485,488
   <151> 2011-05-12
<160> 43
<170> FastSEQ for Windows Version 4.0
<210> 1
   <211> 22
   <212> DNA
   <213> Artificial Sequence
<220>
   <223> Synthetic
<400> 1
   gggacttctc tgggttcagt ta 22
<210> 2
   <211> 23
   <212> DNA
   <213> Artificial Sequence
<220>
   <223> Synthetic
<400> 2
   aaaggctctc aatgggaaac aag 23
<210> 3
   <211> 29
   <212> DNA
   <213> Artificial Sequence
<220>
   <223> Synthetic
<400> 3
   tcaatgactt gacataatgc atgcactcc 29
<210> 4
   <211> 24
   <212> DNA
   <213> Artificial Sequence
<220>
   <223> Synthetic
<400> 4
   atgtcagcca atccttctaa agtg 24
<210> 5
   <211> 20
   <212> DNA
   <213> Artificial Sequence
<220>
   <223> Synthetic
<400> 5
   cgttttgcct aaggcggtac 20
<210> 6
   <211> 25
   <212> DNA
   <213> Artificial Sequence
<220>
   <223> Synthetic
<400> 6
   tcctatgagc ccatcacaac cacac 25
<210> 7
   <211> 19
   <212> DNA
   <213> Artificial Sequence
<220>
   <223> Synthetic
<400> 7
   ggtggagagg ctattcggc 19
<210> 8
   <211> 17
   <212> DNA
   <213> Artificial Sequence
<220>
   <223> Synthetic
<400> 8
   gaacacggcg gcatcag 17
<210> 9
   <211> 23
   <212> DNA
   <213> Artificial Sequence
<220>
   <223> Synthetic
<400> 9
   tgggcacaac agacaatcgg ctg 23
<210> 10
   <211> 115
   <212> DNA
   <213> Artificial Sequence
<220>
   <223> Synthetic
<400> 10
   ctagctgagc tgtcaccaca cattgctcct accacgtatt gtacagctac tgcaagagca 60
   ccacagttgg ctttctgtat cataacttcg tataatgtat gctatacgaa gttat      115
<210> 11
   <211> 148
   <212> DNA
   <213> Artificial Sequence
<220>
   <223> Synthetic
<400> 11
   ataacttcgt ataatgtatg ctatacgaag ttatagcttc ggttttgata cactgtttac 60
   agcctgcgaa ggtgactcac tcgtgttaat aagactcttt tacggaggtc tatgccaaac 120
   tctttttatc aaatattctc aaaggcag                                   148
<210> 12
   <211> 20
   <212> DNA
   <213> Artificial Sequence
<220>
   <223> Synthetic
<400> 12
   atcaaaggaa cccaaagaag 20
<210> 13
   <211> 20
   <212> DNA
   <213> Artificial Sequence
<220>
   <223> Synthetic
<400> 13
   gaagggcagc tgtttgccag 20
<210> 14
   <211> 24
   <212> DNA
   <213> Artificial Sequence
<220>
   <223> Synthetic
<400> 14
   atgaagaagc cccaaagcca agca 24
<210> 15
   <211> 17
   <212> DNA
   <213> Artificial Sequence
<220>
   <223> Synthetic
<400> 15
   tgcggccgat cttagcc 17
<210> 16
   <211> 18
   <212> DNA
   <213> Artificial Sequence
<220>
   <223> Synthetic
<400> 16
   ttgaccgatt ccttgcgg 18
<210> 17
   <211> 21
   <212> DNA
   <213> Artificial Sequence
<220>
   <223> Synthetic
<400> 17
   acgagcgggt tcggcccatt c 21
<210> 18
   <211> 99
   <212> DNA
   <213> Artificial Sequence
<220>
   <223> Synthetic
<400> 18
   ttaggtaagg atccgaaggg gaaataaaac ctacaggatg agaagatggc aatgttgcct 60
   cccccaggac ctcagagctt tgtccatttc acaaaacag 99
<210> 19
   <211> 87
   <212> DNA
   <213> Artificial Sequence
<220>
   <223> Synthetic
<400> 19
   gtatgaataa aaaagcattg aaatagggat tcttgccaac ttgctctctc gagataactt 60
   cgtataatgt atgctatacg aagttat                                     87
<210> 20
   <211> 90
   <212> DNA
   <213> Artificial Sequence
<220>
   <223> Synthetic
<400> 20
   tatacgaagt tatgctagta actataacgg tcctaaggta gcgagctagc agcttcggtt 60
   ttgatacact gtttacagcc tgcgaaggtg                                  90
<210> 21
   <211> 24
   <212> DNA
   <213> Artificial Sequence
<220>
   <223> Synthetic
<400> 21
   ggactacaac tgtttatggg caac 24
<210> 22
   <211> 24
   <212> DNA
   <213> Artificial Sequence
<220>
   <223> Synthetic
<400> 22
   tcaattcttc ttcactctca gcag 24
<210> 23
   <211> 25
   <212> DNA
   <213> Artificial Sequence
<220>
   <223> Synthetic
<400> 23
   tccggaagga ccttgagcag aatga 25
<210> 24
   <211> 20
   <212> DNA
   <213> Artificial Sequence
<220>
   <223> Synthetic
<400> 24
   caacaggtga gcagcaacag 20
<210> 25
   <211> 22
   <212> DNA
   <213> Artificial Sequence
<220>
   <223> Synthetic
<400> 25
   gcaggagaca catacaccag ac 22
<210> 26
   <211> 26
   <212> DNA
   <213> Artificial Sequence
<220>
   <223> Synthetic
<400> 26
   aaacacgcat gtctgaaggc agtcgg 26
<210> 27
   <211> 201
   <212> DNA
   <213> Artificial Sequence
<220>
   <223> Synthetic
<400> 27
   tacattttaa ggactaaaaa ccatcgtggg ggccctgatc caatcagtga agaagctctc 60
   tgacgtcatg atcctcactg tgttctgtct cagtgtgttc gcactaattg gactacagct 120
   gttcatggga aacctgaagc ataaatgttt tcgaaattca cttgaaaata atgaaacatt 180
   agaaagcata atgaataccc t 201
<210> 28
   <211> 157
   <212> DNA
   <213> Artificial Sequence
<220>
   <223> Synthetic
<400> 28
   aggtgagtac caagagaaac atgcattgta tttttgaatg gcatatgtac ctggtgtatg 60
   ttaagagcct gtattaggag gttttttatt tatttgagaa tggaggaaac tctattactc 120
   gagataactt cgtataatgt atgctatacg aagttat 157
<210> 29
   <211> 141
   <212> DNA
   <213> Artificial Sequence
<220>
   <223> Synthetic
<400> 29
   tatacgaagt tatgctagct ctgcagacag tctgggactc cctaatgtgc attattaaaa 60
   ttacaggcaa tttacttggc tgatatgaga acagatagtt ctgaagtcat caataatttt 120
   ctgctgtgtc tgaccagcgt t                                           141
<210> 30
   <211> 19
   <212> DNA
   <213> Artificial Sequence
<220>
   <223> Synthetic
<400> 30
   gcttgggctt gcaccttta 19
<210> 31
   <211> 22
   <212> DNA
   <213> Artificial Sequence
<220>
   <223> Synthetic
<400> 31
   tgcgttgacc actacctgat ac 22
<210> 32
   <211> 23
   <212> DNA
   <213> Artificial Sequence
<220>
   <223> Synthetic
<400> 32
   tctgcattgg cgtctgtttg tca 23
<210> 33
   <211> 25
   <212> DNA
   <213> Artificial Sequence
<220>
   <223> Synthetic
<400> 33
   tgacttgccc tatcaatctg agatc 25
<210> 34
   <211> 28
   <212> DNA
   <213> Artificial Sequence
<220>
   <223> Synthetic
<400> 34
   gctcacactg tatacacaca aaatcttc 28
<210> 35
   <211> 19
   <212> DNA
   <213> Artificial Sequence
<220>
   <223> Synthetic
<400> 35
   tcactgccta tgataaagt 19
<210> 36
   <211> 20
   <212> DNA
   <213> Artificial Sequence
<220>
   <223> Synthetic
<400> 36
   cacggtttcc tgcaagtcaa 20
<210> 37
   <211> 22
   <212> DNA
   <213> Artificial Sequence
<220>
   <223> Synthetic
<400> 37
   gggacactta caacttgaag ca 22
<210> 38
   <211> 26
   <212> DNA
   <213> Artificial Sequence
<220>
   <223> Synthetic
<400> 38
   tcgttccgaa tgttttgccc ttatga 26
<210> 39
   <211> 78
   <212> DNA
   <213> Artificial Sequence
<220>
   <223> Synthetic
<400> 39
   tttcatttat ttgaagtgca atatcatctt ggccatctac tcctctgtat gctagtaggt 60
   aagcctggtg atcacaga 78
<210> 40
   <211> 65
   <212> DNA
   <213> Artificial Sequence
<220>
   <223> Synthetic
<400> 40
   gactagtata caattacaaa tatgcctcga gataacttcg tataatgtat gctatacgaa 60
   gttat 65
<210> 41
   <211> 93
   <212> DNA
   <213> Artificial Sequence
<220>
   <223> Synthetic
<400> 41
   tatacgaagt tatgctagct ttcctgctaa ccatcattct ggggtatgtg ttatgatgga 60
   agttaagtga cagttactta taatatggct gct 93
<210> 42
   <211> 9771
   <212> DNA
   <213> Artificial Sequence
<220>
   <223> Synthetic
<400> 42
   cggggctgct acctccacgg gcgcgccctg gcaggagggg cgcagtctgc ttgcaggcgg 60
   tcgccagcgc tccagcggcg gctgtcggct ttccaattcc gccagctcgg ctgaggctgg 120
   gctagcctgg gtgccagtgg ctgctagcgg caggcgtccc ctgagcaaca ggagcccaga 180
   gaaaaagaag cagccctgag agagcgccgg ggaaggagag gcccgcgccc tctcctggag 240
   ccagattctg caggtgcact gggtggggat gatcggcggg ctaggttgca agcctcttat 300
   gtgaggagct gaagaggaat taaaatatac aggatgaaaa gatggcaatg ttgcctcccc 360
   caggacctca gagctttgtc catttcacaa aacagtctct tgccctcatt gaacaacgca 420
   ttgctgaaag aaaatcaaag gaacccaaag aagaaaagaa agatgatgat gaagaagccc 480
   caaagccaag cagtgacttg gaagctggca aacagctgcc cttcatctat ggggacattc 540
   ctcccggcat ggtgtcagag cccctggagg acttggaccc ctactatgca gacaaaaaga 600
   ctttcatagt attgaacaaa gggaaaacaa tcttccgttt caatgccaca cctgctttat 660
   atatgctttc tcctttcagt cctctaagaa gaatatctat taagatttta gtacactcct 720
   tattcagcat gctcatcatg tgcactattc tgacaaactg catatttatg accatgaata 780
   acccaccgga ctggaccaaa aatgtcgagt acacttttac tggaatatat acttttgaat 840
   cacttgtaaa aatccttgca agaggcttct gtgtaggaga attcactttt cttcgtgacc 900
   cgtggaactg gctggatttt gtcgtcattg tttttgcgta tttaacagaa tttgtaaacc 960
   taggcaatgt ttcagctctt cgaactttca gagtattgag agctttgaaa actatttctg 1020
   taatcccagg cctgaagaca attgtagggg ctttgatcca gtcagtgaag aagctttctg 1080
   atgtcatgat cctgactgtg ttctgtctga gtgtgtttgc actaattgga ctacagctgt 1140
   tcatgggaaa cctgaagcat aaatgttttc gaaattcact tgaaaataat gaaacattag 1200
   aaagcataat gaatacccta gagagtgaag aagactttag aaaatatttt tattacttgg 1260
   aaggatccaa agatgctctc ctttgtggtt tcagcacaga ttcaggtcag tgtccagagg 1320
   ggtacacctg tgtgaaaatt ggcagaaacc ctgattatgg ctacacgagc tttgacactt 1380
   tcagctgggc cttcttagcc ttgtttaggc taatgaccca agattactgg gaaaaccttt 1440
   accaacagac gctgcgtgct gctggcaaaa cctacatgat cttctttgtc gtagtgattt 1500
   tcctgggctc cttttatcta ataaacttga tcctggctgt ggttgccatg gcatatgaag 1560
   aacagaacca ggcaaacatt gaagaagcta aacagaaaga attagaattt caacagatgt 1620
   tagaccgtct taaaaaagag caagaagaag ctgaggcaat tgcagcggca gcggctgaat 1680
   atacaagtat taggagaagc agaattatgg gcctctcaga gagttcttct gaaacatcca 1740
   aactgagctc taaaagtgct aaagaaagaa gaaacagaag aaagaaaaag aatcaaaaga 1800
   agctctccag tggagaggaa aagggagatg ctgagaaatt gtcgaaatca gaatcagagg 1860
   acagcatcag aagaaaaagt ttccaccttg gtgtcgaagg gcataggcga gcacatgaaa 1920
   agaggttgtc tacccccaat cagtcaccac tcagcattcg tggctccttg ttttctgcaa 1980
   ggcgaagcag cagaacaagt ctttttagtt tcaaaggcag aggaagagat ataggatctg 2040
   agactgaatt tgccgatgat gagcacagca tttttggaga caatgagagc agaaggggct 2100
   cactgtttgt gccccacaga ccccaggagc gacgcagcag taacatcagc caagccagta 2160
   ggtccccacc aatgctgccg gtgaacggga aaatgcacag tgctgtggac tgcaacggtg 2220
   tggtctccct ggttgatgga cgctcagccc tcatgctccc caatggacag cttctgccag 2280
   agggcacgac caatcaaata cacaagaaaa ggcgttgtag ttcctatctc ctttcagagg 2340
   atatgctgaa tgatcccaac ctcagacaga gagcaatgag tagagcaagc atattaacaa 2400
   acactgtgga agaacttgaa gagtccagac aaaaatgtcc accttggtgg tacagatttg 2460
   cacacaaatt cttgatctgg aattgctctc catattggat aaaattcaaa aagtgtatct 2520
   attttattgt aatggatcct tttgtagatc ttgcaattac catttgcata gttttaaaca 2580
   cattatttat ggctatggaa caccacccaa tgactgagga attcaaaaat gtacttgcta 2640
   taggaaattt ggtctttact ggaatctttg cagctgaaat ggtattaaaa ctgattgcca 2700
   tggatccata tgagtatttc caagtaggct ggaatatttt tgacagcctt attgtgactt 2760
   taagtttagt ggagctcttt ctagcagatg tggaaggatt gtcagttctg cgatcattca 2820
   gactgctccg agtcttcaag ttggcaaaat cctggccaac attgaacatg ctgattaaga 2880
   tcattggtaa ctcagtaggg gctctaggta acctcacctt agtgttggcc atcatcgtct 2940
   tcatttttgc tgtggtcggc atgcagctct ttggtaagag ctacaaagaa tgtgtctgca 3000
   agatcaatga tgactgtacg ctcccacggt ggcacatgaa cgacttcttc cactccttcc 3060
   tgattgtgtt ccgcgtgctg tgtggagagt ggatagagac catgtgggac tgtatggagg 3120
   tcgctggtca agctatgtgc cttattgttt acatgatggt catggtcatt ggaaacctgg 3180
   tggtcctaaa cctatttctg gccttattat tgagctcatt tagttcagac aatcttacag 3240
   caattgaaga agaccctgat gcaaacaacc tccagattgc agtgactaga attaaaaagg 3300
   gaataaatta tgtgaaacaa accttacgtg aatttattct aaaagcattt tccaaaaagc 3360
   caaagatttc cagggagata agacaagcag aagatctgaa tactaagaag gaaaactata 3420
   tttctaacca tacacttgct gaaatgagca aaggtcacaa tttcctcaag gaaaaagata 3480
   aaatcagtgg ttttggaagc agcgtggaca aacacttgat ggaagacagt gatggtcaat 3540
   catttattca caatcccagc ctcacagtga cagtgccaat tgcacctggg gaatccgatt 3600
   tggaaaatat gaatgctgag gaacttagca gtgattcgga tagtgaatac agcaaagtga 3660
   gattaaaccg gtcaagctcc tcagagtgca gcacagttga taaccctttg cctggagaag 3720
   gagaagaagc agaggctgaa cctatgaatt ccgatgagcc agaggcctgt ttcacagatg 3780
   gttgtgtacg gaggttctca tgctgccaag ttaacataga gtcagggaaa ggaaaaatct 3840
   ggtggaacat caggaaaacc tgctacaaga ttgttgaaca cagttggttt gaaagcttca 3900
   ttgtcctcat gatcctgctc agcagtggtg ccctggcttt tgaagatatt tatattgaaa 3960
   ggaaaaagac cattaagatt atcctggagt atgcagacaa gatcttcact tacatcttca 4020
   ttctggaaat gcttctaaaa tggatagcat atggttataa aacatatttc accaatgcct 4080
   ggtgttggct ggatttccta attgttgatg tttctttggt tactttagtg gcaaacactc 4140
   ttggctactc agatcttggc cccattaaat cccttcggac actgagagct ttaagacctc 4200
   taagagcctt atctagattt gaaggaatga gggtcgttgt gaatgcactc ataggagcaa 4260
   ttccttccat catgaatgtg ctacttgtgt gtcttatatt ctggctgata ttcagcatca 4320
   tgggagtaaa tttgtttgct ggcaagttct atgagtgtat taacaccaca gatgggtcac 4380
   ggtttcctgc aagtcaagtt ccaaatcgtt ccgaatgttt tgcccttatg aatgttagtc 4440
   aaaatgtgcg atggaaaaac ctgaaagtga actttgataa tgtcggactt ggttacctat 4500
   ctctgcttca agttgcaact tttaagggat ggacgattat tatgtatgca gcagtggatt 4560
   ctgttaatgt agacaagcag cccaaatatg aatatagcct ctacatgtat atttattttg 4620
   tcgtctttat catctttggg tcattcttca ctttgaactt gttcattggt gtcatcatag 4680
   ataatttcaa ccaacagaaa aagaagcttg gaggtcaaga catctttatg acagaagaac 4740
   agaagaaata ctataatgca atgaaaaagc tggggtccaa gaagccacaa aagccaattc 4800
   ctcgaccagg gaacaaaatc caaggatgta tatttgacct agtgacaaat caagcctttg 4860
   atattagtat catggttctt atctgtctca acatggtaac catgatggta gaaaaggagg 4920
   gtcaaagtca acatatgact gaagttttat attggataaa tgtggttttt ataatccttt 4980
   tcactggaga atgtgtgcta aaactgatct ccctcagaca ctactacttc actgtaggat 5040
   ggaatatttt tgattttgtg gttgtgatta tctccattgt aggtatgttt ctagctgatt 5100
   tgattgaaac gtattttgtg tcccctaccc tgttccgagt gatccgtctt gccaggattg 5160
   gccgaatcct acgtctagtc aaaggagcaa aggggatccg cacgctgctc tttgctttga 5220
   tgatgtccct tcctgcgttg tttaacatcg gcctcctgct cttcctggtc atgttcatct 5280
   acgccatctt tggaatgtcc aactttgcct atgttaaaaa ggaagatgga attaatgaca 5340
   tgttcaattt tgagaccttt ggcaacagta tgatttgcct gttccaaatt acaacctctg 5400
   ctggctggga tggattgcta gcacctattc ttaacagtaa gccacccgac tgtgacccaa 5460
   aaaaagttca tcctggaagt tcagttgaag gagactgtgg taacccatct gttggaatat 5520
   tctactttgt tagttatatc atcatatcct tcctggttgt ggtgaacatg tacattgcag 5580
   tcatactgga gaattttagt gttgccactg aagaaagtac tgaacctctg agtgaggatg 5640
   actttgagat gttctatgag gtttgggaga agtttgatcc cgatgcgacc cagtttatag 5700
   agttctctaa actctctgat tttgcagctg ccctggatcc tcctcttctc atagcaaaac 5760
   ccaacaaagt ccagctcatt gccatggatc tgcccatggt tagtggtgac cggatccatt 5820
   gtcttgacat cttatttgct tttacaaagc gtgttttggg tgagagtggg gagatggatt 5880
   ctcttcgttc acagatggaa gaaaggttca tgtctgcaaa tccttccaaa gtgtcctatg 5940
   aacccatcac aaccacacta aaacggaaac aagaggatgt gtctgctact gtcattcagc 6000
   gtgcttatag acgttaccgc ttaaggcaaa atgtcaaaaa tatatcaagt atatacataa 6060
   aagatggaga cagagatgat gatttactca ataaaaaaga tatggctttt gataatgtta 6120
   atgagaactc aagtccagaa aaaacagatg ccacttcatc caccacctct ccaccttcat 6180
   atgatagtgt aacaaagcca gacaaagaga aatatgaaca agacagaaca gaaaaggaag 6240
   acaaagggaa agacagcaag gaaagcaaaa aatagagctt catttttgat atattgttta 6300
   cagcctgtga aagtgattta tttgtgttaa taaaactctt ttgaggaagt ctatgccaaa 6360
   atccttttta tcaaaatatt ctcgaaggca gtgcagtcac taactctgat ttcctaagaa 6420
   aggtgggcag cattagcaga tggttatttt tgcactgatg attctttaag aatcgtaaga 6480
   gaactctgta ggaattattg attatagcat acaaaagtga ttcagttttt tggtttttaa 6540
   taaatcagaa gaccatgtag aaaactttta catctgcctt gtcatctttt cacaggattg 6600
   taattagtct tgtttcccat gtaaataaac aacacacgca tacagaaaaa tctattattt 6660
   atctattatt tggaaatcaa caaaagtatt tgccttggct ttgcaatgaa atgcttgata 6720
   gaagtaatgg acattagtta tgaatgttta gttaaaatgc attattaggg agcttgactt 6780
   tttatcaatg tacagaggtt attctatatt ttgaggtgct taaatttatt ctacattgca 6840
   tcagaaccaa tttatatgtg cctataaaat gccatgggat taaaaatata tgtaggctat 6900
   tcatttctac aaatgttttt cattcatctt gactcacatg ccaacaagga taagacttac 6960
   ctttagagta ttgtgtttca tagcctttct tctttcatat ccctttttgt tcatagaata 7020
   accacagaac ttgaaaaatt attctaagta catattacac tcctcaaaaa aaacaaagat 7080
   aactgagaaa aaagttattg acagaagttc tatttgctat tatttacata gcctaacatt 7140
   tgactgtgct gcccaaaata ctgataatag tctcttaaac tcttttgtca aattttcctg 7200
   ctttcttatg cagtattgtt tagtcatcct ttcgctgtaa gcaaagttga tgaaatcctt 7260
   cctgatatgc agttagttgt ttgaccacgg tacatacttg agcagataat aacttgggca 7320
   cagtatttat tgcatcactt gtatacaatc ccgtgtttgg caagctttca aatcatgtaa 7380
   tatgacagac tttacacaga tatgtgttta gtatgaataa aaaagcattg aaatagggat 7440
   tcttgccaac ttgctctctt gccaccaact tactttccta aattatggaa gtaatctttt 7500
   ttggatatac ttcaatgtat acaatgagga agatgtcacc ttctccttaa aattctatga 7560
   tgtgaaatat attttgcctc aatcaacaca gtaccatggg cttctaattt atcaagcaca 7620
   tattcatttt gcattagctg tagacatcta gttttttgaa aacacctatt aatagtaatt 7680
   tgaaaagaaa taaccataat gctttttttc gtgagtttat ttcaggaata tgagatcttt 7740
   cttctataaa gttattcatg cacaggcaaa aattgagcta cacaggtaga atgtagtttt 7800
   acttagaaga tttttgtggg aggttttgaa gcaaatatat aaaacaactt tcactaattt 7860
   gctttccata tttaaaaaat aataaattac atttatataa taaatgttta aagcacatat 7920
   tttttgttgt tctggcaatt taaaaagaaa gaggatttaa acgtacctat agaaacaaag 7980
   atttatggtt aaagaatgag atcagaagtc tagaatgttt ttaaattgtg atatatttta 8040
   caacatccgt tattactttg agacatttgt cctaatctac gtataaaact caatctaggg 8100
   ctaaagattc tttataccat cttaggttca ttcatcttag gctatttgaa ccacttttta 8160
   atttaatatg aaagacacca tgcagtgttt tccgagacta catagatcat tttatcacat 8220
   acctaccaag cctgttggaa ataggttttg ataatttaag tagggaccta tacaaaatat 8280
   attacattta tcagattttt aaatacattc aattaagaat ttaacatcac cttaaatttg 8340
   aattcaatct accgttattt caaactcaca aatataactg cattatgaat acttacataa 8400
   tgtagtaaga caagatgttt gacaggttcg tgtgtaattt tctattaatg tttttacatt 8460
   gccttgtttt tatgtaaaat aaaaaatatg ggcaactggt ttgttaacaa cacaatttct 8520
   tcttagcatt tcaaaaatat atataaagtt gttctttttc ctatttcatg aactatgttt 8580
   ttttttaaaa taacatggtt aagttttata tatatttacg tttgtttcag gaatgtctac 8640
   ttgtgacttt ttatcaatta aaaataatat ttggaagaaa gagcttatta agtataagct 8700
   tgaagtaaaa ttagacctct ctttccatgt agattactgt ttgtactgat ggtttcaccc 8760
   ttcagaaggc actgtcatat taatatttaa attttataat cgctgaactt attacaccca 8820
   acaatacaga aaggcagtta cactgaagaa cttaacttag aataaaatgg aagcaaacag 8880
   gttttctaaa aactttttta agtgaccagg tctcgctctg tcacccaggc tagagtgcaa 8940
   tggcatgatc atagctctct gcagcctcaa ctctgggctc aagcaaccct cctgcctcag 9000
   cctcccaagt agctaagact acaggtacat gccaccatgc ctggctaata tttaaatttt 9060
   tgtagataag gggtcttgct atgttgccca ggctagtctc aaactcctgg cttcaagtgt 9120
   tcctactgtc atgacctgcc aacatgctgg ggttacaggc atgagccacc atgccccaaa 9180
   caggtttgaa cacaaatctt tcggatgaaa attagagaac ctaattttag ctttttgata 9240
   gttacctagt ttgcaaaaga tttgggtgac ttgtgagctg tttttaaatg ctgattgttg 9300
   aacatcacaa cccaaaatac ttagcatgat tttatagagt tttgatagct ttattaaaaa 9360
   gagtgaaaat aaaatgcata tgtaaataaa gcagttctaa atagctattt cagagaaatg 9420
   ttaatagaag tgctgaaaga agggccaact aaattaggat ggccagggaa ttggcctggg 9480
   tttaggacct atgtatgaag gccaccaatt ttttaaaaat atctgtggtt tattatgtta 9540
   ttatcttctt gaggaaaaca atcaagaatt gcttcatgaa aataaataaa tagccatgaa 9600
   tatcataaag ctgtttacat aggattcttt acaaatttca tagatctatg aatgctcaaa 9660
   atgtttgagt ttgccataaa ttatattgta gttatattgt agttatactt gagactgaca 9720
   cattgtaata taatctaaga ataaaagtta tacaaaataa aaaaaaaaaa a          9771
<210> 43
   <211> 1977
   <212> PRT
   <213> Artificial Sequence
<220>
   <223> Synthetic
<400> 43
   Met Ala Met Leu Pro Pro Pro Gly Pro Gln Ser Phe Val His Phe Thr
    1               5                  10                  15
   Lys Gln Ser Leu Ala Leu Ile Glu Gln Arg Ile Ala Glu Arg Lys Ser
               20                  25                  30
   Lys Glu Pro Lys Glu Glu Lys Lys Asp Asp Asp Glu Glu Ala Pro Lys
           35                  40                  45
   Pro Ser Ser Asp Leu Glu Ala Gly Lys Gln Leu Pro Phe Ile Tyr Gly
       50                  55                  60
   Asp Ile Pro Pro Gly Met Val Ser Glu Pro Leu Glu Asp Leu Asp Pro
   65                  70                  75                  80
   Tyr Tyr Ala Asp Lys Lys Thr Phe Ile Val Leu Asn Lys Gly Lys Thr
                   85                  90                  95
   Ile Phe Arg Phe Asn Ala Thr Pro Ala Leu Tyr Met Leu Ser Pro Phe
               100                 105                 110
   Ser Pro Leu Arg Arg Ile Ser Ile Lys Ile Leu Val His Ser Leu Phe
           115                 120                 125
   Ser Met Leu Ile Met Cys Thr Ile Leu Thr Asn Cys Ile Phe Met Thr
       130                 135                 140
   Met Asn Asn Pro Pro Asp Trp Thr Lys Asn Val Glu Tyr Thr Phe Thr
   145                 150                 155                 160
   Gly Ile Tyr Thr Phe Glu Ser Leu Val Lys Ile Leu Ala Arg Gly Phe
                   165                 170                 175
   Cys Val Gly Glu Phe Thr Phe Leu Arg Asp Pro Trp Asn Trp Leu Asp
               180                 185                 190
   Phe Val Val Ile Val Phe Ala Tyr Leu Thr Glu Phe Val Asn Leu Gly
           195                 200                 205
   Asn Val Ser Ala Leu Arg Thr Phe Arg Val Leu Arg Ala Leu Lys Thr
       210                 215                 220
   Ile Ser Val Ile Pro Gly Leu Lys Thr Ile Val Gly Ala Leu Ile Gln
   225                 230                 235                 240
   Ser Val Lys Lys Leu Ser Asp Val Met Ile Leu Thr Val Phe Cys Leu
                   245                 250                 255
   Ser Val Phe Ala Leu Ile Gly Leu Gln Leu Phe Met Gly Asn Leu Lys
               260                 265                 270
   His Lys Cys Phe Arg Asn Ser Leu Glu Asn Asn Glu Thr Leu Glu Ser
           275                 280                 285
   Ile Met Asn Thr Leu Glu Ser Glu Glu Asp Phe Arg Lys Tyr Phe Tyr
       290                 295                 300
   Tyr Leu Glu Gly Ser Lys Asp Ala Leu Leu Cys Gly Phe Ser Thr Asp
   305                 310                 315                 320
   Ser Gly Gln Cys Pro Glu Gly Tyr Thr Cys Val Lys Ile Gly Arg Asn
                   325                 330                 335
   Pro Asp Tyr Gly Tyr Thr Ser Phe Asp Thr Phe Ser Trp Ala Phe Leu
               340                 345                 350
   Ala Leu Phe Arg Leu Met Thr Gln Asp Tyr Trp Glu Asn Leu Tyr Gln
           355                 360                 365
   Gln Thr Leu Arg Ala Ala Gly Lys Thr Tyr Met Ile Phe Phe Val Val
       370                 375                 380
   Val Ile Phe Leu Gly Ser Phe Tyr Leu Ile Asn Leu Ile Leu Ala Val
   385                 390                 395                 400
   Val Ala Met Ala Tyr Glu Glu Gln Asn Gln Ala Asn Ile Glu Glu Ala
                   405                 410                 415
   Lys Gln Lys Glu Leu Glu Phe Gln Gln Met Leu Asp Arg Leu Lys Lys
               420                 425                 430
   Glu Gln Glu Glu Ala Glu Ala Ile Ala Ala Ala Ala Ala Glu Tyr Thr
           435                 440                 445
   Ser Ile Arg Arg Ser Arg Ile Met Gly Leu Ser Glu Ser Ser Ser Glu
       450                 455                 460
   Thr Ser Lys Leu Ser Ser Lys Ser Ala Lys Glu Arg Arg Asn Arg Arg
   465                 470                 475                 480
   Lys Lys Lys Asn Gln Lys Lys Leu Ser Ser Gly Glu Glu Lys Gly Asp
                   485                 490                 495
   Ala Glu Lys Leu Ser Lys Ser Glu Ser Glu Asp Ser Ile Arg Arg Lys
               500                 505                 510
   Ser Phe His Leu Gly Val Glu Gly His Arg Arg Ala His Glu Lys Arg
           515                 520                 525
   Leu Ser Thr Pro Asn Gln Ser Pro Leu Ser Ile Arg Gly Ser Leu Phe
       530                 535                 540
   Ser Ala Arg Arg Ser Ser Arg Thr Ser Leu Phe Ser Phe Lys Gly Arg
   545                 550                 555                 560
   Gly Arg Asp Ile Gly Ser Glu Thr Glu Phe Ala Asp Asp Glu His Ser
                   565                 570                 575
   Ile Phe Gly Asp Asn Glu Ser Arg Arg Gly Ser Leu Phe Val Pro His
               580                 585                 590
   Arg Pro Gln Glu Arg Arg Ser Ser Asn Ile Ser Gln Ala Ser Arg Ser
           595                 600                 605
   Pro Pro Met Leu Pro Val Asn Gly Lys Met His Ser Ala Val Asp Cys
       610                 615                 620
   Asn Gly Val Val Ser Leu Val Asp Gly Arg Ser Ala Leu Met Leu Pro
   625                 630                 635                 640
   Asn Gly Gln Leu Leu Pro Glu Gly Thr Thr Asn Gln Ile His Lys Lys
                   645                 650                 655
   Arg Arg Cys Ser Ser Tyr Leu Leu Ser Glu Asp Met Leu Asn Asp Pro
   660 665 670
   Asn Leu Arg Gln Arg Ala Met Ser Arg Ala Ser Ile Leu Thr Asn Thr
   675 680 685
   Val Glu Glu Leu Glu Glu Ser Arg Gln Lys Cys Pro Pro Trp Trp Tyr
   690 695 700
   Arg Phe Ala His Lys Phe Leu Ile Trp Asn Cys Ser Pro Tyr Trp Ile
   705 710 715 720
   Lys Phe Lys Lys Cys Ile Tyr Phe Ile Val Met Asp Pro Phe Val Asp
   725 730 735
   Leu Ala Ile Thr Ile Cys Ile Val Leu Asn Thr Leu Phe Met Ala Met
   740 745 750
   Glu His His Pro Met Thr Glu Glu Phe Lys Asn Val Leu Ala Ile Gly
   755 760 765
   Asn Leu Val Phe Thr Gly Ile Phe Ala Ala Glu Met Val Leu Lys Leu
   770 775 780
   Ile Ala Met Asp Pro Tyr Glu Tyr Phe Gln Val Gly Trp Asn Ile Phe
   785 790 795 800
   Asp Ser Leu Ile Val Thr Leu Ser Leu Val Glu Leu Phe Leu Ala Asp
   805 810 815
   Val Glu Gly Leu Ser Val Leu Arg Ser Phe Arg Leu Leu Arg Val Phe
   820 825 830
   Lys Leu Ala Lys Ser Trp Pro Thr Leu Asn Met Leu Ile Lys Ile Ile
   835 840 845
   Gly Asn Ser Val Gly Ala Leu Gly Asn Leu Thr Leu Val Leu Ala Ile
   850 855 860
   Ile Val Phe Ile Phe Ala Val Val Gly Met Gln Leu Phe Gly Lys Ser
   865 870 875 880
   Tyr Lys Glu Cys Val Cys Lys Ile Asn Asp Asp Cys Thr Leu Pro Arg
   885 890 895
   Trp His Met Asn Asp Phe Phe His Ser Phe Leu Ile Val Phe Arg Val
   900 905 910
   Leu Cys Gly Glu Trp Ile Glu Thr Met Trp Asp Cys Met Glu Val Ala
   915 920 925
   Gly Gln Ala Met Cys Leu Ile Val Tyr Met Met Val Met Val Ile Gly
   930 935 940
   Asn Leu Val Val Leu Asn Leu Phe Leu Ala Leu Leu Leu Ser Ser Phe
   945 950 955 960
   Ser Ser Asp Asn Leu Thr Ala Ile Glu Glu Asp Pro Asp Ala Asn Asn
   965 970 975
   Leu Gln Ile Ala Val Thr Arg Ile Lys Lys Gly Ile Asn Tyr Val Lys
   980 985 990
   Gln Thr Leu Arg Glu Phe Ile Leu Lys Ala Phe Ser Lys Lys Pro Lys
   995 1000 1005
   Ile Ser Arg Glu Ile Arg Gln Ala Glu Asp Leu Asn Thr Lys Lys Glu
   1010 1015 1020
   Asn Tyr Ile Ser Asn His Thr Leu Ala Glu Met Ser Lys Gly His Asn
   1025 1030 1035 1040
   Phe Leu Lys Glu Lys Asp Lys Ile Ser Gly Phe Gly Ser Ser Val Asp
   1045 1050 1055
   Lys His Leu Met Glu Asp Ser Asp Gly Gln Ser Phe Ile His Asn Pro
   1060 1065 1070
   Ser Leu Thr Val Thr Val Pro Ile Ala Pro Gly Glu Ser Asp Leu Glu
   1075 1080 1085
   Asn Met Asn Ala Glu Glu Leu Ser Ser Asp Ser Asp Ser Glu Tyr Ser
   1090 1095 1100
   Lys Val Arg Leu Asn Arg Ser Ser Ser Ser Glu Cys Ser Thr Val Asp
   1105 1110 1115 1120
   Asn Pro Leu Pro Gly Glu Gly Glu Glu Ala Glu Ala Glu Pro Met Asn
   1125 1130 1135
   Ser Asp Glu Pro Glu Ala Cys Phe Thr Asp Gly Cys Val Arg Arg Phe
   1140 1145 1150
   Ser Cys Cys Gln Val Asn Ile Glu Ser Gly Lys Gly Lys Ile Trp Trp
           1155                1160                1165
   Asn Ile Arg Lys Thr Cys Tyr Lys Ile Val Glu His Ser Trp Phe Glu
       1170                1175                1180
   Ser Phe Ile Val Leu Met Ile Leu Leu Ser Ser Gly Ala Leu Ala Phe
   1185                1190                1195                1200
   Glu Asp Ile Tyr Ile Glu Arg Lys Lys Thr Ile Lys Ile Ile Leu Glu
                   1205                1210                1215
   Tyr Ala Asp Lys Ile Phe Thr Tyr Ile Phe Ile Leu Glu Met Leu Leu
               1220                1225                1230
   Lys Trp Ile Ala Tyr Gly Tyr Lys Thr Tyr Phe Thr Asn Ala Trp Cys
           1235                1240                1245
   Trp Leu Asp Phe Leu Ile Val Asp Val Ser Leu Val Thr Leu Val Ala
       1250                1255                1260
   Asn Thr Leu Gly Tyr Ser Asp Leu Gly Pro Ile Lys Ser Leu Arg Thr
   1265                1270                1275                1280
   Leu Arg Ala Leu Arg Pro Leu Arg Ala Leu Ser Arg Phe Glu Gly Met
                   1285                1290                1295
   Arg Val Val Val Asn Ala Leu Ile Gly Ala Ile Pro Ser Ile Met Asn
               1300                1305                1310
   Val Leu Leu Val Cys Leu Ile Phe Trp Leu Ile Phe Ser Ile Met Gly
           1315                1320                1325
   Val Asn Leu Phe Ala Gly Lys Phe Tyr Glu Cys Ile Asn Thr Thr Asp
       1330                1335                1340
   Gly Ser Arg Phe Pro Ala Ser Gln Val Pro Asn Arg Ser Glu Cys Phe
   1345                1350                1355                1360
   Ala Leu Met Asn Val Ser Gln Asn Val Arg Trp Lys Asn Leu Lys Val
                   1365                1370                1375
   Asn Phe Asp Asn Val Gly Leu Gly Tyr Leu Ser Leu Leu Gln Val Ala
               1380                1385                1390
   Thr Phe Lys Gly Trp Thr Ile Ile Met Tyr Ala Ala Val Asp Ser Val
           1395                1400                1405
   Asn Val Asp Lys Gln Pro Lys Tyr Glu Tyr Ser Leu Tyr Met Tyr Ile
       1410                1415                1420
   Tyr Phe Val Val Phe Ile Ile Phe Gly Ser Phe Phe Thr Leu Asn Leu
   1425                1430                1435                1440
   Phe Ile Gly Val Ile Ile Asp Asn Phe Asn Gln Gln Lys Lys Lys Leu
                   1445                1450                1455
   Gly Gly Gln Asp Ile Phe Met Thr Glu Glu Gln Lys Lys Tyr Tyr Asn
               1460                1465                1470
   Ala Met Lys Lys Leu Gly Ser Lys Lys Pro Gln Lys Pro Ile Pro Arg
           1475                1480                1485
   Pro Gly Asn Lys Ile Gln Gly Cys Ile Phe Asp Leu Val Thr Asn Gln
       1490                1495                1500
   Ala Phe Asp Ile Ser Ile Met Val Leu Ile Cys Leu Asn Met Val Thr
   1505                1510                1515                1520
   Met Met Val Glu Lys Glu Gly Gln Ser Gln His Met Thr Glu Val Leu
                   1525                1530                1535
   Tyr Trp Ile Asn Val Val Phe Ile Ile Leu Phe Thr Gly Glu Cys Val
               1540                1545                1550
   Leu Lys Leu Ile Ser Leu Arg His Tyr Tyr Phe Thr Val Gly Trp Asn
           1555                1560                1565
   Ile Phe Asp Phe Val Val Val Ile Ile Ser Ile Val Gly Met Phe Leu
       1570                1575                1580
   Ala Asp Leu Ile Glu Thr Tyr Phe Val Ser Pro Thr Leu Phe Arg Val
   1585                1590                1595                1600
   Ile Arg Leu Ala Arg Ile Gly Arg Ile Leu Arg Leu Val Lys Gly Ala
                   1605                1610                1615
   Lys Gly Ile Arg Thr Leu Leu Phe Ala Leu Met Met Ser Leu Pro Ala
               1620                1625                1630
   Leu Phe Asn Ile Gly Leu Leu Leu Phe Leu Val Met Phe Ile Tyr Ala
           1635                1640                1645
   Ile Phe Gly Met Ser Asn Phe Ala Tyr Val Lys Lys Glu Asp Gly Ile
       1650                1655                1660
   Asn Asp Met Phe Asn Phe Glu Thr Phe Gly Asn Ser Met Ile Cys Leu
   1665                1670                1675                1680
   Phe Gln Ile Thr Thr Ser Ala Gly Trp Asp Gly Leu Leu Ala Pro Ile
                   1685                1690                1695
   Leu Asn Ser Lys Pro Pro Asp Cys Asp Pro Lys Lys Val His Pro Gly
               1700                1705                1710
   Ser Ser Val Glu Gly Asp Cys Gly Asn Pro Ser Val Gly Ile Phe Tyr
           1715                1720                1725
   Phe Val Ser Tyr Ile Ile Ile Ser Phe Leu Val Val Val Asn Met Tyr
       1730                1735                1740
   Ile Ala Val Ile Leu Glu Asn Phe Ser Val Ala Thr Glu Glu Ser Thr
   1745                1750                1755                1760
   Glu Pro Leu Ser Glu Asp Asp Phe Glu Met Phe Tyr Glu Val Trp Glu
                   1765                1770                1775
   Lys Phe Asp Pro Asp Ala Thr Gln Phe Ile Glu Phe Ser Lys Leu Ser
               1780                1785                1790
   Asp Phe Ala Ala Ala Leu Asp Pro Pro Leu Leu Ile Ala Lys Pro Asn
           1795                1800                1805
   Lys Val Gln Leu Ile Ala Met Asp Leu Pro Met Val Ser Gly Asp Arg
       1810                1815                1820
   Ile His Cys Leu Asp Ile Leu Phe Ala Phe Thr Lys Arg Val Leu Gly
   1825                1830                1835                1840
   Glu Ser Gly Glu Met Asp Ser Leu Arg Ser Gln Met Glu Glu Arg Phe
                   1845                1850                1855
   Met Ser Ala Asn Pro Ser Lys Val Ser Tyr Glu Pro Ile Thr Thr Thr
               1860                1865                1870
   Leu Lys Arg Lys Gln Glu Asp Val Ser Ala Thr Val Ile Gln Arg Ala
           1875                1880                1885
   Tyr Arg Arg Tyr Arg Leu Arg Gln Asn Val Lys Asn Ile Ser Ser Ile
       1890                1895                1900
   Tyr Ile Lys Asp Gly Asp Arg Asp Asp Asp Leu Leu Asn Lys Lys Asp
   1905                1910                1915                1920
   Met Ala Phe Asp Asn Val Asn Glu Asn Ser Ser Pro Glu Lys Thr Asp
                   1925                1930                1935
   Ala Thr Ser Ser Thr Thr Ser Pro Pro Ser Tyr Asp Ser Val Thr Lys
               1940                1945                1950
   Pro Asp Lys Glu Lys Tyr Glu Gln Asp Arg Thr Glu Lys Glu Asp Lys
           1955                1960                1965
   Gly Lys Asp Ser Lys Glu Ser Lys Lys
       1970                1975

## Description

This application claims priority to U.S. Provisional Patent Application No. 61/485,488, filed 12 May 2011, U.S. Application Serial No. 13/155,491, filed on 08 June 2011, and U.S. Application Serial No. 13/236,117 filed on 19 September 2011; each application of which is herein specifically incorporated by reference in its entirety.

### FIELD OF INVENTION

Genetically modified non-human animals are provided that express human voltage-gated sodium (Naᵥ) channels or fragments thereof, in particular Naᵥ1.7 (*Scn9A*). Genetically modified mice useful for the identification and testing of antagonists for treating chronic pain states or disorders associated with aberrant Naᵥ1.7 activity and/or function are provided. Methods for making genetically modified non-human animals that express human Naᵥ1.7 protein, and, alternatively, that express a partially human Naᵥ1.7 protein, are provided. Non-human animals are provided that do not express an endogenous Naᵥ1.7 protein.

### BACKGROUND

Sodium channels are integral membrane proteins that form ion channels in the plasma membrane of excitable cells. They are classified as voltage-gated sodium (Naᵥ) channels, which permit the influx of Na⁺ ions that mediate action potentials in excitable cells; and ligand-gated sodium channels, which bind a ligand that triggers the influx of ions leading to similar action potentials.

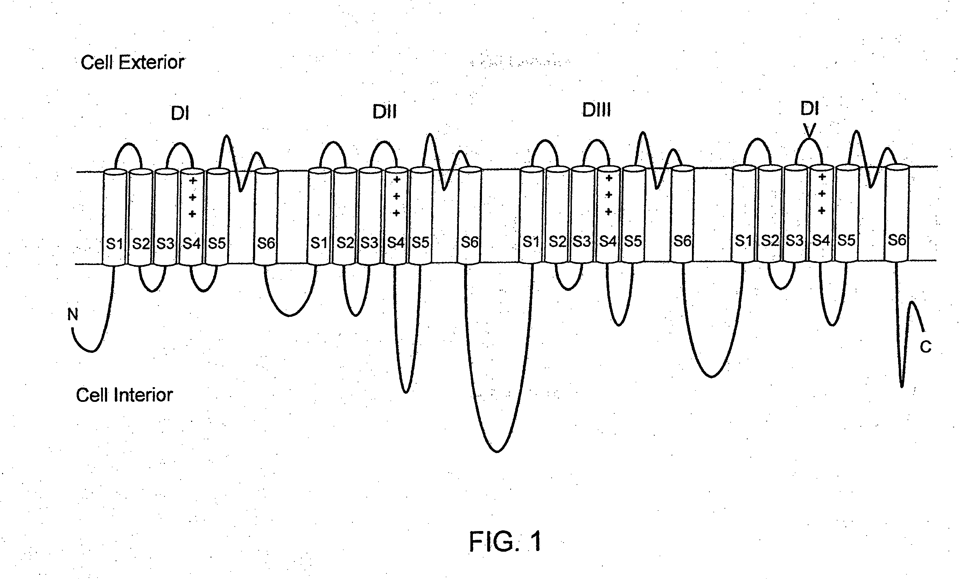

Naᵥ channels, like calcium and potassium channels, are composed of a very large and complex α-subunit on the surface of the cell which includes four domains (DI-DIV), each with six transmembrane α-helix segments (S1-S6) and including a pore that allows the influx of Na⁺ ions into the cell (FIG. 1; see also Clare 2010 Expert Opin. Investig. Drugs 19(1): 45-62). For Naᵥ channels, a single gene encodes all of these domains. Transmembrane segment 4 (S4) within each domain of Naᵥ channels contains positively charged amino acids (FIG. 1) that act as a voltage sensor. The intracellular loop that connects Domains III and IV contains sequences that are reportedly involved in inactivation. Naᵥ channels interact with other proteins on the cell surface termed β-subunits, which are involved in channel kinetics and voltage-dependent gating functions. Naᵥ channels reportedly exhibit diverse functional properties and distinct expression patterns, which imply specialized functions among the channels and predisposes some for roles in transmitting specific signals, for example, pain signals.

In spite of many efforts to elucidate the properties and functions of human Naᵥ channels, the large size and complex nature of their structure makes it difficult to study the global aspects of their biological activity and their involvement in the pain response. This difficulty is increased by the fact that global deletion is lethal; *Scn9A*^{*-*/*-*} pups die shortly after birth, apparently due to a failure to feed. Therefore, there is a need in the art for compositions and methods that supplement and enhance current *in vitro* systems (for example, *in vitro*-transfected cells containing constructs expressing human Naᵥ channels in culture) by employing more biologically sensible approaches to making non-human animals and cells that include whole human Naᵥ channels or chimeric Naᵥ channels containing specific human fragments associated with Naᵥ channel activation and that can function in facilitating the pain response.

### SUMMARY OF INVENTION

Genetically engineered non-human animals, tissues, and cells are provided that express a human Naᵥ α-subunit, or a functional fragment thereof, on the surface of a cell. In various embodiments, the Naᵥ α-subunit is a Naᵥ1.7 α-subunit.

In one aspect, genetically engineered non-human animals are provided that express a Naᵥ1.7 α-subunit on the surface of a cell provide an *in vivo* system to identify antagonists of the channel, and to identify therapeutic agents for the treatment of pain disorders or syndromes, such as, for example, chronic pain, erythromelalgia (IEM) and paroxysmal extreme pain disorder (PEPD).

In one aspect, genetically engineered non-human animals are provided that express a Naᵥ1.7 α-subunit on the surface of a cell and provide a system to selectively test efficacy and toxicity of a therapeutic on mutant or variant forms of human Naᵥ1.7. In one embodiment, the therapeutic agent is a compound that functions as a sodium channel blocker. In a specific embodiment, the compound is a synthetic compound. In one embodiment, the synthetic compound is selected from lidocaine, mexiletine, carbamazepine, amitryptiline and biphenyl pyrazoles, or a combination thereof. In another embodiment, compound is a toxin. In a specific embodiment, the toxin is selected from tetrodotoxin and neosaxitosin or a combination thereof.

In one embodiment, genetically engineered non-human animals are provided that express a Naᵥ1.7 α-subunit on the surface of a cell and provide a system to selectively test functionality (*e.g*., efficacy) and/or toxicity of combinations of therapeutic agents on mutant or variant forms of a human Naᵥ1.7. In one embodiment, the combination of therapeutic agents comprises provide a synergistic effect upon administration to the genetically engineered non-human animal. In a specific embodiment, the combination of therapeutic agents comprises at least two of a synthetic compound, a naturally occurring toxin, or a protein (*e.g*., an anti-Naᵥ antibody).

In one aspect, genetically engineered mice are provided that express a human Naᵥ channel protein, specifically a human Naᵥ1.7 α-subunit. The mice are genetically engineered to include all or substantially all of a human Naᵥ1.7 gene.

In one embodiment, the human Naᵥ1.7 gene replaces an endogenous mouse Naᵥ1.7 gene at the endogenous mouse Naᵥ1.7 locus.

In one aspect, genetically engineered mice are provided that express a chimeric Naᵥ1.7 α-subunit, wherein the mice include a mouse Naᵥ1.7 α-subunit engineered with one or more extracellular pore loops containing a corresponding sequence from a human Naᵥ1.7 gene.

In one embodiment, the chimeric Naᵥ1.7 α-subunit comprises an extracellular pore loop connecting transmembrane segments 5 and 6 of Domain I that comprises the corresponding sequence from the human Naᵥ1.7 gene. In another embodiment, the chimeric Naᵥ1.7 α-subunit comprises an extracellular pore loop connecting transmembrane segments 5 and 6 of Domain III that comprises the corresponding sequence from the human Naᵥ1.7 gene.

In one aspect, a genetically engineered mouse is provided that comprises substantially all of the human genomic DNA that encodes a Naᵥ1.7 protein. In another aspect, the genetically modified mouse comprises a portion of human genomic DNA, and the mouse expresses a chimeric Naᵥ1.7 protein.

In one embodiment, the portion of human genomic DNA comprises human sequence that encodes the extracellular pore loop connecting transmembrane segments 5 and 6 of Domain I of the human Naᵥ1.7 gene. In another embodiment, the portion of human genomic DNA comprises human sequence that encodes the extracellular pore loop connecting transmembrane segments 5 and 6 of Domain III of the human Naᵥ1.7 gene.

In one aspect, a genetically engineered mouse is provided that is capable of expressing a human or a chimeric Naᵥ1.7 protein on the surface of a cell of the mouse.

In one embodiment, the Naᵥ1.7 is chimeric and comprises a human extracellular pore loop. In a specific embodiment, the human extracellular pore loop is the loop connecting transmembrane segments 5 and 6 of Domain I. In another specific embodiment, the human extracellular pore loop is the loop connecting transmembrane segments 5 and 6 of Domain III.

In one embodiment, the cell is an excitable cell. In another embodiment the cell is a non-excitable cell. In a specific embodiment, the cell is a neuron. In a specific embodiment, the cell is a dorsal root ganglion (DRG) neuron. In another specific embodiment, the cell is a sympathetic ganglion neuron.

In one embodiment, the human or chimeric Naᵥ1.7 gene is operably linked to a human or mouse leader sequence. In one embodiment, the leader sequence is a mouse leader sequence.

In one embodiment, the human or chimeric Naᵥ1.7 gene is operably linked to a human or mouse promoter. In a specific embodiment, the promoter is an endogenous mouse Naᵥ1.7 gene promoter.

In one embodiment, the genetically modified mouse comprises a human Naᵥ1.7 gene locus that encodes a human Naᵥ1.7 protein. In another embodiment, the genetically modified mouse comprises a chimeric Naᵥ1.7 gene locus that comprises a human sequence that encodes an extracellular pore loop that is substantially human. In a specific embodiment, the human sequence encodes an extracellular pore loop connecting transmembrane segments 5 and 6 of Domain I of the chimeric Naᵥ1.7 protein. In another specific embodiment, the human sequence encodes an extracellular pore loop connecting transmembrane segments 5 and 6 of Domain III of the chimeric Naᵥ1.7 protein.

In one embodiment, the Naᵥ1.7 gene locus comprises a human genomic fragment comprising about 113 kb of DNA that encodes a human Naᵥ1.7 protein. In a specific embodiment, the Naᵥ1.7 gene locus comprises exons 2 to 28 of a human Naᵥ1.7 gene.

In another embodiment, the Naᵥ1.7 gene locus comprises a nucleic acid sequence of a human Naᵥ1.7 gene locus comprising about 10 kb of DNA that encodes an extracellular pore loop of a human Naᵥ1.7 protein. In a specific embodiment, the nucleic acid sequence comprises exons 7 to 9 of a human Naᵥ1.7 gene. In specific embodiment, the extracellular pore loop is the loop connecting transmembrane segments 5 and 6 of Domain I of a human Naᵥ1.7 protein.

In another embodiment, the Naᵥ1.7 gene locus comprises a human genomic nucleic acid sequence comprising about 2.8 kb of DNA that encodes an extracellular pore loop of a human Naᵥ1.7 protein. In a specific embodiment, the human genomic nucleic acid sequence comprises exons 23 to 25 of a human Naᵥ1.7 gene. In a specific embodiment, the extracellular pore loop is the loop connecting transmembrane segments 5 and 6 of Domain III of a human Naᵥ1.7 protein.

In one embodiment, the genetically modified mouse is capable of expressing a fully human Naᵥ1.7 protein. In another embodiment, the genetically modified mouse is capable of expressing a partially human Naᵥ1.7 protein. In a specific embodiment, the genetically modified mouse is capable of expressing a chimeric Naᵥ1.7 protein comprising an extracellular sequence from a human Naᵥ1.7 protein.

In one embodiment, the partially human Naᵥ1.7 protein comprises an extracellular pore loop that contains a human sequence. In a specific embodiment, the extracellular pore loop is selected from the group consisting of the loop connecting transmembrane segments 5 and 6 of Domain I, and the loop connecting transmembrane segments 5 and 6 of Domain III. In a specific embodiment, the human extracellular pore loop is the loop connecting transmembrane segments 5 and 6 of Domain I. In another embodiment, the human extracellular pore loop is the loop connecting transmembrane segments 5 and 6 of Domain III.

In one embodiment, the mouse comprises a cell that expresses a human Naᵥ1.7 protein. In another embodiment, the mouse comprises a cell that expresses a chimeric Naᵥ1.7 protein that comprises one or more human extracellular pore loops. In a specific embodiment, the human extracellular pore loops are selected from the group consisting of the loop connecting transmembrane segments 5 and 6 of Domain I, the loop connecting transmembrane segments 5 and 6 of Domain III, and a combination thereof. In a specific embodiment, the human extracellular pore loop is the loop connecting transmembrane segments 5 and 6 of Domain I. In another embodiment, the human extracellular pore loop is the loop connecting transmembrane segments 5 and 6 of Domain III. In one embodiment, the cell is an excitable cell. In another embodiment the cell is a non-excitable cell. In a specific embodiment, the cell is a neuron. In a specific embodiment, the neuron is a DRG neuron. In another specific embodiment, the neuron is a sympathetic ganglion neuron.

In one embodiment, the mouse comprises a combination of one or more embodiments and/or aspects described in this disclosure.

In one embodiment, the genetically modified mouse is a C57BL strain, in a specific embodiment selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, C57BL/Ola. In a specific embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain.

In one aspect, a mouse cell is provided that is isolated from a mouse as described herein. In one embodiment, the cell is an ES cell. In one embodiment, the cell is an excitable cell. In another embodiment, the cell is a non-excitable cell. In one embodiment, the cell is a neuron. In a specific embodiment, the neuron is a DRG neuron. In another specific embodiment, the neuron is a sympathetic ganglion neuron.

In one aspect, a cell is provided; wherein the cell bears a Naᵥ1.7 protein that comprises a human sequence corresponding to an extracellular pore loop of the Naᵥ1.7 channel protein.

In one embodiment, the cell is a neuronal cell. In a specific embodiment, the cell is selected from a dorsal root ganglion (DRG) cell, a trigeminal ganglion cell and a sympathetic ganglion neuron. In a specific embodiment, the cell is a DRG cell that expresses a Naᵥ1.7 protein that comprises a human loop selected from the loop connecting transmembrane segments 5 and 6 of Domain I, the loop connecting transmembrane segments 5 and 6 of Domain II, the loop connecting transmembrane segments 5 and 6 of Domain III, the loop connecting transmembrane segments 5 and 6 of Domain IV, and a combination thereof. In one embodiment, the human loop is the loop connecting transmembrane segments 5 and 6 of Domain I. In one embodiment, the human loop is the loop connecting transmembrane segments 5 and 6 of Domain III.

In one embodiment, the cell is immortalized.

In one aspect, a mouse embryo is provided, wherein the embryo comprises a donor ES cell that is derived from a mouse as described herein.

In one aspect, a targeting vector is provided, comprising a human genomic nucleic acid sequence containing a human Naᵥ1.7 gene or a fragment thereof and a selection cassette. In one aspect, a targeting vector is provided, comprising a ~113 kb human genomic nucleic acid sequence comprising exons 2 to 28 of a human Naᵥ1.7 gene and a hygromycin cassette. In another aspect, a targeting vector is provided, comprising a ~10 kb human genomic nucleic acid sequence comprising exons 7 to 9 of a human Naᵥ1.7 gene and a neomycin cassette. In another aspect, a targeting vector is provided, comprising a ~2.8 kb human genomic nucleic acid sequence comprising exons 23 to 25 of a human Naᵥ1.7 gene and a neomycin cassette.

In one aspect, a Naᵥ1.7 protein made by a mouse as described herein is provided, wherein the Naᵥ1.7 protein comprises a human sequence encoded by a fragment of a human Naᵥ1.7 gene selected from the group consisting of exons 2 to 28, exons 7 to 9, and exons 23 to 25 of a human Naᵥ1.7 gene. In one aspect, the fragment of the human Nav1.7 gene is exons 2 to 28. In another aspect, the fragment of the human Naᵥ1.7 gene is exons 7 to 9. In another aspect, the human fragment of the human Naᵥ1.7 gene is exons 23 to 25.

In one embodiment, the Naᵥ1.7 protein is reconstituted in a vesicle. In one embodiment, the Naᵥ1.7 is present in a vesicle preparation from a mouse as described herein.

In one aspect, a method for making a mouse that expresses a fully or partially humanized Naᵥ1.7 protein on a surface of an excitable cell, is provided, comprising (a) genetically modifying a mouse ES cell by replacing one or more Naᵥ1.7 mouse DNA sequences with one or more human Naᵥ1.7 DNA sequences to form a mouse donor ES cell; (b) introducing the mouse donor ES cell into a host mouse embryo to form a modified embryo; (c) gestating the modified embryo in a suitable mouse; and, (d) obtaining a mouse pup that expresses the fully or partly humanized Naᵥ1.7 protein on the surface of an excitable cell of the mouse pup.

In one embodiment, the one or more human Naᵥ1.7 DNA sequences are selected from exons 2 to 28 of a human Naᵥ1.7 gene, exons 7 to 9 of a human Naᵥ1.7 gene and exons 23 to 25 of a human Naᵥ1.7 gene.

In one embodiment, the one or more human Naᵥ1.7 DNA sequences is all or substantially all of a human Nav1.7 DNA sequence. In a specific embodiment, the sequence is exons 2 to 28 of a human Naᵥ1.7 gene. In another specific embodiment, the sequence is exons 7 to 9 of a human Naᵥ1.7 gene. In another specific embodiment, the sequence is exons 23 to 25 of a human Naᵥ1.7 gene.

In one aspect, a mouse is provided that expresses a human Naᵥ1.7 α-subunit from an endogenous mouse Naᵥ1.7 locus, wherein the mouse expresses an endogenous mouse Naᵥ β-subunit, and wherein the mouse expresses an endogenous Naᵥ protein selected from the group consisting of Naᵥ1.6, Naᵥ1.8, and Naᵥ1.9.

In one embodiment, the human Naᵥ1.7 α-subunit is a variant Naᵥ1.7 α-subunit, wherein the variant comprises an amino acid substitution that comprises a Q10R, I136V, F216S, S241T, N395K, V400M, L823R, I848T, L858H, L858F, A863P, V872G, F1449V, or a combination thereof.

In one embodiment, the human Naᵥ1.7 α-subunit is a variant Naᵥ1.7 α-subunit, wherein the variant comprises an amino acid substitution that comprises a R996C, V1298D, V1298F, V1299F, I1461T, F1462V, T1464I, M1627K, A1632E, or a combination thereof.

In one embodiment, the human Naᵥ1.7 α-subunit is a variant Naᵥ1.7 α-subunit, wherein the variant comprises an amino acid substitution that comprises a F1200L, I1235L, or a combination thereof.

In one embodiment, the human Naᵥ1.7 α-subunit is a truncated Naᵥ1.7 α-subunit, wherein the truncated Naᵥ1.7 α-subunit protein ends at an amino acid residue selected from 259, 277, 328, 459, 693, 767, 830, 897, 1488, 1659 and 1689. In a specific embodiment, the truncated Naᵥ1.7 α-subunit protein ends at amino acid residue 693. In another specific embodiment, the truncated Naᵥ1.7 α-subunit protein ends at amino acid residue 1488.

In one aspect, a method is provided for making a cell line from a cell that expresses a human Naᵥ1.7 sequence, comprising obtaining a cell that expresses a human Naᵥ1.7 sequence from a mouse as described herein, isolating and cloning the cell, and maintaining the isolated and cloned cell in culture. In one embodiment, the method further comprises immortalizing the cell. In one embodiment, the cell is a neuronal cell, e.g., a dorsal root ganglion (DRG) neuron.

In one aspect, a method for making an immortalized cell line from an isolated cell of a mouse as described herein is provided, comprising providing an isolated cell that expresses a human, chimeric or variant human Naᵥ1.7 channel, transfecting the isolated cell with a vector that encodes an oncogene and a selectable marker (*e.g*., neomycin), growing cells in culture under selection to allow for expansion of cells that have been transfected with the retroviral vector, selecting a transfected cell from the culture containing the vector, isolating cells containing the vector by typsinization and limiting dilution of the transfected cell in culture, and creating a clonal cell line from the isolated clone that has survived selection by passage into a new culture.

In one embodiment, the isolated cell is a neuron. In one embodiment, the isolated cell is a DRG neuron.

In one embodiment, the human Naᵥ1.7 channel is encoded by exons 2 - 28 of a human Naᵥ1.7 gene. In another embodiment, the chimeric Naᵥ1.7 channel is encoded by a genomic sequence that comprises a sequence from a human Naᵥ1.7 gene that encodes an extracellular sequence from a human Naᵥ1.7 gene.

In one embodiment, the extracellular sequence encodes a pore loop sequence. In a specific embodiment, the pore loop sequence is selected from a loop connecting transmembrane segments 5 and 6 of Domain I and a loop connecting transmembrane segments 5 and 6 of Domain III. In a specific embodiment, the pore loop is the loop connecting transmembrane segments 5 and 6 of Domain I. In another embodiment, the pore loop is the loop connecting transmembrane segments 5 and 6 of Domain III.

In one aspect, a method for identifying an antagonist of a human Naᵥ1.7 protein is provided, comprising exposing a mouse as described herein to a suspected antagonist of human Naᵥ1.7, and determining an effect of the antagonist on Naᵥ1.7 function in the mouse.

In one embodiment, determining the effect of the antagonist comprises measuring the presence or absence of an action potential upon stimulation of a cell comprising the human Naᵥ1.7.

In one embodiment, the antagonist is specific for Naᵥ1.7 and does not exhibit antagonist activity with respect to Naᵥ1.6, Naᵥ1.8, and Naᵥ1.9.

In one aspect, a method is provided for determining binding activity of a therapeutic agent that binds a human Naᵥ1.7 sequence, comprising contacting the therapeutic agent to a cell that expresses a human Naᵥ1.7 sequence, and determining whether the therapeutic agent binds to the human Naᵥ1.7 sequence. In one embodiment, the cell is derived from a mouse as described herein.

In one embodiment, the cell is a neuronal cell. In a specific embodiment, the cell is selected from a dorsal root ganglion (DRG) cell, a trigeminal ganglion cell and a sympathetic ganglion neuron. In a specific embodiment, the cell is a DRG cell that expresses a Naᵥ1.7 protein that comprises a human loop selected from the loop connecting transmembrane segments 5 and 6 of Domain I, the loop connecting transmembrane segments 5 and 6 of Domain II, the loop connecting transmembrane segments 5 and 6 of Domain III, the loop connecting transmembrane segments 5 and 6 of Domain IV, and a combination thereof. In one embodiment, the human loop is the loop connecting transmembrane segments 5 and 6 of Domain I. In one embodiment, the human loop is the loop connecting transmembrane segments 5 and 6 of Domain III.

In one embodiment, the cell is immortalized.

In one embodiment, the therapeutic agent binds a human Naᵥ1.7 but does not bind a Naᵥ1.7 sequence selected from a mouse, rat, monkey, and a combination thereof.

In one embodiment, the therapeutic agent that binds the human Naᵥ1.7 sequence is selected from a benzodiazepine, a benzazepinone, a tetrodotoxin, a biphenyl pyrazole dicarboxamide, a sodium channel blocker (*e.g*., amitryptiline, mexiletine, lidocaine, carbamazepine, biphenyl pyrazoles), a piperidine T-type antagonist (*e.g*., Z123212), and analogs thereof.

In one embodiment, the therapeutic agent that binds the human Naᵥ1.7 sequence is selected from a binding protein that comprises an immunoglobulin V_{H} and/or V_{L} or Naᵥ1.7-binding fragment thereof, an antibody, a bispecific antibody, an immunoadhesin, a ligandbody, a peptibody, and a domain antibody (*e.g*. dAb). In a specific embodiment, the therapeutic agent comprises a human immunoglobulin or T cell receptor variable region. In a specific embodiment, the therapeutic agent is a human antibody.

In one aspect, an *in vitro* system for identifying an antagonist of a human Naᵥ1.7 protein is provided, comprising isolating a Naᵥ1.7-containing component from a mouse as described herein, exposing the component to a suspected antagonist of human Naᵥ1.7, and determining an effect of the antagonist on Naᵥ1.7 function. In one embodiment, the Naᵥ1.7-containing component is a membrane fraction. In one embodiment, the Naᵥ1.7-containing component is a cell. In one embodiment, the Naᵥ1.7-containing component is a tissue of the mouse.

In one embodiment, determining the effect comprises measuring the presence or absence of a Naᵥ1.7-dependent response in a cell derived from a mouse as described in this disclosure. In one embodiment, the response is an action potential.

In one aspect, a method for the identification of a modulator of a human, chimeric or variant Naᵥ1.7 channel is provided, comprising exposing a mouse as described herein to a test compound and detecting activity or inactivity of the Naᵥ1.7 channel. In one embodiment, the method comprises assaying test compounds that modulate sodium ion flux of the Naᵥ1.7 channel. In another embodiment, the method comprises employing patch clamp technology. In a specific embodiment, the method is used to identify physiologically active compounds useful for treatment of a disease condition of the brain. In one embodiment, the disease condition of the brain is selected from convulsions, seizures, panic disorders, hyperactivity disorders, depression, obsessive compulsive disorders, dementia, memory deficits, attention deficit, obesity, anxiety, eating disorders, drug addiction and misuse, altered sexual drive, Parkinson's disease and Alzheimer's disease. In another embodiment, the disease condition is related to a visceral response originating to the limbic system. In one embodiment, the visceral response is selected from respiration and gastrointestinal function.

In one embodiment, the modulator increases the activity of the Naᵥ1.7 channel. In another embodiment, the modulator decreases the activity of the Naᵥ1.7 channel.

In one embodiment, the human, chimeric or variant human Naᵥ1.7 channel is associated with a pain disorder. In a specific embodiment, the pain disorder is selected from congenital insensitivity to pain (CIP), erythromelalgia (IEM), and paroxysmal extreme pain disorder (PEPD).

In one aspect, a method for determining the probability of disease resulting from a variant Naᵥ1.7 channel is provided, comprising identifying mutations at one or more sites within a nucleic acid sequence of a Naᵥ1.7 gene isolated from a cell of a mouse as described herein that encodes an intracellular N-terminal region, an extracellular loop in domain I, an intracellular loop between domains I and II, an intracellular loop between domains II and III, an intramembrane region of domain II, or any combination thereof, wherein the identified mutations encode a Naᵥ1.7 channel protein that displays a change in function not observed in a nonvariant Naᵥ1.7 channel.

In one embodiment, the human, chimeric or variant human Naᵥ1.7 channel is associated with a pain disorder. In a specific embodiment, the pain disorder is selected from congential insensitivity to pain (CIP), erythromelalgia (IEM), and paroxysmal extreme pain disorder (PEPD).

In one aspect, a method for selecting a batch or lot of a pharmaceutical preparation that contains a therapeutic agent that binds a human Naᵥ1.7 sequence is provided, comprising exposing a cell that bears a Naᵥ1.7 protein that comprises at least one contiguous human sequence to a sample of the batch or lot of the pharmaceutical preparation, determining whether the sample binds the cell, and selected a batch or lot that corresponds to the sample that bound the at least one contiguous human sequence. In one embodiment, the at least one contiguous human sequence encodes an extracellular pore loop of the Naᵥ1.7 protein. In a specific embodiment, the extracellular pore loop of the Naᵥ1.7 protein is selected from the loop connecting transmembrane segments 5 and 6 of Domain I, the loop connecting transmembrane segments 5 and 6 of Domain II, the loop connecting transmembrane segments 5 and 6 of Domain III, the loop connecting transmembrane segments 5 and 6 of Domain IV, and a combination thereof. In one embodiment, the extracellular pore loop is the loop connecting transmembrane segments 5 and 6 of Domain I. In one embodiment, the extracellular pore loop is the loop connecting transmembrane segments 5 and 6 of Domain III.

In one embodiment, the batch or lot of pharmaceutical preparation comprises a non-protein human Naᵥ1.7-binding compound. In one embodiment, the batch or lot of pharmaceutical preparation comprises a protein that binds to a human Naᵥ1.7. In a specific embodiment, the pharmaceutical preparation that comprises a protein includes an antibody.

In one embodiment, the cell that bears a Naᵥ1.7 protein that comprises at least one contiguous human sequence is in a mouse at the time that the sample of the batch or lot of the pharmaceutical preparation is exposed to the cell.

In one aspect, a method is provided for determining the efficacy of a Naᵥ1.7-binding protein for mediating a response resulting from a nociceptive stimulus, comprising exposing a mouse as described herein to the Naᵥ1.7-binding protein and measuring a nociceptive response of the mouse to the stimulus, wherein an attenuated nociceptive response of the mouse is an indicator of efficacy of the Naᵥ1.7-binding protein.

In one embodiment, the efficacy is determined for a batch or lot of pharmaceutical preparation. In a specific embodiment, the efficacy is determined as a quality assurance or quality control step in the manufacture of the pharmaceutical preparation for use in humans.

In one aspect, a method is provided for selectively engaging a target receptor on the surface of a neuronal cell, comprising: contacting an isolated cell from a mouse as described herein with one or more inflammatory mediators, thereby selectively engaging the target receptor; and, measuring the release of a neuropeptide.

In one embodiment, the method is employed as a functional assay to select an antagonist for the target receptor.

In one embodiment, the target receptor is a sodium channel. In a specific embodiment, the sodium channel is Naᵥ1.7.

In one embodiment, the neuronal cell is a dorsal root ganglion cell (DRG).

In one embodiment, the one or more inflammatory mediators are selected from prostaglandin E₂, bradykinin, capsaicin, protons, low pH, and a neurotrophic factor.

In one embodiment, the neuropeptide is calcitonin gene-related peptide (CGRP).

In one embodiment, a method is provided for selectively engaging a Naᵥ1.7 protein on the surface of a DRG, comprising exposing an isolated DRG from a mouse as described herein, to one or more inflammatory mediators thereby selectively engaging Naᵥ1.7 and measuring the release of CGRP.

In one embodiment, the one or more inflammatory mediators comprise prostaglandin E₂, bradykinin and capsaicin.

In one embodiment, the method is employed as a functional assay to select an antagonist for Naᵥ1.7. In a specific embodiment, the antagonist is an antibody.

In one aspect, a method for identifying a Naᵥ1.7 antagonist is provided, comprising: providing *ex vivo* a cell that expresses a human or chimeric Naᵥ1.7 protein; contacting the cell with an agent that binds to the human or chimeric Naᵥ1.7 protein; after a subsequent period of time, contacting the cell with one or more inflammatory mediators; after another subsequent period of time, determining the amount of calcitonin gene-related peptide (CGRP) released from the cell; and, comparing the amount of CGRP released from the cell in the presence of the agent that binds to the human or chimeric Naᵥ1.7 protein to a reference sample, wherein an increase in CGRP release over the reference sample indicates inhibition of Naᵥ1.7; and, determining if the agent inhibits the function of Naᵥ1.7.

In one embodiment, the cell can be a non-human cell, preferably a non-human animal cell. In a further embodiment, the cell can be a human or non-human cell exogenously expressing the human or chimeric Naᵥ1.7 protein. "Exogenous expression" thereby means that the cell is genetically modified to express the human or chimeric Naᵥ1.7 protein and would not express this protein without genetic modification. The genetic modification can include introduction of a gene encoding the human or chimeric Naᵥ1.7 protein into a cell that does not contain such a gene in its genome (such as a non-human cell), or any of its precursors. The genetic modification can also include introduction of a gene encoding the human or chimeric Naᵥ1.7 protein into a cell that does already contain such a gene in its genome (such as a human cell), or any of its precursors, wherein the introduced gene is under the control of a promoter or promoters different from those controlling said gene already present in the genome. As an example, the human cells exogenously expressing a human or chimeric Naᵥ1.7 protein can be HEK293 cells engineered to stably express human Naᵥ1.7 protein.

In one embodiment, the agent is an antibody. In a specific embodiment, the antibody is human. In one embodiment, the agent is a peptide. In one embodiment, the agent is a non-proteinaceous organic compound. In one embodiment, the non-proteinaceous organic compound is selected from the group consisting of 2-alkyl-alkanoic acids (e.g., valproic acid and analogs thereof), 3-alkylamino-alkanoic acids (e.g., gabapentin and analogs thereof), 1-benzazapin-2-ones, bupivicaine, capsaicins, flecainide, flunarizine, lidocaine, mexiletine, phenytoin, propafefenone, prostaglandins, quinidine, saxitoxin, tetracaine, tetrodotoxin, and analogs thereof.

In one embodiment, the one or more inflammatory mediators are selected from prostaglandin E₂, bradykinin, capsaicin, protons, low pH, and a neurotrophic factor. In a specific embodiment, the one or more inflammatory mediators are prostaglandin E₂, bradykinin and capsaicin.

In one embodiment, the cell is a neuronal cell. In a specific embodiment, the neuronal cell is a dorsal root ganglion cell (DRG). In a specific embodiment, the cell is a mouse DRG cell. In one embodiment, the cell is isolated from a mouse as described herein. In a specific embodiment, the cell is an isolated DRG cell from a mouse as described herein.

In one embodiment, the amount of CGRP released in the presence of the agent that binds to the human or chimeric Naᵥ1.7 protein is about 1.5-fold, about 2-fold, about 3-fold, about 4-fold, about 5-fold, about, 6-fold, about 7-fold, about 8-fold, about 9-fold, about 10-fold, about 15-fold, or about 15-fold to about 20-fold higher than the reference sample. In a specific embodiment, the agent is a non-proteinaceous molecule. In one embodiment, the non-proteinaceous molecule is a toxin selected from tetrodotoxin and ProTx-II. In a specific embodiment, the agent is an antibody. In one embodiment, the antibody is an anti-Naᵥ1.7 antibody.

In one embodiment, the reference sample contains a sodium channel blocker. In a specific embodiment, the sodium channel blocker inhibits one or more functions of a sodium channel protein by binding to and inhibiting an extracellular pore opening of the channel protein. In one embodiment, the sodium channel blocker that inhibits one or more functions of a sodium channel protein by binding to and inhibiting an extracellular pore opening of the channel protein is an alkaloid-based toxin (*e.g*., tetrodotoxin or saxitoxin). In a specific embodiment, the sodium channel blocker inhibits one or more functions of a sodium channel by binding to and inhibiting an intracellular portion of the channel. In one embodiment, the sodium channel blocker that inhibits one or more functions of a sodium channel by binding to and inhibiting an intracellular portion of the channel is a local anesthetic (*e.g*., lidocaine). In one embodiment, the sodium channel blocker that inhibits one or more functions of a sodium channel by binding to and inhibiting an intracellular portion of the channel is an anticonvulsant (*e.g*., gabapentin, carbamazepine, clonazepam, divalproex, lamotrigine, phenytoin, oxcarbazepine, tiagabine, topiramate and valproic acid).

In one embodiment, the reference sample contains a toxin isolated from the venom of an arthropod, an invertebrate, a fish, or a reptile. In a specific embodiment, the toxic venom is derived from a tarantula (*e.g*., ProTx-II).

In one aspect, a method for identifying a Naᵥ1.7 antagonist is provided, comprising providing *ex vivo* a dorsal root ganglion (DRG) cell that expresses a human or chimeric Naᵥ1.7 protein, contacting the DRG with an agent that binds to the human or chimeric Naᵥ1.7 protein and waiting a first period of time, and, after the first period of time, contacting the DRG with one or more inflammatory mediators and waiting a second period of time, determining the amount of calcitonin gene-related peptide (CGRP) released from the DRG, comparing the amount of CGRP released from the DRG in the presence of the agent that binds to the human or chimeric Naᵥ1.7 protein to a reference sample, wherein an increase in CGRP release over the reference sample indicates inhibition of Naᵥ1.7, and determining if the agent inhibits the function of Naᵥ1.7 on the DRG.

In one embodiment, the DRG cell can be a non-human DRG cell. In a further embodiment, the DRG cell can be a human or non-human DRG cell exogenously expressing the human or chimeric Naᵥ1.7 protein. "Exogenous expression" thereby means that the DRG cell is genetically modified to express the human or chimeric Naᵥ1.7 protein and would not express this protein without genetic modification. The genetic modification can include introduction of a gene encoding the human or chimeric Naᵥ1.7 protein into a DRG cell that does not contain such a gene in its genome (such as a non-human DRG cell), or any of its precursors. The genetic modification can also include introduction of a gene encoding the human or chimeric Naᵥ1.7 protein into a DRG cell that does already contain such a gene in its genome (such as a human DRG cell), or any of its precursors, wherein the introduced gene is under the control of a promoter or promoters different from those controlling said gene already present in the genome.

In one embodiment, the agent is an antibody. In a specific embodiment, the antibody is human. In one embodiment, the agent is a peptide. In one embodiment, the agent is an organic compound.

In one embodiment, the one or more inflammatory mediators are prostaglandin E₂, bradykinin and capsaicin.

In one embodiment, the DRG is isolated from a member of the group consisting of human, mouse, rat, or monkey. In a specific embodiment, the DRG is isolated from a mouse as described herein. In one embodiment, the mouse comprises a nucleotide sequence encoding a human Naᵥ1.7 α-subunit or fragment thereof operably linked to a Naᵥ promoter.

In one aspect, an *ex vivo* complex is provided; wherein the *ex vivo* complex comprises a mouse cell that expresses a Naᵥ1.7 protein comprising a mouse transmembrane domain and a human loop domain; and, a Naᵥ1.7 antagonist bound to the human loop. In one embodiment, the mouse cell is an immortalized cell. In one embodiment, the Naᵥ1.7 antagonist comprises an immunoglobulin variable domain that specifically binds a human Naᵥ1.7 loop but not a mouse Naᵥ1.7 loop.

In one aspect, an *ex vivo* complex is provided; wherein the *ex vivo* complex comprises a membrane or fragment thereof that comprises a Naᵥ1.7 protein that comprises a mouse transmembrane domain and a human loop domain; and, a Naᵥ1.7 antagonist bound to the human loop. In one embodiment, the Naᵥ1.7 antagonist comprises an immunoglobulin variable domain that specifically binds a human Naᵥ1.7 loop but not a mouse Naᵥ1.7 loop.

Any of the embodiments and aspects described herein can be used in conjunction with one another, unless otherwise indicated or apparent from the context. Other embodiments will become apparent to those skilled in the art from a review of the ensuing description. It is to be understood that both the foregoing general description and the following detailed description are exemplary and explanatory only and are not restrictive.

### BRIEF DESCRIPTION OF THE FIGURES

**FIG. 1** shows a diagram of a Naᵥ channel.

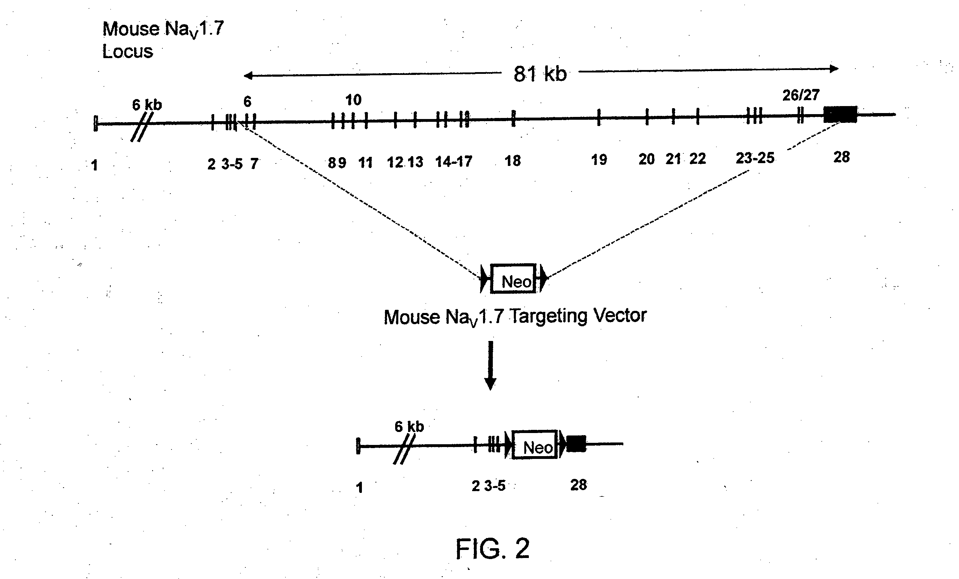

**FIG. 2** shows the murine Naᵥ1.7 gene locus (top) with the exons numbered above and below the locus. The Mouse Naᵥ1.7 Targeting Vector (middle) was used to replace an 81 kb region of the endogenous locus spanning exons 6-28 with a neomycin cassette flanked by *lox*P sites. The targeted allele results in a deleted endogenous Naᵥ1.7 locus (bottom).

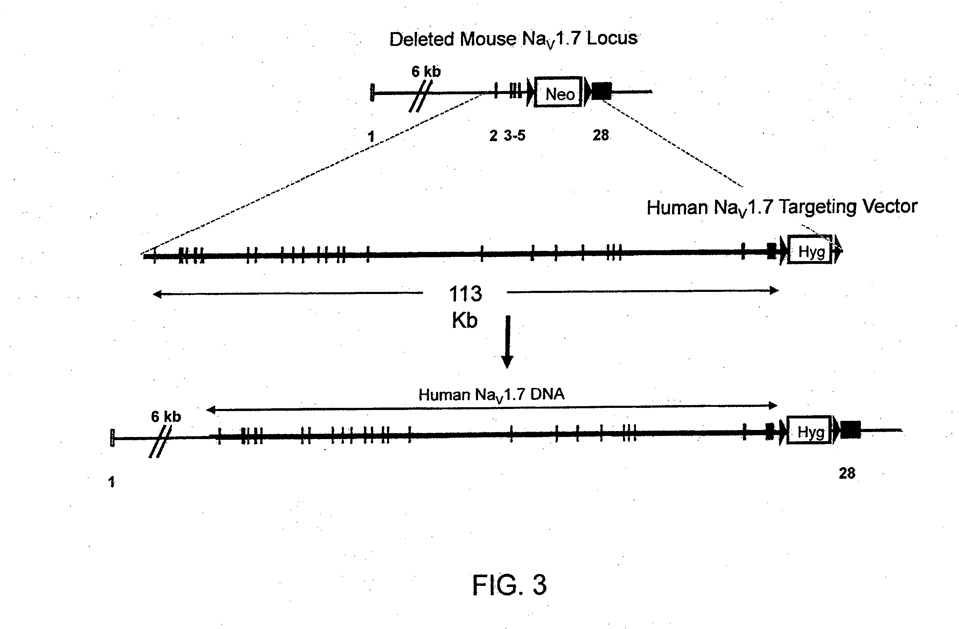

**FIG. 3** shows the deleted endogenous Naᵥ1.7 locus (top) targeted with a Human Naᵥ1.7 Targeting Vector (middle). The deleted endogenous locus previously targeted with a neomycin cassette was replaced with a targeting vector comprising exons 2-28 of a human Naᵥ1.7 locus. The targeted allele results in an endogenous locus that expresses human Naᵥ1.7 protein.

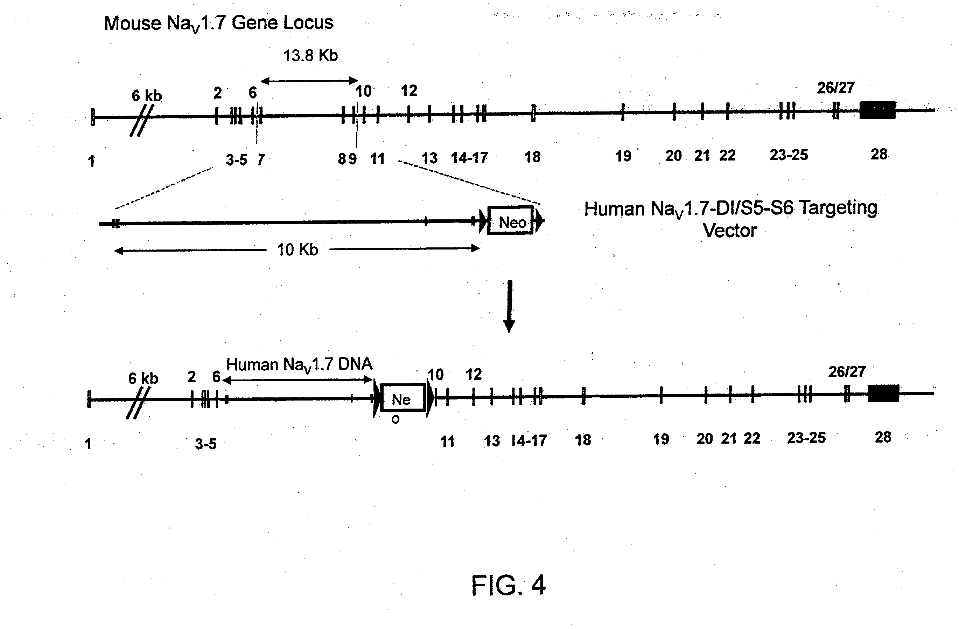

**FIG. 4** shows the mouse Naᵥ1.7 locus (top) targeted with a Human Naᵥ1.7- DI/S5-S6 Targeting Vector (middle). The targeted allele results in a partially humanized endogenous Naᵥ1.7 locus that expresses a chimeric Naᵥ1.7 protein that includes a human extracellular S5-S6 pore loop in Domain I.

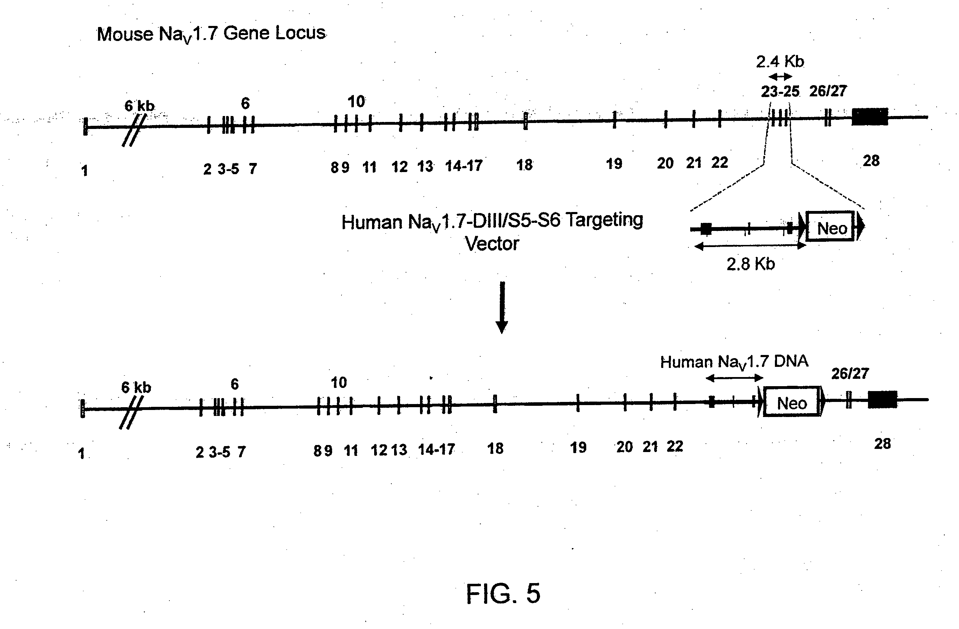

**FIG. 5** shows the mouse Naᵥ1.7 locus (top) targeted with a Human Naᵥ1.7-DIII/S5-S6 Targeting Vector (middle). The targeted allele results in a partially humanized endogenous Naᵥ1.7 gene locus that expresses a chimeric Naᵥ1.7 protein that includes a human extracellular S5-S6 pore loop in Domain III.

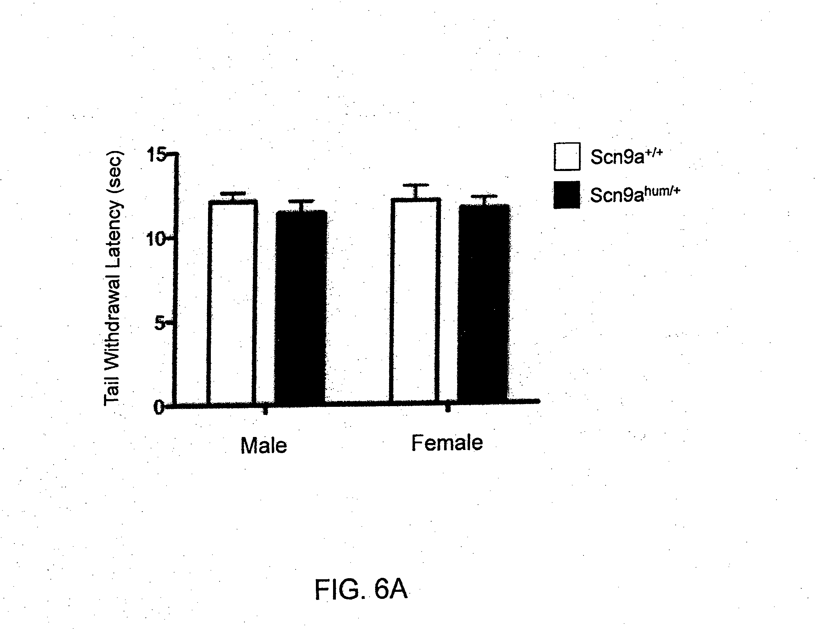

**FIG. 6A** shows the tail withdrawal latency (in seconds) in response to a nociceptive simulus (tail flick) in male and female cohorts of wild type (*Scn9A*^{+/+}) and mice heterozygous for a full length human Naᵥ1.7 gene (*Scn9A*^{*hum*/+}).

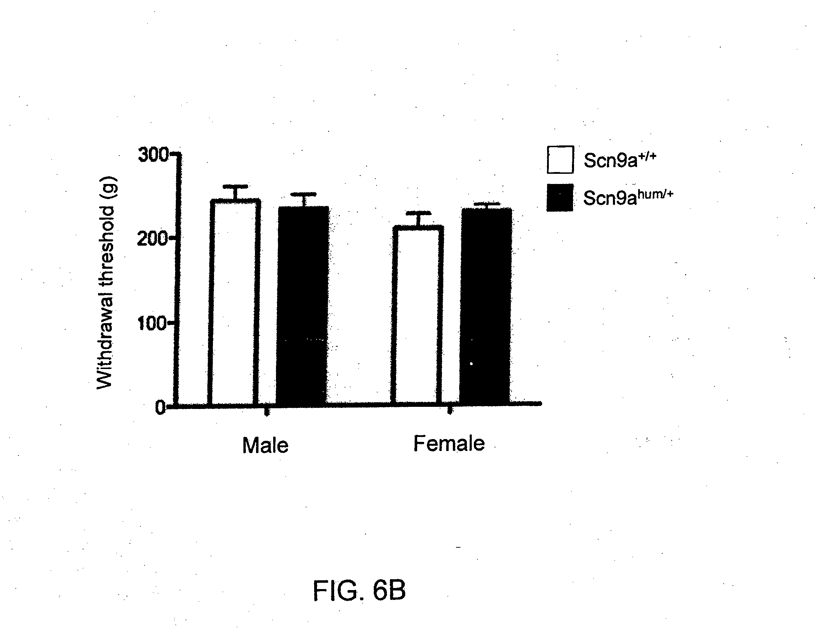

**FIG. 6B** shows the withdrawal threshold (in grams) in response to a nociceptive simulus (tail pinch) in male and female cohorts of wild type (*Scn9A*^{+/+}) and mice heterozygous for a full length human Naᵥ1.7 gene (*Scn9A*^{*hum*/+}).

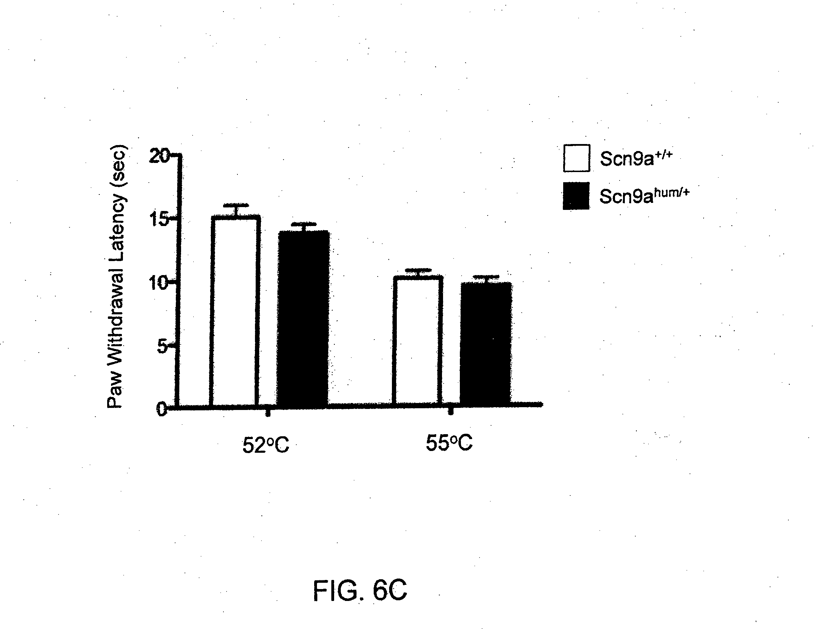

**FIG. 6C** shows the paw withdrawal latency (in seconds) in response to a nociceptive simulus (52°C and 55°C hot plate) in cohorts of wild type (*Scn9*A^{+/+}) and mice heterozygous for a full length human Naᵥ1.7 gene (*Scn9A*^{*hum*/+}).

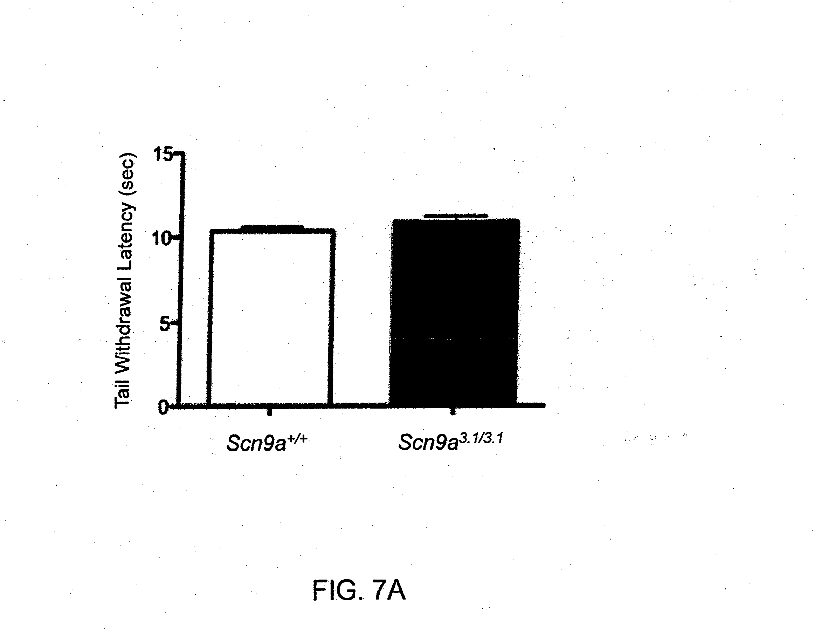

**FIG. 7A** shows the tail withdrawal latency (in seconds) in response to a nociceptive simulus (tail flick) in female cohorts of wild type (*Scn9A*^{+/+}) and mice homozygous for chimeric Naᵥ1.7 gene containing a human extracellular pore loop connecting transmembrane segments 5 and 6 of Domain I (*Scn9A*^{*3.1*/*3.1*})*.*

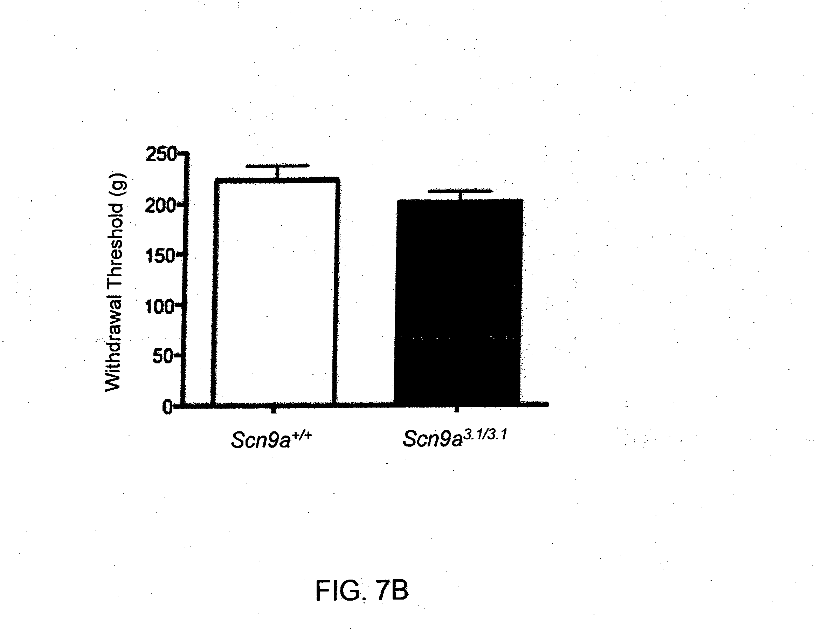

**FIG. 7B** shows the withdrawal threshold (in grams) in response to a nociceptive simulus (tail pinch) in female cohorts of wild type (*Scn9A*^{*+*/*+*}) and mice homozygous for chimeric Naᵥ1.7 gene containing a human extracellular pore loop connecting transmembrane segments 5 and 6 of Domain I (*Scn9A*^{*3.1*/*3.1*})*.*

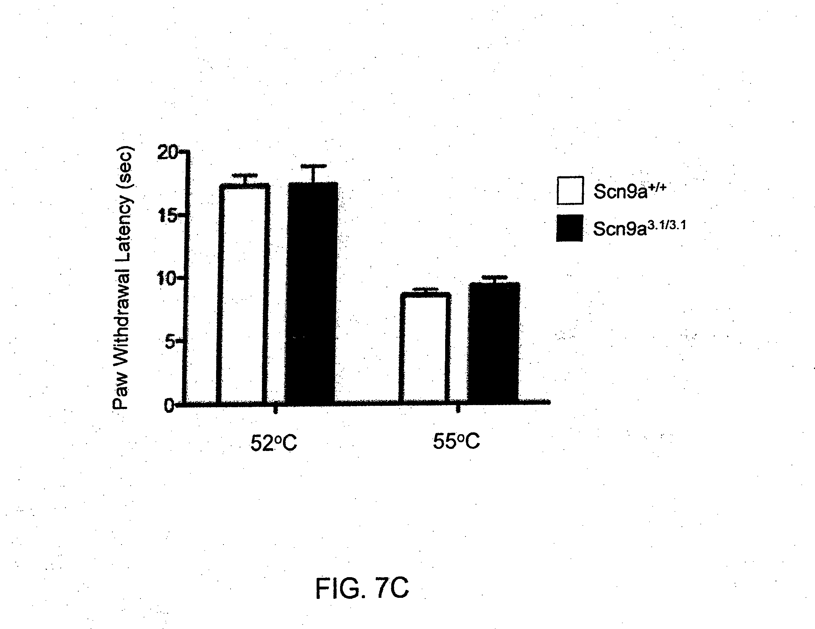

**FIG. 7C** shows the paw withdrawal latency (in seconds) in response to a nociceptive simulus (52°C and 55°C hot plate) in female cohorts of wild type (*Scn9A*^{+/+}) and mice homozygous for chimeric Naᵥ1.7 gene containing a human extracellular pore loop connecting transmembrane segments 5 and 6 of Domain I (*Scn9A*^{*3.1*/*3.1*}).

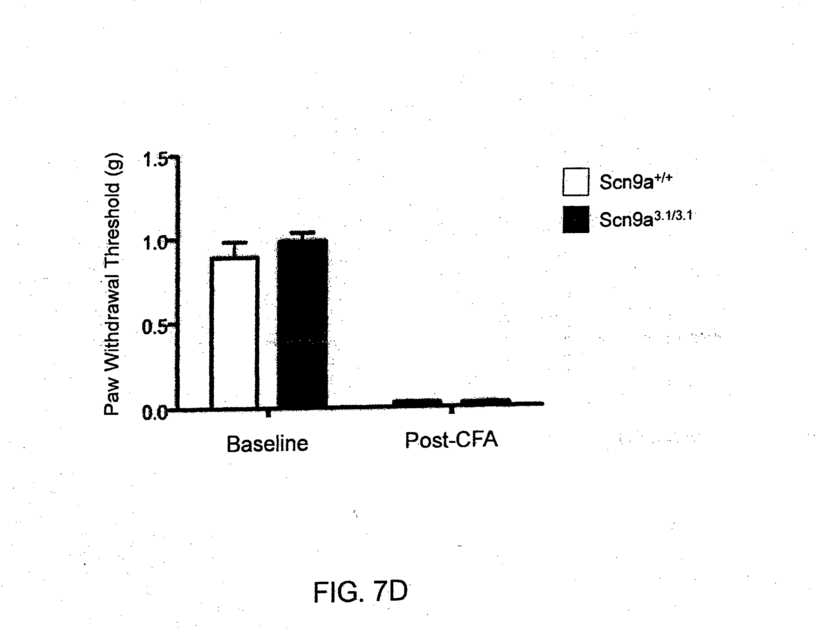

**FIG. 7D** shows mechanical allodynia measured as paw withdrawal threshold (in grams) before (baseline) and after (Post-CFA) administration of Complete Freund's Adjuvant in female cohorts of wild type (*Scn9A*^{*+*/*+*}) and mice homozygous for chimeric Naᵥ1.7 gene containing a human extracellular pore loop connecting transmembrane segments 5 and 6 of Domain I (*Scn9A*^{*3.1*/*3.1*}).

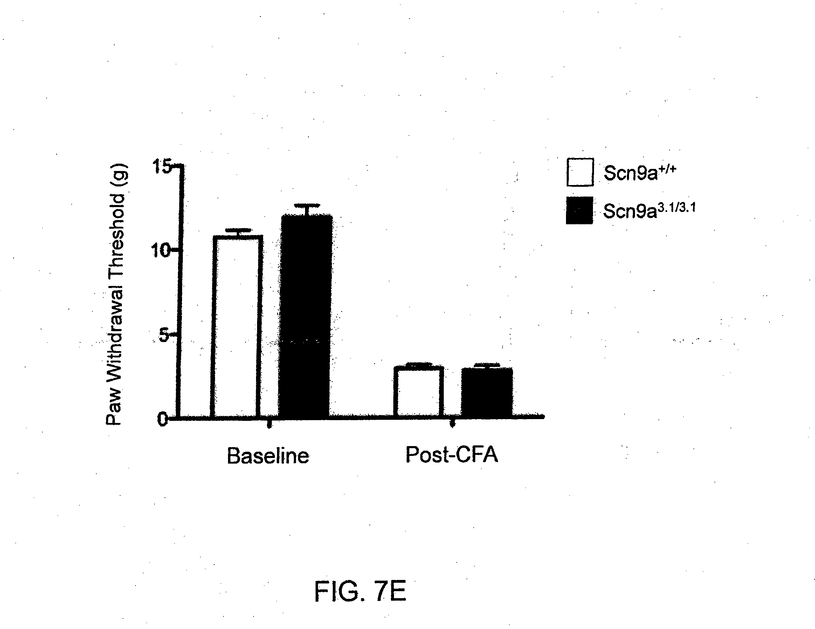

**FIG. 7E** shows thermal hyperalgesia measured as paw withdrawal threshold (in grams) before (baseline) and after (Post-CFA) administration of Complete Freund's Adjuvant in female cohorts of wild type (*Scn9A*^{+/+}) and mice homozygous for chimeric Naᵥ1.7 gene containing a human extracellular pore loop connecting transmembrane segments 5 and 6 of Domain I (*Scn9A*^{*3.1*/*3.1*}).

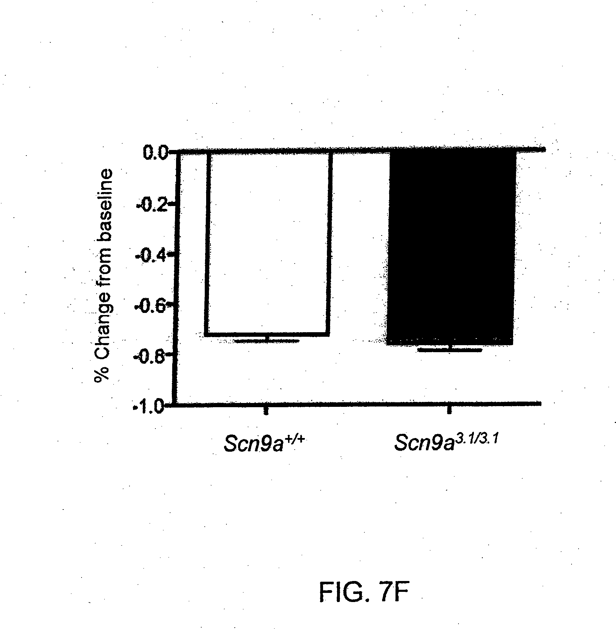

**FIG. 7F** shows the percent change from baseline in response to nociceptive simuli in female cohorts of wild type (*Scn9A*^{+/+}) and mice homozygous for chimeric Naᵥ1.7 gene containing a human extracellular pore loop connecting transmembrane segments 5 and 6 of Domain I (*Scn9A*^{*3.1*/*3.1*}).

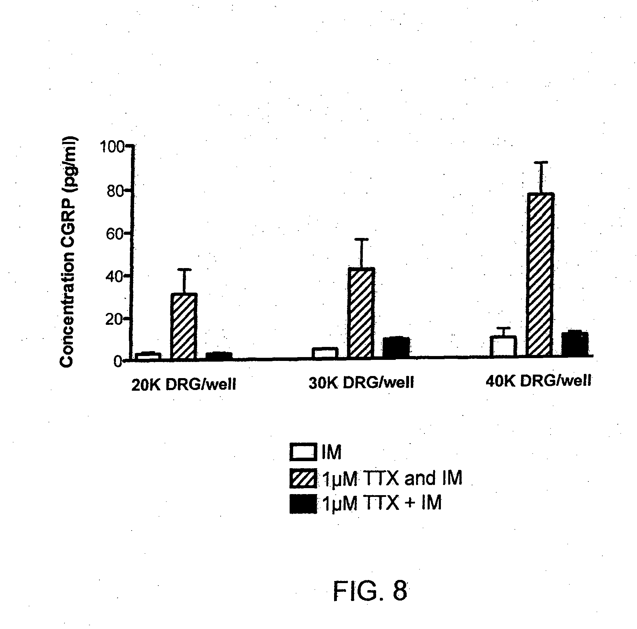

**FIG. 8** shows the concentration (pg/mL) of calcitonin gene-related peptide (CGRP) released from three populations of dorsal root ganglia (DRG) isolated from wild type mice in response to exposure to an inflammatory mix (IM), 20 minute pre-incubation with 1µM TTX followed by addition of an inflammatory mix (1µM TTX and IM) or incubation with 1µM TTX plus an inflammatory mix for 20 minutes (1µM TTX + IM).

### DETAILED DESCRIPTION

This invention is not limited to particular methods, and experimental conditions described, as such methods and conditions may vary. It is also to be understood that the terminology used herein is for the purpose of describing particular embodiments only, and is not intended to be limiting, since the scope of the present invention is defined by the claims.

Unless defined otherwise, all terms and phrases used herein include the meanings that the terms and phrases have attained in the art, unless the contrary is clearly indicated or clearly apparent from the context in which the term or phrase is used. Although any methods and materials similar or equivalent to those described herein can be used in the practice or testing of the present invention, particular methods and materials are now described. All publications mentioned are hereby incorporated by reference.

The phrase "targeting vector" or "targeting construct" includes a polynucleotide molecule that comprises a targeting region. A targeting region comprises a sequence that is identical or substantially identical to a sequence in a target cell, tissue or animal and provides for integration of the targeting construct into a position within the genome of the cell, tissue or animal via homologous recombination.

Targeting regions that target using site-specific recombinase recognition sites (*e.g*., lox or FRT sites) are also included.

In a specific embodiment, the targeting construct further comprises a nucleic acid sequence or gene of particular interest, a selectable marker, control and or regulatory sequences, and other nucleic acid sequences that allow for recombination mediated through the exogenous addition of proteins that aid in or facilitate recombination involving such sequences. In another specific embodiment, the targeting construct further comprises a gene of interest, wherein the gene of interest is a heterologous gene that encodes a protein that has a similar function as a protein encoded by the endogenous sequence.

The term "replacement" includes wherein a DNA sequence is placed into a genome of a cell in such a way as to replace a sequence within a genome, at the locus of the genomic sequence, with a heterologous sequence (*e.g*., a human sequence in a mouse). The DNA sequence so placed may include one or more regulatory sequences that are part of source DNA used to obtain the sequence so placed (*e.g*., promoters, enhancers, 5'- or 3'-untranslated regions, *etc.*). For example, in various embodiments, the replacement is a substitution of an endogenous sequence for a heterologous sequence that results in the production of a gene product from the DNA sequence so placed (comprising the heterologous sequence), but not expression of the endogenous sequence; the replacement is of an endogenous genomic sequence with a DNA sequence that encodes a protein that has a similar function as a protein encoded by the endogenous genomic sequence (*e.g*., the endogenous genomic sequence encodes a Naᵥ channel, and the DNA fragment encodes one or more human Naᵥ channels). In various embodiments, an endogenous gene or fragment thereof is replaced with a corresponding human gene or fragment thereof. A corresponding human gene or fragment thereof is a human gene or fragment that is an ortholog of, or is substantially similar or the same in structure and/or function, as the endogenous gene or fragment thereof that is replaced.

The phrase "Naᵥ channel" includes a voltage-gated sodium channel, *e.g*., a Naᵥ1.7 channel. Naᵥ channel genes include an α-subunit that is expressed on the surface of the cell and serves as a gate that allows the influx of Na+ into the cell through a pore formed by transmembrane segments that are part of the α-subunit. The α-subunit associates with other subunits, *e.g*. β1, β2, β3 and β4, to carry out action potentials. There are several different Naᵥ channel genes and they can be categorized by sensitivity to puffer fish toxin (tetrodotoxin, or TTX). TTX-sensitive channels, i.e. those blocked by low nanomolar TTX concentrations, include Naᵥ1.1, Naᵥ1.2, Naᵥ1.3, Naᵥ1.4, Naᵥ1.6 and Naᵥ1.7. TTX-resistant channels, i.e., those blocked by µM concentrations of TTX, include Naᵥ1.5, Naᵥ1.8 and Naᵥ1.9. Within the Naᵥ channel genes, subtypes or mutants have been described in human subjects. By way of illustration, nucleotide and amino acid sequences of a human Naᵥ1.7 gene are provided in SEQ ID NOs: 42 and 43, respectively. Persons of skill upon reading this disclosure will recognize that one or more endogenous Naᵥ channel genes in a genome (or all) can be replaced by one or more heterologous Naᵥ channel genes (*e.g*., subtypes or mutants, genes from another species, chimeric forms, *etc.*).

The term "variants" includes variations of a normal sequence of a gene resulting in a series of different forms of the same gene. The different forms may comprise differences of up to, *e.g*., 20 amino acids in the sequence of a protein from a gene. For example, alleles can be understood to be alternative DNA sequences at the same physical gene locus, which may or may not result in different traits (*e.g*., heritable phenotypic characteristics) such as susceptibility to certain diseases or conditions that do not result in other alleles for the same gene or result in varying degrees in the other alleles.

An "excitable cell" includes a cell that is involved generating action potentials on stimulation. Exemplary excitable cells include neurons, myocytes and electrocytes. Excitable cells change the electrical potential of their membranes on stimulation in sudden and reversible manner to transmit electrical signals to other excitable cells thereby providing cell-to-cell communication. For example, voluntary muscle contraction is controlled by action potentials via neurons that innervate muscle fibers. In various embodiments, the genetically modified non-human animals of the present invention display action potentials controlled by the expression of the human and/or chimeric Naᵥ1.7 proteins on the surface of neurons in various types of tissues, *e.g*. muscle, within the non-human animal.

A "neuron" includes a nerve cell and is a specialized cell that exhibits, for example, electrical excitability. Neurons, as described herein, form complex membrane junctions with other neurons to form a contact thereby allowing one neuron to transmit signals to another. Such contacts between neurons are referred to in the art as synapses, which can be excitatory or inhibitory. Neurons can be a part of the central nervous system of an animal or be found in the periphery of the animal in other specialized nervous tissue, *e.g*. ganglia. For example, some neurons are situated in sensory organs such as the retina and cochlea.

The term "disruption" is used to refer to when a fragment of DNA recombines with an endogenous homologous sequence, *e.g.* a gene or gene locus. These sequence disruptions may include insertions, deletion, substitutions, replacements, missense, or a frameshift of DNA sequence, or any combination thereof. Insertions may include the insertion of entire genes or fragments of genes, *e.g.* exons, which may be of an origin other than the endogenous sequence. Disruption of an endogenous homologous sequence may alter the protein produced from a normal gene such that it is inhibited entirely or in part, or by the production of protein from a disrupted gene may be enhanced over the normal level of production from the non-disrupted endogenous homologous sequence. In one embodiment, the disruption results in a lack of functional protein produced from the endogenous homologous sequence. In another embodiment, the disruption has no significant effect on expression of the gene.

The phrase "endogenous locus" refers to the naturally occurring genetic locus found in a wild-type host animal that is to disrupted, deleted, replaced or altered. In one embodiment, the endogenous locus is deleted. In another embodiment, the endogenous locus is altered, wherein a portion of the endogenous locus is replaced with a heterologous sequence. In another embodiment, all or substantially all of the endogenous locus is replaced with a heterologous locus. In one embodiment, the heterologous locus is a human locus.

The term "heterologous" when used in conjunction with polypeptide or gene refers to a polypeptide having an amino acid sequence or a DNA encoding the polypeptide that is not found in the non-human host animal. Thus, a genetically modified mouse having a human Naᵥ channel gene can be described as having a heterologous Naᵥ channel gene. The replaced Naᵥ channel gene can be detected using a variety of methods including, for example, PCR, Western blot, Southern blot, restriction fragment length polymorphism (RFLP), or a gain or loss of allele assay.

The phrase "endogenous promoter" refers to the promoter that is naturally associated, e.g., in a wild-type organism, with the polynucleotide sequence that encodes the endogenous protein.

The term "cell" includes any cell that is suitable for expressing a recombinant nucleic acid sequence. Cells include those of prokaryotes and eukaryotes (single-cell or multiple-cell), bacterial cells (*e.g.,* strains of *E. coli, Bacillus spp., Streptomyces spp., etc*.), mycobacteria cells, fungal cells, yeast cells (*e.g., S. cerevisiae, S. pombe, P. pastoris, P. methanolica, etc*.), plant cells, insect cells (*e.g.,* SF-9, SF-21, baculovirus-infected insect cells, *Trichoplusia ni, etc*.), non-human animal cells, human cells, or cell fusions such as, for example, hybridomas or quadromas. In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (*e.g*., COS-7), retinal cell, Vero, CV1, kidney (*e.g*., HEK293, 293 EBNA, MSR 293, MDCK, HaK, BHK), HeLa, HepG2, WI38, MRC 5, Colo205, HB 8065, HL-60, (*e.g*., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, *e.g.* a retinal cell that expresses a viral gene (*e.g*., a PER.C6™ cell).

The phrase "non-human animals" is intended to include any vertebrate such as cyclostomes, bony fish, cartilaginous fish such as sharks and rays, amphibians, reptiles, mammals, and birds. Suitable mammals include non-human primates, goats, sheep, pigs, dogs, cows, and rodents. Suitable non-human animals are selected from the rodent family including rat and mouse. In one embodiment, the non-human animals are mice.

The term "conservative," when used to describe a conservative amino acid substitution, includes substitution of an amino acid residue by another amino acid residue having a side chain R group with similar chemical properties *(e.g.,* charge or hydrophobicity). In general, a conservative amino acid substitution will not substantially change the functional properties of interest of a protein, for example, the ability of a variable region to specifically bind a target epitope with a desired affinity. Examples of groups of amino acids that have side chains with similar chemical properties include aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and, sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be substitution of any native residue in a protein with alanine, as used in, for example, alanine scanning mutagenesis. In some embodiments, a conservative substitution is made that has a positive value in the PAM250 log-likelihood matrix disclosed in Gonnet, G.H., Cohen, M.A., and Benner, S.A. (1992) Exhaustive Matching of the Entire Protein Sequence Database, Science 256:1443-45, hereby incorporated by reference. In some embodiments, the substitution is a moderately conservative substitution wherein the substitution has a nonnegative value in the PAM250 log-likelihood matrix.

The term "identity" when used in connection with a comparison of sequences, includes identity as determined by a number of different algorithms known in the art that can be used to measure nucleotide and/or amino acid sequence identity. In some embodiments described herein, identities are determined using a ClustalW v. 1.83 (slow) alignment employing an open gap penalty of 10.0, an extend gap penalty of 0.1, and using a Gonnet similarity matrix (MacVector™ 10.0.2, MacVector Inc., 2008).

The term "antibody", as used herein, is intended to refer to immunoglobulin molecules comprising four polypeptide chains, two heavy chains and two light chains interconnected by disulfide bonds. Each heavy chain comprises a heavy chain variable region (V_{H}) and a heavy chain constant region (C_{H}). The heavy chain constant region comprises three domains for IgG, C_{H}1, C_{H}2 and C_{H}3 and a fourth domain, C_{H}4, in the case of IgM and IgE constant regions. Each light chain comprises a light chain variable region (V_{L}) and a light chain constant region. The light chain constant region comprises one domain of either a κ or λ type (Cκ or Cλ). The V_{H} and V_{L} regions can be further subdivided into regions of hypervariability, termed complementary determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4.

The term "human antibody", as used herein, is intended to include antibodies having variable and constant regions derived from human germline immunoglobulin sequences, and may include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

A "neutralizing" or "blocking" antibody, as used herein, is intended to refer to an antibody whose binding to a target molecule (*e.g*., Naᵥ1.7) results in inhibition of at least one function of the target molecule. For example, the inhibition caused by an Naᵥ1.7 neutralizing or blocking antibody need not be complete so long as it is detectable using an appropriate assay. Exemplary assays for detecting Naᵥ1.7 inhibition are described elsewhere herein.

The phrase "micromolar range" is intended to mean 1-999 micromolar; the phrase "nanomolar range" is intended to mean 1-999 nanomolar; the phrase "picomolar range" is intended to mean 1-999 picomolar.

### Naᵥ Channel Expression and Function

There are nine known members in the family of Naᵥ channels. The gene names are SCN1A through SCN11A, and the respective proteins are designated Naᵥ1.1-Naᵥ1.9. Each have been further classified based on sensitivity to puffer fish toxin (tetrodotoxin, or TTX). The nine Naᵥ channels have been reported to exhibit diverse functional properties and distinct expression patterns, which imply specialized functions among the channels. Expression of Naᵥ channels can be detected, for example, in neurons of the central and peripheral nervous systems, cardiac myocytes, skeletal muscle, glia cells, and Schwann cells. Naᵥ1.7, a TTX-sensitive Naᵥ channel also known as PN1 and SCN9A, has been detected in sympathetic neurons, Schwann cells, neuroendocrine cells and dorsal root ganglia (DRG). Naᵥ1.7 is almost exclusively expressed in DRG and concentrates in the tips of these specialized neurons. Such a distribution predisposes this channel for a role in transmitting pain signals.

Naᵥ channels contain an α-subunit (FIG.1) that forms a pore in the membrane of cells that allows the flow of Na⁺ ions thereby mediating action potentials. This α-subunit also associates with one to two β-subunits, which function in the regulation of channel gating. The expression of the α-subunit on the surface of the cell appears to be required for channel function. Naᵥ channels open and close through a pore, which is made up of the transmembrane segments 5 (S5) and 6 (S6) of each of the Domains (FIG.1). It is through this pore formed by the cyclical arrangement of the four domains at the cell surface that Na⁺ ions move into the cell and cause depolarization of the cell membrane. This action changes the electrochemical gradient of the cell and generates an action potential, which leads to the transmission of electrical signals between cells. Mutation studies have demonstrated that the amino acids making up the loops connecting the transmembrane segments both on the extracellular side and the intracellular side of the cell regulate the opening and closing of the channel in a gate-receptor type fashion. Such mutations alter and/or destabilize the state of the channel leaving the channel in a perpetual "on" or "off" state, therefore, causing what has been termed as channelopathies, *e.g.* hyperexcitability, which leads to severe and persistent pain states.

### Naᵥ1.7 and Pain Pathways

Among the Naᵥ channels, Naᵥ1.7 is associated with fast activation and inactivation, and has been postulated to act as a threshold channel. Genetic studies have linked Naᵥ1.7 to both severe pain as well as indifference to pain. For example, erythromelalgia (IEM) and paroxysmal extreme pain disorder (PEPD) result from Naᵥ1.7 mutations that increase channel activity by either shifting channel activation to a more negative potential or impairing inactivation. Other mutations have been described that lead to a non-functional Naᵥ1.7 protein and thus a complete absence of pain, called congenital indifference to pain (CIP). CIP mutations, while impairing the ability to smell, appear to have no effect on motor, cognitive and cardiac functions. Several Naᵥ1.7 mutations relating to IEM, PEPD and CIP and the resulting aberrant effect on Naᵥ1.7 function have been reported. Naᵥ1.7 has also been suggested to have a role in metastasis due to the finding that Naᵥ1.7 is up-regulated in some cancer cell lines. Further, nerve growth factor (NGF) has been demonstrated to increase Naᵥ1.7 levels, which has suggested a relationship to inflammatory pain. Accordingly, these findings indicate that Naᵥ1.7 is involved in several control points critical for the perception of pain, inflammation and, perhaps, the persistence of cancer.

Conventional therapies employing nonselective sodium channel inhibitors such as, for example, lidocaine, mexiletine, and carbamazepine show some value in the treatment of pain, however they are limited due to significant motor, cognitive and cardiac side effects from their inhibition on Naᵥ channels not involved in the pain response. The use of analgesics, anticonvulsants and anti-arrhythmics for treating abnormal Naᵥ activity has been met with similar results. This reveals the importance and immediate need for specific Naᵥ inhibitors. Identification of therapeutics that selectively inhibit Naᵥ1.7 could prove effective to treat pain and inflammation in humans and the assessment of such therapeutics requires a suitable animal model that expresses human Naᵥ1.7. The present invention fulfils this and other needs.

Cell lines that stably express Naᵥ channel proteins have proved difficult to construct and thus the development of suitable animal models to elucidate Naᵥ channel function and identify specific inhibitors of Naᵥ channels has been adversely affected. Also, deletion of murine Naᵥ1.7 is lethal, caused by an alleged decrease in olfaction, which is postulated to result in the failure to feed. Deletions of Naᵥ1.7 within subsets of cells have been achieved and confirmed a role in mechanisms of pain, but the applicability of this approach is not without limitation. A mouse in which the entire and/or specific portions of the human Naᵥ1.7 protein is expressed, in various embodiments could be used to accurately reflect human pain mechanisms and pathologies associated with disorders resulting from Naᵥ1.7 mutations. Such a mouse would serve as a vital tool in the engineering, analysis and evaluation of therapeutics for treatment of human pain disorders such as, *e.g*., IEM, PEPD, chronic and acute pain, and inflammatory pain, by providing an animal model capable of achieving a more accurate expression and function profile of Naᵥ channel processes in humans. Further, cell lines derived from such mice would be exceptionally useful tools for evaluating human therapeutics.

### Mice Expressing Heterologous Naᵥ1.7 Channels

Genetically modified non-human animals are provided that express fully or partially human Naᵥ1.7 protein. Naᵥ1.7 protein can be expressed on the surface of excitable cells, *e.g*. neurons, of the animal's nervous system.

The genetic modification, in various embodiments, comprises a deletion of a functional mouse Naᵥ1.7 gene in whole or in part, and in some embodiments a further modification comprising a replacement with a human Naᵥ1.7 gene in whole or in part, wherein the non-human animal expresses functional mouse β-subunits. Genetically modified non-human embryos, cells, and targeting constructs for making the non-human animals, non-human embryos, and cells are also provided.

Compositions and methods for making a mouse that expresses a human Naᵥ1.7 protein, including specific variants (*e.g*., single amino acid differences), are provided, including compositions and method for making a mouse that expresses such genes from a mouse promoter and a mouse regulatory sequence. The methods include selectively rendering an endogenous mouse Naᵥ1.7 gene nonfunctional *(e.g.,* by a deletion of its α-subunit), and employing an α-subunit of a human Naᵥ1.7 gene at the endogenous mouse Naᵥ1.7 gene locus to express a human Naᵥ1.7 α-subunit gene in a mouse. The deletion of the mouse Naᵥ1.7 gene is made by deletion of the α-subunit gene, but not a β-subunit gene. The approach selectively renders the endogenous Naᵥ1.7 α-subunit gene nonfunctional while retaining a functional endogenous β-subunit.

The endogenous Naᵥ1.7 α-subunit replacement approach employs a relatively minimal disruption in natural Naᵥ1.7-mediated signal transduction in the animal, in various embodiments, because the genomic sequence of the Naᵥ1.7 α-subunits are replaced in a single fragment and therefore retain normal functionality by including necessary regulatory sequences. Thus, in such embodiments the Naᵥ1.7 α-subunit modification does not affect other endogenous Naᵥ channel genes dependent upon functional β-subunits. Further, in various embodiments the modification does not affect the assembly of a functional receptor complex involving an Naᵥ1.7 α-subunit and an endogenous β-subunit, which are believed to be required for proper channel gating and modulation of channel expression of Naᵥ1.7 α-subunits on the cell surface and for downstream signaling resulting from an activated channel. Because the β-subunits are not deleted, animals containing a replacement of an endogenous Naᵥ1.7 α-subunit gene with a human Naᵥ1.7 α-subunit gene should be able to process normal voltage-gated Naᵥ channel functions from Na+ passage into the cell through the pore of the human Naᵥ1.7 α-subunit present on the surface of neurons.

A schematic illustration (not to scale) of a deleted endogenous mouse Naᵥ1.7 gene is provided in FIG. 2. As illustrated, the mouse Naᵥ1.7 α-subunit is encoded by 28 exons spanning more than 80 kb of sequence. The endogenous mouse Naᵥ1.7 gene is deleted by a targeting construct (Mouse Naᵥ1.7 Targeting Vector) with a neomycin cassette flanked by recombination sites. This endogenous locus encodes the α-subunit of the mouse Naᵥ1.7 gene responsible for the generation of action potentials triggered by the depolarization of the cell membrane in response to flow of Na⁺ ions into the interior of the cell.

A genetically modified mouse lacking a nucleotide sequence encoding an α-subunit of the endogenous Naᵥ1.7 gene can be made by any method known in the art. For example, a targeting vector can be made that deletes the mouse Naᵥ1.7 gene with selectable marker gene. FIG. 2 illustrates a mouse genome (bottom) targeted by a targeting construct having a 5' homology arm containing sequence upstream of exon 6 of the endogenous Naᵥ1.7 locus, followed by a drug selection cassette (*e.g*. a neomycin resistance gene flanked by *loxP* sequences), and a 3' homology arm containing sequence downstream of exon 27 of the endogenous Naᵥ1.7 locus. Upon homologous recombination at the locus, the endogenous Naᵥ1.7 locus is replaced by a drug selection cassette (FIG. 2, bottom). The endogenous Naᵥ1.7 locus is thereby deleted resulting in a cell or non-human animal that does not express endogenous Naᵥ1.7 α-subunit. The drug selection cassette may optionally be removed by the subsequent addition of a recombinase *(e.g.,* by Cre treatment).

Genetically modifying a mouse to render endogenous Naᵥ1.7 gene nonfunctional, in various embodiments, results in a mouse that exhibits defects in processes of the nervous system, *e.g*. the transmission of nociceptive information, making the mouse useful for evaluating the role of the endogenous Naᵥ1.7 gene in normal and disordered neuronal function. In various embodiments, modifying the α-subunit of the endogenous Naᵥ1.7 gene, but not the β-subunits, avoids the potential reduction of other Naᵥ genes (*e.g*., Naᵥ1.6, Naᵥ1.8, Naᵥ1.9, *etc*.) that require the β-subunits for regulating voltage-gating of the channel, thereby maintaining various other functions and processes mediated through β-subunit-dependent processes.

According to reports, complete deletions of the endogenous Naᵥ1.7 gene in mice are lethal. However, deletions in specific subsets of cells have been achieved and appear otherwise normal. Mice according to the present invention have a functionally silenced endogenous Naᵥ1.7 locus in that they lack the capacity of producing a functional Naᵥ1.7 α-subunit on the cell surface.

A schematic illustration (not to scale) of a replaced endogenous mouse Naᵥ1.7 gene with a human Naᵥ1.7 gene is provided in FIG. 3. As illustrated, an endogenous mouse Naᵥ1.7 locus that had been deleted is replaced by a targeting construct (Human Naᵥ1.7 Targeting Vector) with a hygromycin cassette flanked by recombination sites. The resulting replaced locus encodes a human Naᵥ1.7 α-subunit protein expressed on the surface of neurons in the host animal capable of mediating action potentials triggered by the depolarization of the cell in response to flow of Na⁺ ions into the cell within the host animal.

A genetically modified mouse that expresses a human Naᵥ1.7 α-subunit at the endogenous mouse Naᵥ1.7 locus can be made by any method known in the art. For example, a targeting vector can be made that introduces the human Naᵥ1.7 gene with a selectable marker gene. FIG. 3 illustrates a mouse genome comprising a replacement of the endogenous Naᵥ1.7 locus (bottom). The targeting construct contains a 5' homology arm containing sequence upstream of the endogenous mouse Naᵥ1.7 locus, followed by a genomic fragment containing a human Naᵥ1.7 gene, a drug selection cassette (e.g. a hygromycin resistance gene flanked on both sides by *lox*P sequences), and a 3' homology arm containing sequence downstream of the endogenous mouse Naᵥ1.7 locus. Upon homologous recombination at the endogenous locus, the drug selection cassette is replaced by the sequence contained in the targeting vector (bottom of FIG. 3). The deleted endogenous Naᵥ1.7 locus is thereby replaced with a human Naᵥ1.7 gene resulting in a cell or animal that expresses a human Naᵥ1.7 gene. The drug selection cassette may optionally be removed by the subsequent addition of a recombinase (*e.g*., by Cre treatment).

Other modifications to the endogenous locus can be achieved with minimal effort using similar techniques to create a locus comprising a chimeric gene. For example, schematic illustrations of the replacement of two extracellular pore loops between transmembrane segments 5 and 6 of Domain I and III of the endogenous mouse Naᵥ1.7 gene are provided in FIG. 4 and FIG. 5, respectively. As illustrated, discrete portions of a human Naᵥ1.7 gene are inserted into the endogenous mouse Naᵥ1.7 locus by other targeting constructs (Human Naᵥ1.7 DI/S5-S6 Targeting Vector and Human Naᵥ1.7 DIII/S5-S6 Targeting Vector) with genomic fragments that each encode an extracellular loop of a human Naᵥ1.7 gene located at the channel pore and responsible for allowing passage of Na+ ions into the intracellular space. Upon recombination with either one of the illustrated targeting vectors, a genomic fragment of the endogenous Naᵥ1.7 locus, which encodes an extracellular pore loop of the endogenous Naᵥ1.7 protein, is replaced with a human genomic fragment encoding the corresponding pore loop in a human Naᵥ1.7 protein. This creates a chimeric locus that produces a chimeric Naᵥ1.7 protein that comprises human extracellular loops in the pore of a Naᵥ1.7 channel protein.

A genetically modified mouse that expresses an extracellular pore loop of a human Naᵥ1.7 channel at the endogenous mouse Naᵥ1.7 locus can be made by any method known in the art. For example, a targeting vector can be made that introduces a genomic fragment that encodes an extracellular pore loop of a human Naᵥ1.7 channel with a selectable marker gene. FIGs. 4 and 5 each illustrate a mouse genome comprising separate replacements of extracellular loops located at the pore of a Naᵥ1.7 channel protein. Each targeting construct contains a 5' homology arm containing sequence upstream of the endogenous mouse Naᵥ1.7 sequence to be replaced, followed by a genomic fragment containing a human sequence corresponding to the endogenous mouse Naᵥ1.7 gene sequence that encodes a specific extracellular pore loop, a drug selection cassette (*e.g*. a neomycin resistance gene flanked on both sides by *lox*P sequences), followed by a 3' homology arm containing sequence downstream of the endogenous mouse Naᵥ1.7 sequence to be replaced. Upon homologous recombination at the endogenous locus with either of the targeting vectors, a genomic fragment is inserted into the endogenous mouse Naᵥ1.7 locus resulting in a chimeric locus capable of expressing a Naᵥ1.7 channel protein comprising a human sequence corresponding to an extracellular pore loop (FIG. 4 and 5, bottom). The drug selection cassette may optionally be removed by the subsequent addition of a recombinase (*e.g*., by Cre treatment).

### Experimental Models of Naᵥ1.7 Humanized Mice

Genetically modified non-human animals that express human Naᵥ1.7 genes are useful, *e.g.,* to elucidate the various functions of Naᵥ1.7 in the cells of the nervous system, to measure the efficacy of a therapeutic agent that binds to the Naᵥ1.7 protein expressed on the cell surface, to determine a Naᵥ1.7 channel's role in mechanisms of pain and pain disorders, to serve as models of acute and/or chronic pain, and to serve as breeding mates to generate other genetically modified mice of interest. They are also useful for preparing membrane fractions or vesicles that comprise fully human or chimeric human-mouse Naᵥ1.7 proteins, for identifying antagonists of human Naᵥ1.7.

In one embodiment, a mouse according to the invention is used to determine the mechanism of channel gating that is regulated by the extracellular loops located in the pore of human Naᵥ channels. In one embodiment, a mouse of the present invention is injected with toxins that bind to extracellular pore loops of a human Naᵥ channel on the cell surface and, after a subsequent period of time, subjected to a range of stimuli to trigger firing of action potentials. The identity of the toxin is known prior to injection and the animals are analyzed for impairment of Naᵥ1.7-dependent electrical responses by comparison to electrical responses observed in wild type animals.

In another aspect, genetically modified non-human animals comprising a replacement of the endogenous Naᵥ1.7 gene with a human Naᵥ1.7 gene is provided. Such animals are useful for studying the efficacy of therapeutic agents to block Naᵥ1.7 function. In addition, human Naᵥ1.7 has been shown to exhibit mutant forms associated with disease (e.g. IEM, PEPD and CIP). Thus, genetically modified non-human animals that comprise a replacement of the endogenous Naᵥ1.7 gene with specific mutant forms of human Naᵥ1.7 genes can be used to study human disorders associated with Naᵥ1.7 mutations in the animal. In a specific embodiment, the mutant forms of human Naᵥ1.7 are associated with the pain response.

Suitable variants include mutant forms of human Naᵥ1.7 that are known in the art. Variants associated with the IEM disorder include, for example, mutations that shift activation of Naᵥ1.7 to a more negative potential. Exemplary Naᵥ1.7 mutations that lead to IEM include Q10R, I136V, F216S, S241T, N395K, V400M, L823R, I848T, L858H, L858F, A863P, V872G and F1449V. In one embodiment, the human Naᵥ1.7 sequence comprises a missense mutation that causes the IEM disorder. In a specific embodiment, the missense mutation that causes the IEM disorder is selected from I848T and L858H.

Variants associated with the PEPD disorder include, for example, mutations that compromise inactivation of a Naᵥ1.7 α-subunit. Mutations that cause PEPD have been shown to shift the steady-state fast inactivation of a Naᵥ1.7 α-subunit toward a state characterized by more depolarized potentials causing a notable increase in continuous current. Such mutations have been reported to occur, for example, in the amino acids linking DIII and DIV, which contains an inactivation motif associated with inactivating the Naᵥ1.7 α-subunit. Exemplary Naᵥ1.7 mutations that lead to PEPD include R996C, V1298D, V1298F, V1299F, I1461T, F1462V, T1464I, M1627K and A1632E. In one embodiment, the human Naᵥ1.7 sequence comprises a mutation selected from I1461T, M1627K and A1632E.

Variants associated with the CIP disorder include, for example, homozygous single-nucleotide non-sense mutations and compound heterozygous mutations, which include non-sense mutations on one allele and a deletion mutation on the other allele. The deletion mutation can be of coding or non-coding sequences, the latter of which can lead to defective splicing that functionally silence the Naᵥ1.7 α-subunit. Nonsense mutations are changes in DNA sequence, which introduce premature stop codons, causing any resulting protein to be abnormally shortened. This can cause a loss of function in the protein, as critical parts of the amino acid chain are no longer translated. Accordingly, non-human animals of the present invention comprising a human Naᵥ1.7 α-subunit with a mutation that functionally silence the human Naᵥ1.7 α-subunit demonstrate an absence of pain in response to nociceptive stimuli.

Exemplary silencing mutations in a Naᵥ1.7 gene include nonsense mutations, deletions of one or more nucleotide in a Naᵥ1.7 DNA sequence, mutations in the splice junction of exons, and frameshift mutations. In one embodiment, the genetically modified non-human animal is heterozygous for a silencing mutation that leads to CIP, wherein one allele of a Naᵥ1.7 gene comprises a non-sense mutation and the other Naᵥ1.7 allele comprises a frameshift mutation selected from F1200L and I1235L. In another embodiment, the genetically modified non-human animal is homozygous for a non-sense mutation that leads to CIP. In one embodiment, the non-sense mutation comprises a truncated Naᵥ1.7 α-subunit protein that ends at an amino acid residue selected from 259, 277, 328, 459, 693, 767, 830, 897, 1488, 1659 and 1689. In a specific embodiment, the human Naᵥ1.7 α-subunit protein ends at an amino acid residue selected from 693 and 1488.

Expression of mouse Naᵥ1.7 in whole mice was analyzed using a reporter system comprising a fusion of a LacZ reporter gene with mouse Naᵥ1.7. Analysis of LacZ signal in whole mice revealed that Naᵥ1.7 is expressed in the entire mouse nervous system, including in brain (including olfactory bulb ganglia), thalamus, hypothalamus, midbrain, pons, medulla, colliculus, optic nucleus, cerebral cortex, spinal cord gray matter (*e.g*., dorsal/sensory), dorsal root ganglia, sympathetic ganglion chain, trigeminal ganglia, celiac ganglion, intestine nervous plexus, and in smaller ganglia throughout the body (*e.g*., tongue, esophagus, trachea, bronchi, heart).

Thus, cells, cell lines, and cell cultures can be made from the above-mentioned tissues as a source of mouse Naᵥ1.7 protein. Further, genetically modified mice according to the invention express partially or fully humanized Naᵥ1.7 on the above-mentioned tissues. Thus the tissues and cells from the genetically modified mice, including cell lines and cell cultures, can be generated to serve as a source of humanized Naᵥ1.7 for use in binding and functional assays, *e.g*., to assay for binding or function of a Naᵥ1.7 agonist or antagonist, particularly where the agonist or antagonist is specific for a human Naᵥ1.7 sequence.

Cells from genetically modified mice can be isolated and used on an ad hoc basis, or can be maintained in culture for many generations. In one embodiment, cells from the genetically modified mice are immortalized and maintained in culture indefinitely (*e.g*., in serial cultures).

In one aspect, the genetically modified mice are used to make modified dorsal root ganglia (DRG) that comprise one or more of the modified Naᵥ1.7 proteins. The modified DRG(s) are employed in *ex vivo* assays to determine the effect of a Naᵥ1.7 binding agent on the function of the Naᵥ1.7 protein and on the function of other proteins, e.g., other Naᵥ family members. In one embodiment, modified DRG(s) from a mouse are isolated and assayed for one or more Naᵥ1.7 functions in the presence and absence of a Naᵥ1.7 binding agent (*e.g.,* a Naᵥ1.7 agonist or antagonist). In one embodiment, the modified DRG(s) are isolated and assayed for the function of one or more Naᵥ family members in the presence and absence of a Naᵥ1.7 binding agent. In one embodiment, the modified DRG(s) are assayed in the presence of the binding agent for function of a non-Naᵥ family protein or channel.

In one embodiment, a method for determining the effect of a Naᵥ1.7 binding agent on a DRG channel that is not a Naᵥ family member is provided, comprising exposing modified DRG(s) comprising to a Naᵥ1.7 binding agent, and measuring a function of a non-Naᵥ family member DRG channel.

In another aspect, a method is provided for determining an effect of a human therapeutic on a human Naᵥ1.7, wherein the human therapeutic does not bind a human Naᵥ1.7 protein, comprising exposing a modified DRG to the human therapeutic in an *ex vivo* assay, and measuring an effect of the human therapeutic on a function of a human Naᵥ1.7 protein.

In various embodiments and aspects, a DRG or modified DRG is assayed or a function of a DRG protein or modified DRG protein is ascertained in a patch clamp protocol, a calcium imaging protocol, a membrane-sensitive dye protocol, in an *ex vivo* assay.

In one aspect, a cell culture is provided, wherein the cell culture comprises a cell of a genetically modified mouse as described herein, wherein a substantial number of the cells in the culture express a human Naᵥ1.7 sequence. In one embodiment, the cells that express the human Naᵥ1.7 sequence are immortalized. In one embodiment, the cells are derived from a tissue selected from brain (including olfactory bulb ganglia), thalamus, hypothalamus, midbrain, pons, medulla, colliculus, optic nucleus, cerebral cortex, spinal cord gray matter (*e.g*., dorsal/sensory), dorsal root ganglia, sympathetic ganglion chain, trigeminal ganglia, celiac ganglion, intestine nervous plexus, and in smaller ganglia throughout the body (*e.g*., tongue, esophagus, trachea, bronchi, heart).

In one aspect, a method for determining whether a putative Naᵥ1.7 agonist or antagonist binds a human Naᵥ1.7 protein is provided, comprising exposing the putative Naᵥ1.7 agonist or antagonist to a cell as described herein, and determining whether the putative Naᵥ1.7 agonist or antagonist binds the cell.

In one embodiment, the human Naᵥ1.7 agonist or antagonist is selected from a protein, a peptide, and a small molecule (e.g, non-protein organic compound). In a specific embodiment, the protein comprises an immunoglobulin variable domain or Naᵥ1.7 -binding fragment thereof. In a specific embodiment, the protein is an anti-human Naᵥ1.7 antibody.

In one aspect, a method for determining whether a pharmaceutical preparation affects a function of a human Naᵥ1.7 is provided, comprising exposing the pharmaceutical preparation to a cell as provided herein that expresses a human Naᵥ1.7 protein, and measuring a Naᵥ1.7 function of the cell.

In one embodiment, the Naᵥ1.7 function measured is primary nociceptor activation. In a specific embodiment, the function measured is calcitonin gene-related peptide (CGRP) release by the cell.

In one embodiment, the pharmaceutical preparation comprises a protein, a peptide, or a peptide analog. In a specific embodiment, the protein comprises an immunoglobulin variable domain or Naᵥ1.7-binding fragment thereof. In a specific embodiment, the protein is an anti-human Naᵥ1.7 antibody.

In one aspect, a quality assurance assay for a pharmaceutical preparation comprising an agent that binds a human Naᵥ1.7 sequence is provided, comprising obtaining a sample of a pharmaceutical preparation and exposing the preparation to a mouse or a cell as described herein, where the mouse or the cell expresses a human Naᵥ1.7 sequence, and determining (a) whether the pharmaceutical preparation binds the cell, and/or (b) determining whether the pharmaceutical preparation affects a Naᵥ1.7 function of the cell.

In one embodiment, the pharmaceutical preparation is an isolated human antibody or fragment thereof. In one embodiment, the pharmaceutical preparation is a non-antibody ion channel blocker or analog thereof.

### Neuropeptide Release From Neurons of Naᵥ1.7 Humanized Mice

Primary nociceptive neurons are amenable to a number of well-established research techniques, such as electrophysiology recordings, calcium imaging and measurement of spontaneous and stimulated neuropeptide release, that provide insights in cellular and molecular functions of single neurons or neuronal subpopulations.

Calcitonin gene-related peptide (CGRP), a 37-amino-acid peptide, is predominantly synthesized and stored in sensory neurons and can be released from both central and peripheral axons (Poyner, D.R. 1992. Calcitonin gene-related peptide: multiple actions, multiple receptors. Pharmacol. Ther. 56(1): 23-51). The release of CGRP from sensory nerve terminals in peripheral tissues plays a key role in neurogenic inflammation whereas the release from terminals in the dorsal horn of the spinal cord modulates pain transmission (Oku, R., M. Satoh, N, Fujii, A. Otaka, H. Yajima, and H. Takagi. 1987. Calcitonin gene-related peptide promotes mechanical nociception by potentiating release of substance P from the spinal dorsal horn in rats. Brain Res. 403(2): 350-354). CGRP knock out mice show an attenuated response to chemical pain and inflammation (Salmon, A., M.I. Damaj, L.M. Marubio, M.P. Epping-Jordan, E. Merlo-Pich, and J.P. Changeux. 2001. Altered neuroadaptation in opiate dependence and neurogenic inflammatory nociception in αCGRP-deficient mice. Nature Neuroscience 4(4): 357-358), which implicates CGRP in regulating the pain response.

A variety of inflammatory mediators, such as prostaglandin E₂, bradykinin, and protons, or other stimulants such as low pH, neurotrophic factors and capsaicin play an essential role in the induction of pain. Further, inflammatory mediators are known to induce the release of neuropeptides by activating specific membrane receptors. For example, inflammatory mediators such as prostaglandin E₂ and bradykinin excite and sensitize DRG neurons leading to action potentials that are blocked by tetrodotoxin (Noda, K., Y. Ueda, K. Suzuki, and K. Yoda. 1997. Excitatory effects of algesic compounds on neuronal processes in murine dorsal root ganglion cell culture. Brain Res. 751 (2): 348-351; Nicol G.D. and M. Cui. 1994. Enhancement by prostaglandin E2 of bradykinin activation of embryonic rat sensory neurones. Journal Physiol. 480(Pt 3): 485-492; Momin A. and P.A. McNaughton. 2009. Regulation of firing frequency in nociceptive neurons by pro-inflammatory mediators. Exp Brain Res. 196(1): 45-52). These mediators have also been shown to regulate CGRP expression in cultured dorsal root ganglion (DRG) neurons (Supowit S.C., H. Zhao, K.A. Katki, P. Gupta, and D. J. Dipette 2011.. Bradykinin and prostaglandin E1 regulate calcitonin gene-related peptide expression in cultured rat sensory neurons. Regul Pept. 167(1): 105-111; Averbeck, B., I. Izydorczyk, and M. Kress. 2000. Inflammatory mediators release calcitonin gene-related peptide from dorsal root ganglion neurons of the rat. Neuroscience 98(1): 135-140). Moreover, prostaglandin E₂ enhances bradykinin- and capsaicin-stimulated release of CGRP (Vasko M.R., W. B. Campbell, and K. J. Waite. 1994. Prostaglandin E2 enhances bradykinin-stimulated release of neuropeptides from rat sensory neurons in culture. J. Neurosci. 14(8): 4987-4997; Southhall, M.D. and M. R. Vasko. 2001. Prostaglandin receptor subtypes EP3C and EP4 mediate the prostaglandin E2-induced cAMP production and sensitization of sensory neurons. J. Biol. Chem. 276: 16083-16091). The release of CGRP from stimulated nerves is blocked by the sodium channel antagonist tetrodotoxin (TTX) and by omega conotoxin (CTX) but not nifedipine, a known calcium channel blocker (Lundberg, J. M., A. Franco-Cereceda, K. Alving, P. Delay-Goyet, and Y. Lou. 1992. Release of Calcitonin Gene-Related Peptide from Sensory Neurons. Ann NY Acad. Sci. 657:187-93). These latter findings suggest that while N-type calcium channels are important for peptide exocytosis from stores located in large dense cored vesicles of primary afferents, sodium channels also play a role in CGRP release. Similar characteristics have been demonstrated for the peptide release evoked by low concentrations of capsaicin, which is also sensitive to both TTX and CTX. However, high concentration of capsaicin causes a massive release of CGRP which is only marginally inhibited, if at all, by TTX or CTX (Lou Y.P., A. Franco-Cereceda, and J. M. Lundberg. 1992. Different ion channel mechanisms between low concentrations of capsaicin and high concentrations of capsaicin and nicotine regarding peptide release from pulmonary afferents. Acta Physiol. Scand. 146(1): 119-127). These reports suggest that the CGRP release induced by different inflammatory agents is dependent on different channels/receptors and/or pathways. Thus, the correct stimulation for engagement of specific target receptors known to be associated with pain responses can be identified through the measurement of CGRP release from neurons. This allows for the specific functional analysis of a target receptor and the discovery of how the target receptor functions in pain responses.

Depolarization of neurons leads to an increase in neuropeptide expression and release, in particular an increase in expression of CGRP. The release of CGRP has been used in several studies to assess the mediation of the pain response and characterization of neuronal function including the generation of action potentials with respect to other channels (although not sodium channels). As described above, inflammatory mediators, *e.g*., bradykinin and prostanglin E₂, have been shown to induce CGRP release from dorsal root ganglion (DRG) in culture (Averbeck, B. et al., *supra*; Tumati, S., W. R. Roeske, T. W. Vanderah, and E. V. Varga. 2010. Sustained morphine treatment augments prostaglandin E2-evoked calcitonin gene-related peptide release from primary sensory neurons in a PKA-dependent manner. Eur. J. Pharmacol. 648(1-3):95-101). Although *in vitro* CGRP release from DRG has revealed an essential role for calcium channels, involvement in sodium channels has remained less clear (Ai, X., S. E. MacPhedran, and A. K. Hall. 1998. Depolarization stimulates initial CGRP expression by embryonic sensory neurons in vitro. J. Neurosci. 18(22): 9294-9302; Strecker, T., K. Messlinger, M. Weyand, and P. W. Reeh. 2005. Role of different proton-sensitive channels in releasing calcitonin gene-related peptide from isolated hearts of mutant mice. Cardiovasc. Res. 65(2):405-410; Spitzer, M. J., P. W. Reeh, and S. K. Sauer. 2008. Mechanisms of potassium- and capsaicin-induced axonal calcitonin gene-related peptide release: involvement of L- and T-type calcium channels and TRPV1 but not sodium channels. Neurosci. 151(3):836-842). Thus, there remains a need to develop assays that assess neuronal function mediated by sodium channels, in particular through the application of DRGs that express a human Naᵥ1.7 protein.

Methods for the measurement of neuropeptide release from neurons isolated from mice that express human Naᵥ1.7 are useful, *e.g*., to elucidate the function of nociceptive neurons expressing a human Naᵥ1.7 protein in response to inflammatory mediators by measurement of CGRP release. They are also useful for identification of Naᵥ1.7-specific antagonists through the assessment of their ability to alter or modify inflammatory mediator-induced release of CGRP in DRG isolated from mice expressing a human Naᵥ1.7 protein.

DRG neurons, known to express Naᵥ1.7, release CGRP upon depolarization. This property of DRG neurons suggests that DRGs that express human Naᵥ1.7 can be used in an assay as a means to identify antagonists of Naᵥ1.7 using measurement of CGRP released from isolated DRG in culture. Thus, neurons (for example, DRG neurons) that express a human Naᵥ1.7 or a chimeric Naᵥ1.7 may be employed in an assay to identify Naᵥ1.7 antagonists that engage human or chimeric Naᵥ1.7 channels in DRG neurons and assess the functional activity of the channel through measurement of CGRP release. In such an assay, e.g., the human or chimeric Naᵥ1.7 is incubated with an inhibitor of Naᵥ1.7 (*e.g*., tetrodotoxin) for about twenty minutes, followed by stimulation with one or more inflammatory mediators (*e.g.,* prostaglandin E₂, bradykinin, and capsaicin) for an additional twenty minutes. Additional candidate antagonists may be employed in this assay to assess their therapeutic potential and ability to block Naᵥ1.7 channel function. Once baseline measurements of CGRP release from the neurons that express a human or chimeric NaV1.7 are determined both in the presence of a known inhibitor of Naᵥ1.7 (*e.g*., TTX) and the absence of a known inhibitor (*i.e*., inflammatory mediators only), increase in CGRP release can be easily determined when employing candidate antagonists in this assay. Thus, an assay adapted to engage a Naᵥ channel protein such as Naᵥ1.7 is useful, *e.g*., for identification of Naᵥ1.7-specific antagonists through the assessment of their ability to alter or modify the level of CGRP release in DRG isolated from mice expressing a human Naᵥ1.7 protein that are exposed to inflammatory mediators.

Assays are described herein that measure neuropeptide release in neuronal cells in response to one or more inflammatory mediators, which can be used as functional tests for selecting antagonists to a target receptor. Specifically, assays are described herein that measure CGRP release from cultured DRGs for assessment of sodium channel function in the presence and absence of specific antagonists, in particular the DRGs express a human Naᵥ1.7 protein, and that provide novel *in vitro* methods for the evaluation of primary nociceptor activation. In one embodiment, DRG neurons are contacted with one or more inflammatory mediators (*e.g.,* prostaglandin E₂, bradykinin and capsaicin, to specifically engage TTX-sensitive Naᵥ channels, specifically Naᵥ1.7) and the release of CGRP induced by the inflammatory mediators is determined.

Neurotrophins such as GDNF, neurturin and artemin have been shown to enhance capsaicin-induced release of CGRP (Schmutzler, B. S., S. Roy, and C. M. Hingtgen. 2009. Glial cell line-derived neurotrophic factor family ligands enhance capsaicin-stimulated release of calcitonin gene-related peptide from sensory neurons. Neurosci. 161:148-156; Schmutzler, B. S., S. Roy, S. K. Pittman, R. M. Meadows, and C. M. Hingtgen. 2011. Ret-dependent and Ret-independent mechanisms of Gfl-induced sensitization. Molecular Pain 7:1-22). Neurotrophins preferentially bind to their own GDNF family receptor (GFRα1, GFRα2 and GFRα3), and there is a 90% co-expression of GFRα3 with TRPV1, which is also known as the capsaicin receptor. Capsaicin is an agonist of TRPV1 and almost all neurons expressing TRPV1 also express CGRP. Artemin alone does not change the basal level of CGRP release. However, pre-incubation with artemin for ten minutes before stimulation with 1 µM capsaicin significantly enhances CGRP release. Thus, an assay in which neurons are incubated with artemin (*e.g*., at 10 ng/mL) or GDNF (*e.g*., at 10 ng/mL) for ten minutes, followed by capsaicin stimulation for an additional ten minutes, may be employed to specifically engage GFRα3 and assess functional activity of the receptor through measurement of CGRP release from DRG neurons *in vitro.* Additionally, the assay may employ specific antagonists of, *e.g*., GFRα3 to determine their ability to block receptor activity and/or function.

The assay principle can be adapted for another target receptor, for example, TRPA1. Mustard oil, a specific agonist of TRPA1, has been shown to induce the release of CGRP from isolated rat hindpaw skin (Ruparel, N. B., A. M. Patwardhan, A. N. Akopian, and K. M. Hargreaves. 2008. Homologous and heterologous desensitization of capsaicin and mustard oil responses utilize different cellular pathways in nociceptors. Pain 135:271-279). Thus, an assay in which DRG neurons isolated from a mouse as described herein, are exposed to mustard oil could be used to specifically engage TRPA1 to serve as a means to functionally assess activity of TRPA1 through measurement of CGRP release from DRG neurons *in vitro.* Additionally, specific antagonists could be employed in the assay to determine their ability to block TRPA1 activity and/or function.

The assay principle can be adapted for yet another target receptor, for example, ASIC1 and/or ASIC3. ASIC1 and ASIC3 are proton-gated cationic channels that are activated by external pH variations ranging from pH 6.8 to 4.0. Low pH has been shown to trigger CGRP release. Thus, an assay in which DRG neurons isolated from a mouse as described herein, are exposed to low pH conditions in culture could be used to specifically engage ASIC1 (or ASIC3) to serve as a means to functionally assess activity of ASIC1 (or ASIC3) through measurement of CGRP release from DRG neurons *in vitro.* Additionally, specific antagonists could be employed in the assay to determine their ability to block ASIC1 (or ASIC3) activity and/or function.

In one aspect, a method for measuring activity of a target receptor is provided, comprising contacting an inflammatory mediator to a cell having a target receptor encoded by a humanized mouse gene, wherein the inflammatory mediator binds the target receptor, and determining target receptor-mediated neuropeptide release from the cell. In one embodiment, the inflammatory mediator is a Naᵥ1.7 ligand, and the target receptor is a humanized mouse Naᵥ1.7.

In one aspect, a method for selectively engaging a target receptor is provided, comprising contacting an isolated cell from a mouse as described herein with one or more inflammatory mediators, thereby selectively engaging the target receptor and determining the release of a neuropeptide from the isolated cell.

In one embodiment, the target receptor is a sodium channel. In a specific embodiment, the sodium channel is Naᵥ1.7.

In one embodiment, the cell is a neuronal cell. In a specific embodiment, the neuronal cell is a dorsal root ganglion cell (DRG). In one embodiment, the cell is an immortalized cell.

In one embodiment, the one or more inflammatory mediators are selected from prostaglandin E₂, bradykinin, capsaicin, protons, low pH, a neurotrophic factor and a combination thereof.

In one embodiment, the neuropeptide is calcitonin gene-related peptide (CGRP).

In one embodiment, the method is employed as a functional assay to select an antagonist for the target receptor.

In one embodiment, a method is provided for selectively engaging a Naᵥ1.7 protein on the surface of an isolated DRG from a mouse as described herein, comprising contacting the DRG with one or more inflammatory mediators thereby selectively stimulating Naᵥ1.7 and determining the release of CGRP.

In one embodiment, the one or more inflammatory mediators comprises prostaglandin E₂, bradykinin and capsaicin.

In one embodiment, the method is employed as a functional assay to select an antagonist for Naᵥ1.7. In a specific embodiment, the antagonist is an antibody.

In one aspect, a method for measuring the release of CGRP from an isolated DRG from a mouse as described herein is provided, in particular to determine the effect of a Naᵥ-specific antagonist on CGRP release associated with depolarization of a human Naᵥ1.7 channel protein, in particular through stimulation with one or more inflammatory mediators (*e.g*., prostanglin E₂, bradykinin, serotonin, histamine and capsaicin).

In one embodiment, the DRG is immortalized.

In one embodiment, the Naᵥ-specific antagonist is selected from a protein, a small molecule, and a small interfering RNA (siRNA). In a specific embodiment, the Naᵥ-specific antagonist is a protein.

In one embodiment, an isolated DRG from a mouse as described herein is cultured in the presence of a Naᵥ-specific toxin and, after a subsequent period of time, the release of CGRP is determined, wherein an elevated level of CGRP expression indicates inhibition of Naᵥ-specific function in the DRG cell. The identity of the Naᵥ-specific antagonist is known prior to culture with the DRG and the DRG is analyzed for baseline level of CGRP expression in the presence and absence of the one or more inflammatory mediators.

In one embodiment, the Naᵥ-specific toxin is selected from tetrodotoxin (TTX), ProTx-II, and lidocaine, In a specific embodiment, the Naᵥ-specific toxin is TTX.

In one embodiment, an isolated DRG from a mouse as described herein is cultured in the presence of a Naᵥ1.7-specific antibody, and CGRP release is determined, wherein an elevated level of CGRP expression indicates inhibition of Naᵥ1.7 function in the DRG cell. The identity and biochemical profile (*e.g.,* affinity, specificity, etc.) of the Naᵥ1.7-specific antibody is known prior to culture with the DRG and the DRG is analyzed for baseline level of CGRP expression in the presence and absence of the one or more inflammatory mediators.

In one embodiment, the Naᵥ1.7-specific antibody is a human antibody. In one embodiment, the Naᵥ1.7-specific antibody is a mouse antibody. In one embodiment, the Naᵥ1.7-specific antibody is a bispecific antibody.

### EXAMPLES

The following examples are provided so as to describe to those of ordinary skill in the art how to make and use methods and compositions of the invention, and are not intended to limit the scope of what the inventors regard as their invention. Unless indicated otherwise, temperature is indicated in Celsius, and pressure is at or near atmospheric.

### Example I

### Deletion of an Endogenous Naᵥ1.7 Locus

The targeting vector for introducing a deletion of the endogenous Naᵥ1.7 gene was made using VELOCIGENE® technology (see, *e.g*., US Pat. No. 6,586,251 and Valenzuela, D.M., Murphy, A.J., Frendewey, D., Gale, N.W., Economides, A.N., Auerbach, W., Poueymirou, W.T., Adams, N.C., Rojas, J., Yasenchak, J., Chernomorsky, R., Boucher, M., Elsasser, A.L., Esau, L., Zheng, J., Griffiths, J.A., Wang, X., Su, H., Xue, Y., Dominguez, M.G., Noguera, I., Torres, R., Macdonald, L.E., Stewart, A.F., DeChiara, T.M., Yancopoulos, G.D. 2003. High-throughput engineering of the mouse genome coupled with high-resolution expression analysis. Nature Biotech. 21(6): 652-659) to modify the Bacterial Artificial Chromosome (BAC) RP23-454H3 (Invitrogen). RP23-454H3 BAC DNA was modified to delete the endogenous Naᵥ1.7 gene comprising the α-subunit of this Naᵥ channel gene that is expressed on the cell surface (FIG. 2).

Briefly, upstream and downstream homology arms were derived mouse BAC DNA from locations 5' of exon 6 and 3' of exon 28 of the endogenous Naᵥ1.7 locus, respectively. These homology arms were used to make a cassette that deleted ~81 kb of the endogenous Naᵥ1.7 locus comprising exons 6 to 28. This region was replaced with a neomycin cassette flanked by loxP sites (FIG. 2, middle). The final targeting vector from 5' to 3' included a 17 kb homology arm comprising mouse genomic sequence 5' to exon 6 of the endogenous Naᵥ1.7 locus, a 5' loxP site, a neomycin cassette, a 3' loxP site and a 29 kb homology arm comprising mouse genomic sequence 3' to exon 28 of the endogenous Naᵥ1.7 locus. The targeting vector was linearized by digesting with Agel and then used in homologous recombination in bacterial cells containing the mouse BAC clone RP23-454h3 to achieve a targeted deletion of the endogenous Naᵥ1.7 locus (FIG. 2, bottom).

The targeted BAC DNA (described above) was used to electroporate mouse ES cells to created modified ES cells comprising a deletion of the endogenous Naᵥ1.7 locus. Positive ES cells containing a deleted endogenous Naᵥ1.7 locus were identified by the quantitative PCR assay using TAQMAN® probes (Lie, Y. S. and C. J. Petropoulos. 1998. Curr. Opin. Biotechnology 9: 43-48). The upstream region of the deleted locus was confirmed by PCR using primers 867TUP2F (GGGACTTCTC TGGGTTCAGT TA; SEQ ID NO: 1) and 867TUP2R (AAAGGCTCTC AATGGGAAAC AAG; SEQ ID NO: 2) and probe 867TUP2P (TCAATGACTT GACATAATGC ATGCACTCC; SEQ ID NO: 3), whereas the downstream region of the deleted locus was confirmed using primers 867TDPF (ATGTCAGCCA ATCCTTCTAA AGTG; SEQ ID NO:4) and 867TDPR (CGTTTTGCCT AAGGCGGTAC; SEQ ID NO:5) and probe 867TDPP (TCCTATGAGC CCATCACAAC CACAC; SEQ ID NO: 6). The presence of the neomycin cassette from the targeting vector was confirmed using primers NEOF (GGTGGAGAGG CTATTCGGC; SEQ ID NO:7) and NEOR (GAACACGGCG GCATCAG; SEQ ID NO: 8) and probe NEOP (TGGGCACAAC AGACAATCGG CTG; SEQ ID NO: 9). The nucleotide sequence across the upstream deletion point included the following, which indicates endogenous mouse sequence upstream of the deletion point (contained within the parentheses below) linked contiguously to cassette sequence present at the deletion point: (CTAGCTGAGC TGTCACCACA CATTGCTCCT ACCACGTATT GTACAGCTAC TGCAAGAGCA CCACAGTTGG CTTTCTGTAT C) ATAACTTCGT ATAATGTATG CTATACGAAG TTAT (SEQ ID NO: 10). The nucleotide sequence across the downstream deletion point included the following, which indicates cassette sequence contiguous with endogenous mouse sequence downstream of the deletion point (contained within the parentheses below): ATAACTTCGT ATAATGTATG CTATACGAAG TTAT (AGCTTCGGTT TTGATACACT GTTTACAGCC TGCGAAGGTG ACTCACTCGT GTTAATAAGA CTCTTTTACG GAGGTCTATG CCAAACTCTT TTTATCAAAT ATTCTCAAAG GCAG) (SEQ ID NO: 11). Positive ES cell clones were then used to implant female mice using the VELOCIMOUSE® method (described below) to generate a litter of pups containing a deletion of the endogenous Naᵥ1.7 locus.

Targeted ES cells described above were used as donor ES cells and introduced into an 8-cell stage mouse embryo by the VELOCIMOUSE® method (see, *e.g*., US Pat. No. 7,294,754 and Poueymirou, W.T., Auerbach, W., Frendewey, D., Hickey, J.F., Escaravage, J.M., Esau, L., Dore, A.T., Stevens, S., Adams, N.C., Dominguez, M.G., Gale, N.W., Yancopoulos, G.D., DeChiara, T.M., Valenzuela, D.M. 2007. F0 generation mice that are essentially fully derived from the donor gene-targeted ES cells allowing immediate phenotypic analyses. Nature Biotech. 25(1): 91-99). Mice bearing a deletion of exons 6 to 28 in the endogenous Naᵥ1.7 locus were identified by genotyping using a modification of allele assay (Valenzuela et al., *supra)* that detected the presence of the neomycin cassette and confirmed the absence of endogenous Naᵥ1.7 sequences.

Mice bearing a deletion of exons 6 to 28 in the endogenous Naᵥ1.7 locus can be bred to a Cre deletor mouse strain (see, *e.g.,* International Patent Application Publication No. WO 2009/114400) in order to remove any loxed neomycin cassette introduced by the targeting vector that is not removed, *e.g.,* at the ES cell stage or in the embryo. Optionally, the hygromycin cassette is retained in the mice.

Pups are genotyped and a pup heterozygous for the deleted endogenous Naᵥ1.7 sequences is selected for characterizing endogenous Naᵥ1.7 deletion.

### Example II

### Humanization of an Endogenous Naᵥ1.7 Locus

A targeting vector for replacement of the endogenous Naᵥ1.7 locus with the human Naᵥ1.7 locus (FIG. 3) was constructed using a two step process involving ligation of BAC DNA and GAP repair (Zhang, Y., J. P. P. Muyrers, G. Testa, and F. A. Stewart. 2000. DNA cloning by homologous recombination in Escherichia coli. Nature Biotechnology 18:1314-1317; Zhang, Y., F. Buchholz, J. P. P. Muyrers, and F. A. Stewart. 1998. A new logic for DNA engineering using recombination in Escherichia coli. Nature Genetics 20:123-128).

The first step in constructing the replacement targeting vector was performed by ligation of a DNA fragment of mouse BAC DNA clone RP23-454H3 with a human DNA fragment from human BAC clone RP11-1002M1 (Invitrogen). This ligation of mouse and human BAC DNA fragments created a modified BAC clone containing a replacement of exons 5 to 28 of the mouse Naᵥ1.7 locus (about 81 kb) with exons 5 to 28 of the human Naᵥ1.7 locus (about 100 kb).

The second step in constructing the replacement targeting vector was performed by GAP repair (referenced above) using mouse BAC clone RP23-454H3 and human BAC clone RP11-45AJ20 to add additional exons of the human Naᵥ1.7 locus to the modified BAC clone made in the first step. GAP repair was performed on using these mouse and human BAC clones to replaced exons 2 to 7 of the endogenous Naᵥ1.7 locus with exons 2 to 7 of the human Naᵥ1.7 locus (~13 kb). This second step added the ~13 kb of human Naᵥ1.7 sequence to the ~100 kb of human Naᵥ1.7 sequence to make the replacement of the endogenous Naᵥ1.7 locus. A hygromycin cassette flanked by loxP sites was added to the 3' end of the ~113 kb BAC fragment containing the human Naᵥ1.7 locus (FIG. 3, middle)

Upstream and downstream homology arms were derived from mouse BAC DNA at positions 5' and 3' of the endogenous Naᵥ1.7 locus for addition to the human DNA fragment-hygromycin cassette to create the final targeting vector for replacement of the endogenous Naᵥ1.7 locus which contained from 5' to 3' a 5' homology arm containing 70 kb of mouse DNA 5' of the endogenous Naᵥ1.7 locus, a ~113 kb DNA fragment containing exons 2 to 28 of the human Naᵥ1.7 locus, a hygromycin cassette flanked by loxP sites, and a 3' homology arm containing 147 kb of mouse DNA 3' of the endogenous Naᵥ1.7 locus. The targeting vector was linearized by digesting with Notl and then used in homologous recombination in bacterial cells to achieve a targeted replacement of the endogenous Naᵥ1.7 locus with exons 2 to 28 of the human Naᵥ1.7 locus (FIG. 3, bottom).

The targeted BAC DNA (described above) was used to electroporate mouse ES cells to created modified ES cells comprising a replacement of the endogenous mouse Naᵥ1.7 locus with a genomic fragment comprising a human Naᵥ1.7 locus. Positive ES cells containing a deleted endogenous Naᵥ1.7 locus replaced by a genomic fragment comprising a human Naᵥ1.7 locus were identified by a quantitative PCR assay using Taqman□ probes (Lie and Petropoulos, *supra*). The upstream and downstream regions outside of the modified locus were confirmed by PCR using the same primers and probes as described in Example 1 (867TUP2F/867TUP2R/867TUP2P and 867TDPF/867TDPR/867TDPP). The insertion of the human Naᵥ1.7 sequence was confirmed by PCR using primers 935HF (ATCAAAGGAA CCCAAAGAAG; SEQ ID NO: 12) and 935HR (GAAGGGCAGC TGTTTGCCAG; SEQ ID NO: 13) and probe 935HP (ATGAAGAAGC CCCAAAGCCA AGCA; SEQ ID NO: 14). The presence of the hygromycin cassette from the targeting vector was confirmed with primers HYGF (TGCGGCCGATCTTAGCC; SEQ ID NO: 15) and HYGR (TTGACCGATTCCTTGCGG; SEQ ID NO: 16) and probe HYGP (ACGAGCGGGTTCGGCCCATTC; SEQ ID NO: 17). The nucleotide sequence across the upstream insertion point included the following, which indicates endogenous mouse sequence upstream of the insertion point (contained within the parentheses below) linked contiguously to human Naᵥ1.7 genomic sequence present at the insertion point: (TTAGGTAAGG ATCCGAAGGG GAAATAAAAC CTACAGGATG AGAAG) ATGGCAATGT TGCCTCCCCC AGGACCTCAG AGCTTTGTCC ATTTCACAAA ACAG (SEQ ID NO: 18). The nucleotide sequence across the downstream insertion point at the 5' end of the hygromycin cassette included the following, which indicates human Naᵥ1.7 genomic sequence contiguous with cassette sequence downstream of the insertion point (contained within the parentheses below): GTATGAATAA AAAAGCATTG AAATAGGGAT TCTTGCCAAC TTGCTC (TCTCGAGATA ACTTCGTATA ATGTATGCTA TACGAAGTTA T) (SEQ ID NO: 19). The nucleotide sequence across the downstream insertion point at the 3' end of the hygromycin cassette included the following, which indicates cassette sequence contiguous with mouse genomic sequence at the 3' end of the endogenous Naᵥ1.7 locus (contained within the parentheses below): TATACGAAGT TATGCTAGTA ACTATAACGG TCCTAAGGTA GCGAGCTAG (CAGCTTCGGT TTTGATACAC TGTTTACAGC CTGCGAAGGT G) (SEQ ID NO: 20). Positive ES cell clones were then used to implant female mice using the VELOCIMOUSE® method (*supra*) to generate a litter of pups containing a replacement of the endogenous Naᵥ1.7 locus with a human Naᵥ1.7 locus.

Targeted ES cells described above were used as donor ES cells and introduced into an 8-cell stage mouse embryo by the VELOCIMOUSE® method (*supra*)*.* Mice bearing a human Naᵥ1.7 locus were identified by genotyping using a modification of allele assay (Valenzuela et al., *supra*) that detected the presence of a human Naᵥ1.7 locus.

Mice bearing a human Naᵥ1.7 locus can be bred to a Cre deletor mouse strain (see, e.g., International Patent Application Publication No. WO 2009/114400) in order to remove any loxed hygromycin cassette introduced by the targeting vector that is not removed, e.g., at the ES cell stage or in the embryo. Optionally, the hygromycin cassette is retained in the mice.

Pups are genotyped and a pup heterozygous for a human Naᵥ1.7 locus is selected for characterizing Naᵥ1.7 humanization.

### Example III

### Humanization of the Extracellular Loop of Transmembrane Segments 5 to 6 in Domain I of an Endogenous Naᵥ1.7 Locus

A targeting vector for humanization of the extracellular pore loop connecting transmembrane segments 5 and 6 of Domain I (DI/S5-S6) was constructed by the GAP repair method (described above) using mouse BAC clone RP23-20C24 and human BAC clone RP11-45AJ20 (FIG. 4). The GAP repair method was used to replace a 13.8 kb DNA fragment containing exons 7 to 9 of the endogenous Naᵥ1.7 locus with a 10 kb DNA fragment containing exons 7 to 9 of the human Naᵥ1.7 locus. A neomycin cassette flanked by loxP sites was added to the end of the 10 kb human DNA fragment containing exons 7 to 9 of the human Naᵥ1.7 locus (FIG 4, middle).

Upstream and downstream homology arms were derived from mouse BAC DNA at positions 5' and 3' of exons 7 and 9, respectively, and added to the 10 kb human fragment-neomycin cassette to create the final targeting vector for humanization of the extracellular pore loop connecting transmembrane segments 5 and 6 of Domain I of the endogenous Naᵥ1.7 locus which contained from 5' to 3' a 5' homology arm containing 35 kb of mouse DNA 5' of exon 7 of the endogenous Naᵥ1.7 locus, a 10 kb DNA fragment containing exons 7 to 9 of the human Naᵥ1.7 locus, a neomycin cassette flanked by loxP sites, and a 3' homology arm containing 27 kb of mouse DNA 3' of exon 9 of the endogenous Naᵥ1.7 locus. The targeting vector was linearized by digesting with PspX and Sall and then used in homologous recombination in bacterial cells to achieve a targeted replacement of exons 7 to 9 in endogenous Naᵥ1.7 locus with exons 7 to 9 of a human Naᵥ1.7 gene (FIG. 4, bottom).

The targeted BAC DNA (described above) was used to electroporate mouse ES cells to created modified ES cells comprising a replacement of exons 7 to 9 in the endogenous mouse Naᵥ1.7 locus with a genomic fragment comprising exons 7 to 9 of a human Naᵥ1.7 locus. Positive ES cells containing a genomic fragment comprising exons 7 to 9 of a human Naᵥ1.7 gene were identified by the quantitative PCR assay using Taqman□ probes (Lie and Petropoulos, *supra*). The upstream region outside of the modified region of the endogenous locus were confirmed by PCR using primers 869TUPF (GGACTACAAC TGTTTATGGG CAAC; SEQ ID NO: 21) and 869TUPR (TCAATTCTTC TTCACTCTCA GCAG; SEQ ID NO: 22) and probe 869TUPP (TCCGGAAGGA CCTTGAGCAG AATGA; SEQ ID NO: 23), whereas the downstream region outside the modified region of the endogenous locus was confirmed with primers 869TDPF (CAACAGGTGA GCAGCAACAG; SEQ ID NO: 24) and 869TDPR (GCAGGAGACA CATACACCAG AC; SEQ ID NO: 25) and probe 869TDPP (AAACACGCAT GTCTGAAGGC AGTCGG; SEQ ID NO: 26). The presence of the neomycin cassette from the targeting vector was confirmed using the same primers and probe as described in Example 1. The nucleotide sequence across the upstream insertion point included the following, which indicates endogenous mouse sequence upstream of the insertion point (contained within the parentheses below) linked contiguously to human Naᵥ1.7 genomic sequence present at the insertion point: (TACATTTTAA GGACTAAAAA CCATCGTGGG GGCCCTGATC CAATCAGTGA AGAAGCTCTC TGACGTCATG ATCCTCACTG TGTTCTGTCT CAGTGTGTTC) GCACTAATTG GACTACAGCT GTTCATGGGA AACCTGAAGC ATAAATGTTT TCGAAATTCA CTTGAAAATA ATGAAACATT AGAAAGCATA ATGAATACCC T (SEQ ID NO: 27). The nucleotide sequence across the downstream insertion point at the 5' end of the neomycin cassette included the following, which indicates human Naᵥ1.7 genomic sequence contiguous with cassette sequence downstream of the insertion point (contained within the parentheses below): AGGTGAGTAC CAAGAGAAAC ATGCATTGTA TTTTTGAATG GCATATGTAC CTGGTGTATG TTAAGAGCCT GTATTAGGAG GTTTTTTATT TATTTGAGAA TGGAGGAAAC TCTATTA (CTCGAGATAA CTTCGTATAA TGTATGCTAT ACGAAGTTAT) (SEQ ID NO: 28). The nucleotide sequence across the downstream insertion point at the 3' end of the neomycin cassette included the following, which indicates cassette sequence contiguous with mouse genomic sequence 3' of exon 9 of the endogenous Naᵥ1.7 locus (contained within the parentheses below): TATACGAAGT TATGCTAGC (TCTGCAGACA GTCTGGGACT CCCTAATGTG CATTATTAAA ATTACAGGCA ATTTACTTGG CTGATATGAG AACAGATAGT TCTGAAGTCA TCAATAATTT TCTGCTGTGT CTGACCAGCG TT) (SEQ ID NO: 29). Positive ES cell clones were then used to implant female mice using the VELOCIMOUSE® method (described below) to generate a litter of pups containing a replacement of exons 7 to 9 of the endogenous Naᵥ1.7 locus with the corresponding exons from the human Naᵥ1.7 locus.

Targeted ES cells described above were used as donor ES cells and introduced into an 8-cell stage mouse embryo by the VELOCIMOUSE® method (*supra*)*.* Mice bearing the humanization of exons 7 to 9 of the endogenous Naᵥ1.7 locus were identified by genotyping using a modification of allele assay (Valenzuela et al., *supra)* that detected the presence of the human Naᵥ1.7 sequences.

Mice bearing the humanized DI/S5-S6 in the endogenous Naᵥ1.7 locus can be bred to a Cre deletor mouse strain (see, *e.g*., International Patent Application Publication No. WO 2009/114400) in order to remove any loxed neomycin cassette introduced by the targeting vector that is not removed, *e.g.,* at the ES cell stage or in the embryo. Optionally, the neomycin cassette is retained in the mice.

Pups are genotyped and a pup heterozygous for the humanized DI/S5-S6 in the endogenous Naᵥ1.7 locus is selected for characterizing Naᵥ1.7 DI/S5-S6 humanization.

### Example IV

### Humanization of the Extracellular Loop of Transmembrane Segments 5 to 6 in Domain III of an Endogenous Naᵥ1.7 Locus

A targeting vector for humanization of the extracellular pore loop connecting transmembrane segments 5 and 6 of Domain III (DIII/S5-S6) was constructed by polymerase chain reaction (PCR) using mouse BAC clone BMQ-311E20 and human BAC clone RP11-746P5 (FIG. 5). Exons 23 to 25 of the human Naᵥ1.7 locus were amplified from human BAC clone RP11-746P5. A neomycin cassette flanked by loxP sites was ligated to the 3' end of the 2.8 kb PCR fragment (FIG. 5, middle). This ligated DNA fragment containing exons 23 to 25 of the human Naᵥ1.7 locus and the neomycin cassette was used to replace a 2.4 kb section of the endogenous mouse Naᵥ1.7 locus containing exons 23 to 25 in the mouse BAC clone BMQ-311E20 (FIG 5).

Upstream and downstream homology arms were derived from mouse BAC DNA at positions 5' and 3' of exons 23 and 25, respectively, and added to the human DNA fragment-neomycin cassette to create the final targeting vector for humanization of DIII/S5-S6 of the endogenous Naᵥ1.7 locus which contained from 5' to 3' a 5' homology arm containing 21 kb of mouse DNA 5' of exon 23 of the endogenous Naᵥ1.7 locus, a 2.8 kb DNA fragment containing exons 23 to 25 of the human Naᵥ1.7 locus, a neomycin cassette flanked by loxP sites, and a 3' homology arm containing 108 kb of mouse DNA 3' of exon 25 of the endogenous Naᵥ1.7 locus. The targeting vector was linearized by digesting with Notl and then used in homologous recombination in bacterial cells to achieve a targeted replacement of exons 23 to 25 in endogenous Naᵥ1.7 locus with exons 23 to 25 of the human Naᵥ1.7 locus (FIG. 5, bottom).

The targeted BAC DNA (described above) was used to electroporate mouse ES cells to created modified ES cells comprising a replacement of exons 23 to 25 in the endogenous mouse Naᵥ1.7 locus with a genomic fragment comprising exons 23 to 25 of a human Naᵥ1.7 locus. Positive ES cells containing a genomic fragment comprising exons 23 to 25 of a human Naᵥ1.7 gene were identified by the quantitative PCR assay using TAQMAN® probes (Lie and Petropoulos, *supra*). The upstream region outside of the modified region of the endogenous locus were confirmed by PCR using primers 892TUPF (GCTTGGGCTT GCACCTTTA; SEQ ID NO: 30) and 892TUPR (TGCGTTGACC ACTACCTGAT AC; SEQ ID NO: 31) and probe 892TUPP (TCTGCATTGG CGTCTGTTTG TCA; SEQ ID NO: 32), whereas the downstream region outside the modified region of the endogenous locus was confirmed with primers 892TDP3F (TGACTTGCCC TATCAATCTG AGATC; SEQ ID NO: 33) and 892TDP3R (GCTCACACTG TATACACACA AAATCTTC; SEQ ID NO: 34) and probe 892TDP3P (TCACTGCCTA TGATAAAGT; SEQ ID NO: 35). The presence of the neomycin cassette from the targeting vector was confirmed using the same primers and probe as described in Example 1. The insertion of exons 23 to 25 of the human Naᵥ1.7 gene was confirmed by PCR using primers 892HF (CACGGTTTCC TGCAAGTCAA; SEQ ID NO: 36) and 892HR (GGGACACTTA CAACTTGAAG CA; SEQ ID NO: 37) and probe 892HP (TCGTTCCGAA TGTTTTGCCC TTATGA; SEQ ID NO: 38). The nucleotide sequence across the upstream insertion point included the following, which indicates mouse genomic sequence upstream of exon 23 of the endogenous Naᵥ1.7 locus (contained within the parentheses below) linked contiguously to human Naᵥ1.7 genomic sequence present at the insertion point: (TTTCATTTAT TTGAAGTGCA ATATCATCTT GGCCATCTAC TCCTCTGTAT GCTAGTAG) GTAAGCCTGG TGATCACAGA (SEQ ID NO: 39). The nucleotide sequence across the downstream insertion point at the 5' end of the neomycin cassette included the following, which indicates human Naᵥ1.7 genomic sequence contiguous with cassette sequence downstream of the insertion point (contained within the parentheses below): GACTAGTATA CAATTACAAA TATGC (CTCGAGATAA CTTCGTATAA TGTATGCTAT ACGAAGTTAT) (SEQ ID NO: 40). The nucleotide sequence across the downstream insertion point at the 3' end of the neomycin cassette included the following, which indicates cassette sequence contiguous with mouse genomic sequence 3' of exon 25 of the endogenous Naᵥ1.7 locus (contained within the parentheses below): TATACGAAGT TATGCTAGC (TTTCCTGCTA ACCATCATTC TGGGGTATGT GTTATGATGG AAGTTAAGTG ACAGTTACTT ATAATATGGC TGCT) (SEQ ID NO: 41). Positive ES cell clones were then used to implant female mice using the VELOCIMOUSE® method (described below) to generate a litter of pups containing a replacement of exons 23 to 25 of the endogenous Naᵥ1,7 locus with the corresponding exons from the human Naᵥ1.7 locus.

Mice containing a humanization of exons 23 to 25 in the endogenous Naᵥ1.7 locus with the human Naᵥ1.7-DIII/S5-S6 targeting vector were generated through electroporation of a targeted BAC DNA (described above) into mouse ES cells. Positive ES cells clones were confirmed by Taqman™ screening and karyotyping. Positive ES cell clones were then used to implant female mice using the VELOCIMOUSE® method (described below) to generate a litter of pups containing a humanization of exons 23 to 25 in the endogenous Naᵥ1.7 locus.

Targeted ES cells described above were used as donor ES cells and introduced into an 8-cell stage mouse embryo by the VELOCIMOUSE® method (see, *e.g*., US Pat. No. 7,294,754 and Poueymirou et al., *supra).* Mice bearing the humanization of exons 23 to 25 of the endogenous Naᵥ1.7 locus were identified by genotyping using a modification of allele assay (Valenzuela et al., *supra)* that detected the presence of the human Naᵥ1.7 sequences.

Mice bearing the humanized DIII/S5-S6 in the endogenous Naᵥ1.7 locus can be bred to a Cre deletor mouse strain (see, *e.g*., International Patent Application Publication No. WO 2009/114400) in order to remove any loxed neomycin cassette introduced by the targeting vector that is not removed, *e.g*., at the ES cell stage or in the embryo. Optionally, the neomycin cassette is retained in the mice.

Pups are genotyped and a pup heterozygous for the humanized DIII/S5-S6 in the endogenous Naᵥ1.7 locus is selected for characterizing Naᵥ1.7 DIII/S5-S6 humanization.

### Example V

### Behavioral Phenotyping of Humanized Naᵥ1.7 Mice

Current methods for studying the effects of pharmacological manipulation of human Naᵥ1.7 rely on transfected cells that are cultured *in vitro.* These cells that are engineered to express human Naᵥ1.7 protein lack auxiliary proteins and might not be fully representative of the mechanisms by which human Naᵥ1.7 functions *in vivo.* Thus, mice engineered to express human Naᵥ1.7 or a chimeric Naᵥ1.7 having a humanized extracellular pore loop as described in Examples 2 - 4 were generated and analyzed to understand the function of Naᵥ1.7 *in vivo.*

Briefly, two groups (n=6/6 each; male/female at 10-20 weeks) of mice, wild type (*Scn9a*^{+/+}) and mice heterozygous for a replacement of a mouse Naᵥ1.7 gene with a human Nav1.7 gene (*Scn9a*^{*hum*/+}), were each subjected to a variety of nocifensive stimuli (hot plate thermal, tail flick thermal and noxious mechanical pressure). Results are shown in FIGs. 6A - 6C.

In a similar experiment, two groups (n=10 each; female at 10-20 weeks) of mice, wild type (*Scn9a*^{+/+}) and mice homozygous for a chimeric Naᵥ1.7 gene that contains a human sequence that encodes an extracellular pore loop (DI/S5-S6; *Scn9a*^{*3.1*/*3.1*}), were each subjected to a variety of nocifensive stimuli (hot plate thermal, tail flick thermal, noxious mechanical, and inflammatory hypernociception using Complete Freund's Adjuvant). Results are shown in FIGs. 7A - 7D.

No significant difference in any of the acute endpoints between *Scn9a*^{+/+} and *Scn9a*^{*hum*/*+*} or *Scn9a*^{*+*/*+*} and *Scn9a*^{*3.1*/*3.1*} mice was observed. These results demonstrate that humanized mice containing either a full-length human Naᵥ1.7 gene in place of the endogenous gene (as described in Example 2) or a human sequence that encodes an extracellular pore loop (as described in Example 3) display normal nociceptive behaviors in response to nocifensive stimuli as compared to wild-type control mice.

As shown in this Example, mice engineered to express complete or partially human Naᵥ1.7 protein on the surface of neurons respond to nociceptive stimuli in a similar fashion as compared to wild type and thus provide a platform for identifying antagonists of the α-subunit of Naᵥ1.7 and/or a particular extracellular pore loop. Such antagonists may be useful in blocking specific functions and associated neuronal activities in the treatment of several clinical pain syndromes.

### Example VI

### Identification and Function of DRG in Humanized Naᵥ1.7 Mice

Human antibodies specific for human Naᵥ1.7 were generated and analyzed for binding to neuronal cells isolated from the humanized Naᵥ1.7 mice described in Examples 2 - 4.

Briefly, VELOCIMMUNE^{®} mice (U.S. 6,596,541) were administered human Naᵥ1.7 (hNaᵥ1.7) antigen with adjuvant (*e.g*. complete or incomplete Freund's adjuvant, MPL+TDM adjuvant system (Sigma), or RIBI (muramyl dipeptides) (see O'Hagan 2000 Vaccine Adjuvant, by Human Press, Totowa, NJ)). The immune response was monitored and antibodies containing human variable regions and mouse constant regions were isolated from hybridoma cell lines made from antibody-expressing B cells harvested from immunized mice. Alternatively, antigen-specific hybridoma cells may be isolated by flow cytometry. Antibodies specific to hNaᵥ1.7 may also be identified via direct isolation of splenocytes (*e.g*. see U.S. 7,582,298). Several anti-hNaᵥ1.7 antibodies were obtained by the foregoing methods and HEK293 cells engineered to stably express human Naᵥ1.7 or human Naᵥ1.5 were used to identify antibodies that specifically bind to Naᵥ1.7 but not to Naᵥ1.5 as determined by flow cytometry.

Selected anti-hNaᵥ1.7 antibodies were tested to determine their ability to block electrophysiological function (*e.g*. current, ion flux, etc.) of the Naᵥ1.7 channel using the IONWORKS QUATTRO™ system (IWQ, Molecular Devices), the Port-a-Patch® system (Nanion Technologies) and QPatch system (Sophion Bioscience). Table 1 sets forth the average percent inhibition for five selected anti-hNaᵥ1.7 antibodies: less than 15% (-), 15-30% (+), greater than 30% (++). nd: not determined.

**Table 1**

| **Antibody** | **IWQ (222 nM)** | **Port-a-Patch (50 nM)** | **QPatch (100 nM)** |
|---|---|---|---|
| Ab1 | ++ | ++ | + |
| Ab2 | ++ | nd | + |
| Ab3 | ++ | ++ | + |
| Ab4 | ++ | + | + |
| Ab5 | - | + | + |

**Immunohistochemistry.** Pain processing is the result of complex interactions among several proteins, receptors and channels that are expressed in dorsal root ganglion (DRG) nociceptive neurons. Selected anti-hNaᵥ1.7 antibodies were evaluated for binding to DRG neurons harvested from mice engineered to express human Naᵥ1.7 (*Scn9a*^{*hum*/+}, Example 2), mice engineered to express a chimeric Naᵥ1.7 containing a human extracellular pore loop (*Scn9a*^{*3.1*/}*^{3.1},* Example 3) and wild type mice (*Scn9a^{+!+}*)*.*

Briefly, harvested lumbar DRGs from *Scn9a*^{*hum*/+}, *Scn9a*^{*3.1*/*3.1*} and *Scn9a*^{+/+} mice and *Scn9a*^{+/+} rats (*rScn9a*^{+/+}) were dissociated and plated at a density of 5.5x10⁴ cells/well on 96 well plates treated with poly-DL-ornithine (0.1 mg/mL) and laminin (5 µg/mL) followed by incubation at 37°C in 96.5% air and 3.5% CO₂. Neurons were maintained in culture for 3 to 8 days in DMEM supplemented with 50 ng/mL nerve growth factor, 100 U/mL penicillin/streptomycin, MEM vitamins, and 10% heat-inactivated fetal calf serum. Plated cells were then fixed in 4% PFA and 4% sucrose in PBS pH7.2 for 30 minutes. Cells were then washed in PBS followed by blocking in 20% normal goat serum for one hour at room temperature. Neurons were then permeabilized in 10% normal goat serum with 0.1% Triton X-100 before immunostaining to confirm binding activity to a human or chimeric Naᵥ1.7 (or mouse and/or rat) on the cell surface of the DRG neurons. Table 2 sets forth the species-specific binding (+) or no binding (-) to DRG neurons for selected anti-NaV1.7 antibodies.

**Table 2**

| **Antibody** | **DRG Genotype** | | | |
|---|---|---|---|---|
| | ***Scn9a*^{*hum*/*+*}** | ***Scn9a*^{*3.1*/*3.1*}** | ***Scn9a*^{*+*/*+*}** | ***rScn9a*^{*+*/*+*}** |
| Ab1 | - | | - | - |
| Ab2 | | | + | - |
| Ab3 | + | | - | +/- |
| Ab4 | + | + | + | + |
| Ab5 | +/- | | - | - |

Selected anti-hNaᵥ1.7 antibodies demonstrated binding to DRG neurons expressing a human Naᵥ1.7 protein (*e.g.,* Ab3, Ab4, and Ab5), a chimeric Naᵥ1.7 protein (*e.g.,* Ab4) or a mouse Naᵥ1.7 protein (*e.g.,* Ab2) on the cell surface, while no binding to DRG neurons expressing a mouse or rat Naᵥ1.7 protein was observed for other antibodies (*e.g.,* Ab1 and Ab5). Other anti-hNaᵥ1.7 antibodies demonstrated species cross-reactivity in that the antibodies showed binding to DRG neurons from humanized Naᵥ1.7 mice, wild type mice and rats (*e.g*., Ab3 and Ab4).

**Calcitonin Gene-Related Peptide (CGRP) Release Assay.** The neuropeptide calcitonin gene-related peptide (CGRP) is released from peripheral and spinal terminals of peptidergic A- and C-fibers nociceptive neurons in response to stimuli. Neuropeptide release initiates neurogenic inflammation, degranulation of mast cells, and other inflammatory reactions, which results in hyperalgesia/pain sensation. Inflammatory mediators such as Prostanglin E2, Bradykinin, Serotonin, Histamine and Capsaicin directly sensitize and excite nociceptive DRG *in vitro* and *in vivo* thereby leading to CGRP release. Sensitized nociceptors display a lowered threshold of activation, increased spontaneous activity and an increased response to suprathreshold stimuli. Thus, sensitization of DRGs and firing of action potentials can be achieved *in vitro* with different inflammatory mediators resulting in the release of CGRP and thereby serve as a means to measure DRG nociceptive function. Primary nociceptor activation was determined in primary *in vitro* DRG cultures isolated from humanized Naᵥ1.7 and wild type mice by measurement of CGRP release using an enzymye immunoassay (EIA) kit (Cayman, distributed by SPlbio, France).

Briefly, DRGs between 3 to 8 days old were washed once in assay buffer and kept at 37°C until addition of an inflammatory mix (*e.g*. 10 µM Prostanglin E2, 10 µM Bradykinin, and 1 µM Capsaicin). Two known inhibitors of Naᵥ channels, Tetrodotoxin (TTX) and ProTx-II, were used to determine whether Naᵥ1.7 plays a role in an inflammatory mix induced-release of CGRP *in vitro.* TTX was tested at concentrations of 1 µM, an effective inhibitory concentration for TTX-sensitive channels, and 10 µM. ProTx-II largely inhibits Naᵥ1.7 at 10 nM (Schmalhofer, W.A, Calhoun, J., Burrows, R., Bailey, T., Kohler, M.G., Weinglass, A.B., Kaczorowski, G.J., Garcia, M.L., Koltzenburg, M., Priest, B.T. (2008) ProTx-II, a Selective Inhibitor of Nav1.7 Sodium Channels, Blocks Action Potential Propagation in Nociceptors. Molecular Pharmacology 74(5):1476-1484). Neurons were incubated for 20 minutes with the inhibitor (TTX or ProTx-II), followed by stimulation with the inflammatory mix for 20 minutes or with the inhibitor combined with the inflammatory mix for 20 minutes. Compound dilutions were prepared in assay buffer and samples were added in duplicate onto a Human CGRP EIA plate and incubated overnight. Concentration of CGRP released by DRGs was measured the following day using an ELISA assay. Averages (mean +/-standard deviation) were calculated for each condition group. FIG. 8 shows the dose response for different concentrations of DRGs in the CGRP assays using TTX under the conditions mentioned above. The results for TTX are shown in Table 3. The results for ProTx-II are shown in Table 4. IM: inflammatory mix.

In a similar experiment, the role of Naᵥ1.7 in the TTX-potentiating release of CGRP in DRGs harvested from *Scn9a*^{*3.1*/*3.1*} mice was tested using an amino amide-type local anesthetic (Lidocaine). This experiment was performed to confirm that the enhancement of CGRP release was not due to a non-specific effect of the toxins on DRG neurons. The results are shown in Table 5. IM: inflammatory mix.

In another similar experiment, selected anti-hNaᵥ1.7 antibodies were analyzed for their effect on *in vitro* CGRP release in DRG isolated from *Scn9a*^{*+*/}*⁺, Scn9a*^{*hum*/+} and *Scn9a*^{*3.1*/*3.1*} mice. The results are shown in Tables 6 - 9. IM: inflammatory mix.

As shown in this Example, when neurons (DRGs) expressing a wild type or chimeric Naᵥ1.7 protein on the cell surface are pre-incubated with known inhibitors of Naᵥ1.7 (TTX or ProTx-II, Table 3 or 4, respectively) or a non-Naᵥ1.7 specific local anesthetic (Lidocaine, Table 5), the inflammatory mix-induced release of CGRP is notably enhanced. However, when either a Naᵥ1.7 inhibitor or local anesthetic was added to the inflammatory mix prior exposure to the neurons, no enhancement of the inflammatory-induced release of CGRP was observed. Thus, these experiments show that inhibition of Naᵥ1.7 before stimulation with inflammatory mediators (Prostanglin E₂, Bradykinin, and Capsaicin) potentiates the release of CGRP in DRG neurons from wild type and humanized mice.

Further, when neurons (DRGs) are pre-incubated with an anti-hNaᵥ1.7 antibody, the inflammatory mix-induced release of CGRP is notably enhanced (Tables 6 - 9). These experiments demonstrate that the human and chimeric Naᵥ1.7 proteins expressed on the surface of DRGs in humanized mice are functional. This Example demonstrates that anti-hNaᵥ1.7 antibodies were able to mimic TTX-mediated enhancement of inflammatory mediator-induced CGRP release in DRG neurons from *Scn9a*^{*hum*/+} and *Scn9a*^{*3.1*/*3.1*} mice. Thus, the humanized Naᵥ1.7 mice (*Scn9a*^{*hum*/+}, *Scn9a*^{*3.1*/*3.1*} and *Scn9a*^{*3.3*/*3.3*}) described herein provide a system for *in vitro* characterization of anti-hNaᵥ1.7 antibody binding and inhibition of channel function *in vivo.* Further, these mice represent an *in vivo* model system for the examination of new Naᵥ1.7-specific antagonists and evaluation of their therapeutic potential for treating responses mediated by Naᵥ1.7.

**Table 3**

| **CGRP Released from *Scn9a*^{+/+} DRG Exposed to TTX** | |
|---|---|
| **Conditions** | **CGRP (pg/mL)** |
| Buffer | 3 ± 1 |
| IM | 11 ± 1 |
| 1 µM TTX pre-incubation and IM | 76 ± 14 |
| 1 µM TTX + IM | 10 ± 4 |
| IM | 11 ± 4 |
| 10 µM TTX pre-incubation and IM | 44 ± 7 |

**Table 4**

| **CGRP Released from *Scn9a*^{+/+} DRG Exposed to Pro-Txll** | |
|---|---|
| **Conditions** | **CGRP (pg/mL)** |
| IM | 55 ± 5 |
| 10 nM Pro-TXII pre-incubation and IM | 186 ± 40 |
| 100 nM Pro-TXII pre-incubation and IM | 126 ± 25 |

**Table 5**

| **CGRP Released from *Scn9a*^{3.1/3.1} DRG Exposed to Lidocaine** | |
|---|---|
| **Conditions** | **CGRP (pg/mL)** |
| 5 mM Lidocaine preincubation and IM | 40 ± 6 |
| 5 mM Lidocaine + IM | 12 ± 1 |

**Table 6**

| **CGRP Released from *Scn9a*^{+/+}DRG Exposed to anti-hNaᵥ1.7 antibody** | |
|---|---|
| **Conditions** | **CGRP (pg/mL)** |
| IM | 172 ± 17 |
| Ab1 | 29 ± 4 |
| Ab1 pre-incubation and IM | 168 ± 11 |
| Ab2 | 35 ± 2 |
| Ab2 pre-incubation and IM | 522 ± 32 |
| Ab4 | 21 ± 5 |
| Ab4 pre-incubation and IM | 430 ± 2 |
| Ab5 | 39 ± 6 |
| Ab5 pre-incubation and IM | 62 ± 2 |

**Table 7**

| **CGRP Released from *Scn9a^{huml+}* DRG Exposed to anti-hNaᵥ1.7 antibody** | |
|---|---|
| **Conditions** | **CGRP (pg/mL)** |
| Buffer | 2 ± 1 |
| IM | 21 ± 1 |
| Ab1 pre-incubation and IM | 97 ± 14 |
| Ab1 + IM | 19 ± 10 |
| Ab4 pre-incubation and IM | 122 ± 35 |
| Ab4 + IM | 26 ± 1 |
| Ab5 pre-incubation and IM | 82 ± 19 |
| Ab5 + IM | 20 ± 1 |

**Table 8**

| **CGRP Released from *Scn9a*^{*hum*/+} DRG Exposed to anti-hNaᵥ1.7 antibody** | |
|---|---|
| **Conditions** | **CGRP (pg/mL)** |
| IM | 30 ± 7 |
| Ab4 pre-incubation and IM | 81 ± 12 |
| Ab5 pre-incubation and IM | 117 ± 1 |

**Table 9**

| **CGRP Released from or *Scn9a*^{*3.1*/*3.1*} DRG Exposed to anti-hNa_{V}1.7 antibody** | |
|---|---|
| **Conditions** | **CGRP (pg/mL)** |
| IM | 27 ± 3 |
| Ab1 + IM | 12 ± 2 |
| Ab1 pre-incubation and IM | 38 ± 7 |
| Ab4 pre-incubation and IM | 101 ± 2 |
| Ab5 pre-incubation and IM | 47 ± 6 |

### Example VII

### Generation of Immortilized DRG Cell Lines from Humanized Mice

DRG neurons from humanized mice may be isolated and immortalized for continuous long-term study of human Naᵥ1.7 channel function *in vitro*.

DRG neurons can be immortilized by any method known in the art (*e.g.,* see US 2009/0298095A1). Typically immortalizing isolated DRGs is accomplished by employing a vector of retroviral origin that has been engineered with DNA sequences encoding an oncogene (*e.g.* myc) and a selectable marker (*e.g.,* neomycin). Suitable oncogenes that can be cloned into a vector for immortalizing an isolated DRG cell include growth factors or mitogens (*e.g.,* c-Sis), receptor tyrosine kinases (*e.g.,* epidermal growth factor receptor, platelet-derived growth factor receptor, and vascular endothelial growth factor receptor), cytoplasmic tyrosine kinases (*e.g.,* Src-family, Syk-ZAP-70 family, and BTK family of tyrosine kinases), cytoplasmic serine/threonine kinases and their regulatory subunits (*e.g.,* overexpression of Raf kinase and cyclin-dependent kinases), regulatory GTPases (*e.g.,* Ras), and transcription factors (*e.g.,* myc). Once the vector is constructed to harbor both DNA sequences such that they are capable of transcription within the cell, it can be used to create an immortilized DRG cell line by transfection into isolated DRGs from a humanized mouse as described in Examples 2 - 4.

Briefly, dissociated primary DRG neurons can be prepared by methods known in the art (*e.g*. see Wood J.N., Bevan, S.J., Coote, P.R., Dunn, P.M., Harmar, A. Hogan, P., Latchman, D.S., Morrison, C., Rougon, G., Theveniau, M., Wheatley, S. 1990. Novel Cell lines display properties of nociceptive sensory neurons. Proceedings of Biological Sciences, The Royal Society 241(1302):187-94; Raymond, H.K., Thode, S., Zhou, J., Friedman, G.C., Pardinas, J.R., Barrere, C., Johnson, R.M., Sah, D.W.Y. 1999. Immortilized human dorsal root ganglion cells differentiate into neurons with nociceptive properties. Journal of Neuroscience 19(13):5420-5428; Chen, W., Mi, R., Haughey, N., Oz, M., Höke, A. 2007. Immortialization and characterization of a nociceptive dorsal root ganglion sensory neuronal line. J of the Perhipheral Nervous System 12:121-130). Cultures of isolated DRGs that express a human Naᵥ1.7 as described in Examples 2 - 4 are then transfected, e.g. by electroporation, with a candidate vector engineered as described above.

After transfection, cell cultures are grown in selection medium and maintained in the selection medium for up to 1-2 weeks until isolated colonies with 200-300 cells formed. Colonies are picked and expanded using standard culture methods when reached about 80-90% confluence. Cells from culture may be screened for expression of human Naᵥe1.7 protein by Southern or Western Blot using probes designed from the human Naᵥe1.7 sequence. Alternatively, confirmation of human Naᵥ1.7 channel protein in the transfected cells can be achieved by polymerase chain reaction on isolated DNA or RNA from the transfected cells.

Once the immortilized DRG neuronal cell line has demonstrated self-replication capability for multiple generations, it will be suitable for several different assays, including, for example, analysis of neuronal properties of the human Naᵥ1.7 channel, neuronal toxicity assays, measurement of DRG response to nociceptive stimuli, patch-clamp assays, high-throughput drug screening, and testing of Naᵥ1.7 specific blockers (*e.g.,* an anti-NaV1.7 antibody).

## Claims

1. A method for identifying a Naᵥ1.7 antagonist, comprising:
(a) providing ex vivo a cell that expresses a full-length human Naᵥ1.7 α subunit protein, or a chimeric Naᵥ1.7 α subunit protein comprising an extracellular loop of domain I or III of the mouse Naᵥ1.7 α subunit that has been replaced by the corresponding loop from a human Naᵥ1.7 α subunit;
(b) contacting the cell with a test compound that binds to, or blocks a function of, the human Naᵥ1.7 α subunit protein or the chimeric Naᵥ1.7 α subunit protein of (a) and waiting a first period of time;
(c) contacting the cell from (b) with one or more inflammatory mediators and waiting a second period of time;
(d) determining the amount of calcitonin gene-related peptide (CGRP) released from the cell following step (c); and
(e) comparing the amount of CGRP released in step (d) to the amount of CGRP released in the presence of a known sodium channel inhibitor, wherein an increase in CGRP released in the presence of a test compound indicates inhibition of Naᵥ1.7.

2. The method of claim 1, wherein the cell is a neuronal cell, preferably wherein the neuronal cell is a dorsal root ganglion cell (DRG).

3. The method of claim 2, wherein the cell is a mouse cell.

4. The method of claim 1, wherein the amount of CGRP released in the presence of the test compound inhibits one or more functions of the human or chimeric Naᵥ1.7 protein and is at least 2-fold higher than the known sodium channel inhibitor.

5. A method for identifying a Naᵥ1.7 antagonist, comprising:
(a) contacting a DRG cell that expresses a full-length human Naᵥ1.7 α subunit protein, or a chimeric Naᵥ1.7 α subunit protein comprising an extracellular loop of domain I or III of the mouse Naᵥ1.7 α subunit that has been replaced by the corresponding loop from a human Naᵥ1.7 α subunit with a test compound that binds to, or blocks a function of, the human Naᵥ1.7 α subunit protein or the chimeric Naᵥ1.7 α subunit protein and waiting a first period of time; (b) contacting the DRG from (a) with one or more inflammatory mediators and waiting a second period of time;
(c) determining the amount of calcitonin gene-related peptide (CGRP) released from the DRG following step (b); and
(d) comparing the amount of CGRP released in step (c) to the amount of CGRP released in the presence of a known sodium channel inhibitor, wherein an increase in CGRP released in the presence of a test compound indicates inhibition of Naᵥ1.7.

6. The method of claim 1 or 5, wherein the agent is selected from an antibody, a peptide, an organic compound, and a toxin, preferably wherein the antibody is human.

7. The method of claim 1 or 5, wherein the one or more inflammatory mediators are selected from prostaglandin E₂, bradykinin, capsaicin, protons, and a neurotrophic factor, preferably wherein the one or more inflammatory mediators are prostaglandin E₂, bradykinin and capsaicin.

8. The method of claim 5, wherein the DRG is isolated from a member of the group consisting of human, mouse, rat, or monkey, preferably wherein the DRG is isolated from a mouse.

9. The method of claim 1 or 5, wherein the cell is isolated from a genetically modified mouse that comprises, in its germline, a nucleotide sequence that encodes a human Naᵥ1.7α subunit or fragment thereof operably linked to a Naᵥ promoter.

10. The method of claim 1 or 5, wherein the known sodium channel inhibitor is tetrodotoxin or ProTx-II.

11. The method of claim 1 or 5, wherein the full-length human Naᵥ1.7α subunit protein is encoded by exons 2 to exons 28 of a human Naᵥ1.7 gene.

12. The method of claim 11, wherein the full-length human Naᵥ1.7 α subunit protein is a human Naᵥ1.7 variant.

13. The method of claim 12, wherein the human Naᵥ1.7 variant comprises an amino acid substitution selected from a Q10R, I136V, F216S, S241T, N395K, V400M, L823R, I848T, L858H, L858F, A863P, V872G, F1449V, or a combination thereof.

14. The method of claim 12, wherein the human Naᵥ1.7 variant comprises an amino acid substitution selected from a R996C, V1298D, V1298F, V1299F, I1461T, F1462V, T1464I, M1627K, A1632E, or a combination thereof.

15. The method of claim 12, wherein the human Naᵥ1.7 variant comprises an amino acid substitution selected from a F1200L, I1235L, or a combination thereof.

## Patentansprüche

1. Ein Verfahren zur Identifizierung eines Naᵥ1.7-Antagonisten, umfassend:
(a) *Ex vivo* Bereitstellen einer Zelle, die ein volllängc humanes Naᵥ 1.7 α-Untereinheit-Protein oder ein chimäres Naᵥ 1.7 α-Untereinheit-Protein exprimiert, das eine extrazelluläre Schleife der Domäne I oder III der Maus- Naᵥ1.7 α-Untereinheit umfasst, die durch die entsprechende Schleife einer humanen Naᵥ 1.7 α-Untereinheit ersetzt wurde;
(b) Kontaktieren der Zelle mit einer Testverbindung, die an das humane Naᵥ1.7 α-Untereinheit-Protein oder das chimäre Naᵥ1.7 α-Untereinheit-Protein von (a) bindet oder eine Funktion davon blockiert und Warten für einen ersten Zeitraum;
(c) Kontaktieren der Zelle aus (b) mit ein oder mehr inflammatorischen Mediatoren und Warten für einen zweiten Zeitraum;
(d) Bestimmen der Menge an Calcitonin-Gen-verwandtes Protein (CGRP), das aus der Zelle in Folge des Schritts (c) freigesetzt wurde; und
(e) Vergleichen der in Schritt (d) freigesetzten Menge an CGRP mit der Menge an CGRP, das in Anwesenheit eines bekannten Natriumkanal-Inhibitors freigesetzt wird, wobei eine Zunahme des in Anwesenheit einer Testverbindung freigesetzten CGRPs eine Inhibition des Naᵥ1.7 anzeigt.

2. Das Verfahren nach Anspruch 1, wobei die Zelle eine neuronale Zelle ist, wobei die neuronale Zelle vorzugsweise eine dorsale Wurzelganglienzelle (DRG) ist.

3. Das Verfahren nach Anspruch 2, wobei die Zelle eine Maus- Zelle ist.

4. Das Verfahren nach Anspruch 1, wobei die in Anwesenheit der Testverbindung freigesetzte Menge an CGRP ein oder mehr Funktionen des humanen oder chimären Naᵥ 1.7 -Proteins inhibiert und mindestens zweimal höher ist als der bekannte Natriumkanal-Inhibitor.

5. Ein Verfahren zur Identifizierung eines Naᵥ1.7-Antagonisten, umfassend:
(a) Kontaktieren einer DRG-Zelle, die ein volllänge humanes Naᵥ1.7 α-Untereinheit-Protein oder ein chimäres Naᵥ1.7 α-Untereinheit-Protein exprimiert, das eine extrazelluläre Schleife der Domäne I oder III der Maus- Naᵥ1.7 α-Untereinheit umfasst, die durch die entsprechende Schleife einer humanen Naᵥ1.7 α-Untereinheit ersetzt wurde, mit einer Testverbindung, die an das humane Naᵥ1.7 α-Untereinheit-Protein oder das chimäre Naᵥ1.7 α-Untereinheit-Protein von (a) bindet oder eine Funktion davon blockiert und Warten für einen ersten Zeitraum;
(b) Kontaktieren der DRG aus (a) mit ein oder mehr inflammatorischen Mediatoren und Warten für einen zweiten Zeitraum;
(c) Bestimmen der Menge an Calcitonin-Gen-verwandtes Protein (CGRP), das aus der DRG in Folge des Schritts (b) freigesetzt wurde; und
(d) Vergleichen der in Schritt (c) freigesetzten Menge an CGRP mit der Menge an CGRP, das in Anwesenheit eines bekannten Natriumkanal-Inhibitors freigesetzt wird, wobei eine Zunahme des in Anwesenheit einer Testverbindung freigesetzten CGRPs eine Inhibition des Naᵥ1.7 anzeigt.

6. Das Verfahren von Anspruch 1 oder 5, wobei das Agens ausgewählt ist aus einem Antikörper, einem Peptid, einer organischen Verbindung und einem Toxin, wobei der Antikörper vorzugsweise human ist.

7. Das Verfahren nach Anspruch 1 oder 5, wobei die ein oder mehr inflammatorischen Mediatoren ausgewählt sind aus Prostaglandin E₂, Bradykinin, Capsaicin, Protonen und einen neurotrophen Faktor, wobei vorzugsweise die ein oder mehr inflammatorischen Mediatoren Prostaglandin E₂, Bradykinin und Capsaicin sind.

8. Das Verfahren nach Anspruch 5, wobei die DRG von einem Mitglied der Gruppe bestehend aus Mensch, Maus, Ratte oder Affe isoliert wird, wobei vorzugsweise die DRG von der Maus isoliert wird.

9. Das Verfahren nach Anspruch 1 oder 5, wobei die Zelle von einer genetischmodifizierten Maus isoliert wird, die in ihrer Keimbahn eine Nukleotidsequenz umfasst, die eine humane Naᵥ1.7 α-Untereinheit oder Fragment davon kodiert und funktionsfähig mit einem Naᵥ-Promotor verknüpft ist.

10. Das Verfahren von Anspruch 1 oder 5, wobei der bekannte Natriumkanal-Inhibitor Tetrodotoxin oder ProTx-II ist.

11. Das Verfahren nach Anspruch 1 oder 5, wobei das volllänge humane Naᵥ1.7 α-Untereinheit-Protein von Exon 2 bis Exon 28 des humanen Naᵥ1.7-Gens kodiert wird.

12. Das Verfahren nach Anspruch 11, wobei das volllänge humane Naᵥ1.7 α-Untereinheit-Protein eine humane Nₐᵥ1.7-Variante ist.

13. Das Verfahren nach Anspruch 12, wobei die humane Naᵥ1.7-Variante eine Aminosäuresubstitution umfasst, ausgewählt aus einer Q10R, I136V, F216S, S241T, N395K, V400M, L823R, I848T, L858H, L858F, A863P, V872G, F1449V oder einer Kombination davon.

14. Das Verfahren nach Anspruch 12, wobei die humane Naᵥ1.7-Variante eine Aminosäuresubstitution umfasst, ausgewählt aus einer R996C, V1298D, V1298F, V1299F, I1461T, F1462V, T1464I, M1627K, A1632E oder einer Kombination davon.

15. Das Verfahren nach Anspruch 12, wobei die humane Naᵥ1.7-Variante eine Aminosäuresubstitution umfasst, ausgewählt aus einer F1200L, I1235L oder einer Kombination davon.

## Revendications

1. Procédé d'identification d'un antagoniste de la Naᵥa1.7, comprenant :
(a) la fourniture *ex vivo* d'une cellule qui exprime une protéine de 1a sous-unité α de la Naᵥ1.7 humaine pleine longueur, ou une protéine de la sous-unité α de la Naᵥ1.7 chimérique comprenant une boucle extracellulaire du domaine I ou III de la sous-unité α de la Naᵥa1.7 de souris qui a été remplacée par la boucle correspondante issue d'une sous-unité α de la Naᵥa1.7 humaine ;
(b) la mise en contact de la cellule avec un composé à tester qui se lie à, ou bloque une fonction de, la protéine de la sous-unité α de la Naᵥ1.7 humaine ou la protéine de la sous-unité α de la Naᵥ1.7 chimérique de (a) et l'attente pendant une première période de temps ;
(c) la mise en contact de la cellule issue de (b) avec un ou plusieurs médiateurs inflammatoires et l'attente pendant une seconde période de temps ;
(d) la détermination de la quantité de peptide associé au gène de la calcitonine (CGRP) libéré à partir de la cellule à la suite de l'étape (c) ; et
(e) la comparaison de la quantité de CGRP libéré dans l'étape (d) à la quantité de CGRP libéré en présence d'un inhibiteur de canal sodique connu, dans lequel une augmentation de CGRP libéré en présence d'un composé à tester indique une inhibition de la Naᵥ1.7.

2. Procédé selon la revendication 1, dans lequel la cellule est une cellule neuronale, de préférence dans lequel la cellule neuronale est une cellule de ganglion rachidien (DRG).

3. Procédé selon la revendication 2, dans lequel la cellule est une cellule de souris.

4. Procédé selon la revendication 1, dans lequel la quantité de CGRP libéré en présence du composé à tester inhibe une ou plusieurs fonctions de la protéine Naᵥ1.7 humaine ou chimérique et est au moins 2 fois supérieure à l'inhibiteur de canal sodique connu.

5. Procédé d'identification d'un antagoniste de la Naᵥ1.7, comprenant :
(a) la mise en contact d'une cellule de DRG qui exprime une protéine de la sous-unité α de la Naᵥa1.7 humaine pleine longueur, ou une protéine de la sous-unité α de la Naᵥa1.7 chimérique comprenant une boucle extracellulaire du domaine I ou III de la sous-unité α de la Naᵥa1.7 de souris qui a été remplacée par la boucle correspondante issue d'une sous-unité α de la Naᵥa1.7 humaine avec un composé à tester qui se lie à, ou bloque une fonction de, la protéine de la sous-unité α de la Naᵥ1.7 humaine ou la protéine de la sous-unité α de la Naᵥ1.7 chimérique et l'attente pendant une première période de temps ;
(b) la mise en contact du DRG issu de (a) avec un ou plusieurs médiateurs inflammatoires et l'attente pendant une seconde période de temps ;
(c) la détermination de la quantité de peptide associé au gène de la calcitonine (CGRP) libéré à partir du DRG à la suite de l'étape (b) ; et
(d) la comparaison de la quantité de CGRP libéré dans l'étape (c) à la quantité de CGRP libéré en présence d'un inhibiteur de canal sodique connu, dans lequel une augmentation de CGRP libéré en présence d'un composé à tester indique une inhibition de la Naᵥ1.7.

6. Procédé selon la revendication 1 ou 5, dans lequel l'agent est choisi parmi un anticorps, un peptide, un composé organique, et une toxine, de préférence dans lequel l'anticorps est humain.

7. Procédé selon la revendication 1 ou 5, dans lequel les un ou plusieurs médiateurs inflammatoires sont choisis parmi la prostaglandine E₂, la bradykinine, la capsaïcine, les protons, et un facteur neurotrophique, de préférence dans lequel les un ou plusieurs médiateurs inflammatoires sont la prostaglandine E₂, la bradykinine et la capsaïcine.

8. Procédé selon la revendication 5, dans lequel le DRG est isolé à partir d'un membre du groupe constitué de l'être humain, de la souris, du rat, ou du singe, de préférence dans lequel le DRG est isolé à partir d'une souris.

9. Procédé selon la revendication 1 ou 5, dans lequel la cellule est isolée à partir d'une souris génétiquement modifiée qui comprend, dans sa lignée germinale, une séquence nucléotidique qui code pour une sous-unité α de la Naᵥa1.7 humaine ou l'un de ses fragments liée de façon fonctionnelle à un promoteur de Naᵥ.

10. Procédé selon la revendication 1 ou 5, dans lequel l'inhibiteur de canal sodique connu est la tétrodotoxine ou la ProTx-II.

11. Procédé selon la revendication 1 ou 5, dans lequel la protéine de la sous-unité α de la Naᵥ1.7 humaine pleine longueur est codée par les exons 2 aux exons 28 d'un gène de la Naᵥ1.7 humaine.

12. Procédé selon la revendication 11, dans lequel la protéine de la sous-unité α de la Naᵥa1.7 humaine pleine longueur est un variant de la Naᵥa1.7 humaine.

13. Procédé selon la revendication 12, dans lequel le variant de la Naᵥ1.7 humaine comprend une substitution d'acide aminé choisie parmi Q10R, I136V, F216S, S241T, N395K, V400M, L823R, I848T, L858H, L858F, A863P, V872G, F1449V, ou l'une de leurs combinaisons.

14. Procédé selon la revendication 12, dans lequel le variant de la Naᵥ1.7 humaine comprend une substitution d'acide aminé choisie parmi R996C, V1298D, V1298F, V1299F, I1461T, F1462V, T1464I, M1627K, A1632E, ou l'une de leurs combinaisons.

15. Procédé selon la revendication 12, dans lequel le variant de la Naᵥ1.7 comprend une substitution d'acide aminé choisie parmi F1200L, I1235L, ou l'une de leurs combinaisons.
